(19) Europäisches Patentamt
European Patent Office
Office européen des brevets

(11) **EP 4 218 474 A1**

(12) **EUROPEAN PATENT APPLICATION**

(43) Date of publication:
**02.08.2023 Bulletin 2023/31**

(21) Application number: **23167562.0**

(22) Date of filing: **25.10.2013**

(51) International Patent Classification (IPC):
***A43B 1/00*** *(2006.01)* ***A63B 24/00*** *(2006.01)*
***G01S 13/34*** *(2006.01)* ***G10L 25/51*** *(2013.01)*
***A61B 5/11*** *(2006.01)* ***A61B 5/00*** *(2006.01)*
***G06V 20/10*** *(2022.01)* ***G01S 13/75*** *(2006.01)*
***G01S 13/82*** *(2006.01)* ***G06V 20/40*** *(2022.01)*
***A43B 3/34*** *(2022.01)* ***A43B 5/02*** *(2006.01)*

(52) Cooperative Patent Classification (CPC):
**G01S 13/34; A43B 3/34; A43B 5/02; A61B 5/1123; A61B 5/1126; A61B 5/1128; G01S 13/751; G01S 13/82; G06V 20/10; G06V 20/42; G10L 25/51;** A61B 5/1112; A61B 5/6807; A61B 5/6895; A61B 2562/0219; (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.10.2012 US 201213660743**

(62) Document number(s) of the earlier application(s) in accordance with Art. 76 EPC:
**13786599.4 / 2 912 601**

(71) Applicant: **Nike Innovate C.V.**
**Beaverton, OR 97005 (US)**

(72) Inventors:
- **BALAKRISHNAN, Santoshkumar**
  **Beaverton, Oregon, 97005 (US)**
- **HOLMES, Steve**
  **Beaverton, Oregon, 97005 (US)**
- **WEAST, Aaron B**
  **Beaverton, Oregon, 97005 (US)**

(74) Representative: **Haseltine Lake Kempner LLP**
**One Portwall Square**
**Portwall Lane**
**Bristol BS1 6BH (GB)**

Remarks:
This application was filed on 12-04-2023 as a divisional application to the application mentioned under INID code 62.

# (54) ATHLETIC PERFORMANCE MONITORING SYSTEMS AND METHODS IN A TEAM SPORTS ENVIRONMENT

(57) Systems, apparatuses, and methods for determining when an athlete is in possession of a ball by analyzing image data are provided. A camera is worn by an athlete and is turned on when the athlete is in proximity of a ball. The camera is used to generate an image of a ball. The size of the ball is determined and compared to a threshold. The athlete is considered to be in possession of the ball when the size of the image exceeds the threshold.

102b
10804b
102a
10804a
11200a
11200b
130

**FIG. 112**

EP 4 218 474 A1

(52) Cooperative Patent Classification (CPC): (Cont.)
A61B 2562/0247

## Description

### RELATED APPLICATION DATA

This application claims priority to, and the benefit of, United States Patent Application No. 13/660,743 filed October 25, 2012 and entitled "Athletic Performance Monitoring Systems and Methods in a Team Sports Environment". This earlier patent application is entirely incorporated herein by reference.

### FIELD OF THE INVENTION

The present invention relates to systems and methods for monitoring player performance during athletic activities (*e.g.,* during a game, a practice session, a workout, etc.), including team oriented athletic activities. Such systems and methods may be useful for evaluating performances of one or more players in various team sporting activities, such as soccer, basketball, American football, hockey, rugby, field hockey, lacrosse, baseball, cricket, volleyball, badminton, and the like. The systems and methods may be used by the individual as a measuring stick and motivation to improve, as well as by coaches or trainers.

### BACKGROUND

Many systems are available for measuring features of athletic performance. For example, many gyms and fitness centers are equipped with specialized systems that help track a user's use of the machines (*e.g.,* card readers, RFID equipment, etc.). The usage data may be automatically generated and downloaded to a central computer system and made available for the athlete's review. One disadvantage of such systems is that their use is confined to use with specialized equipment within the "four walls" of the gym or fitness center.

The NIKE+ ™ athletic performance monitoring system (available from NIKE, Inc. of Beaverton, Oregon) provides a convenient system and method that allows individuals to measure and collect data relating to ambulatory exercise, such as walking or running. Data collection using a NIKE+ ™ system is not confined to any specific geographic location. Rather, the system can be used at any desired locations, both indoor and outdoor.

Not all personal exercise and athletic endeavors, however, are limited to walking and running. Many individuals participate in team games, such as soccer, basketball, football, and the like. At present time, there is no easy or convenient system that is useful to automatically collect, compile, and store data that accurately and empirically depicts a player's efforts when participating in these team sports. Such systems would be useful to help a player gauge his or her performance, to help a player identify areas where improvement may be achieved, and to help a player recognize when improvement has been achieved. Additionally, such systems would be useful tools for coaches and trainers, to help them ascertain each individual's strengths and weaknesses and to help them field the best combination of players for a given game situation (*e.g.,* a "scoring" team, a "defense" team, etc.). Moreover, such systems could provide enormous motivation for the athlete by enabling the athlete (or others) to set performance goals and/or challenges.

### SUMMARY OF THE INVENTION

The following presents a general summary of aspects of the present invention in order to provide a basic understanding of the invention and various example features of it. This summary is not intended to limit the scope of the invention in any way, but it simply provides a general overview and context for the more detailed description that follows.

Aspects of this invention relate to systems, methods, and computer-readable media with computer-executable instructions stored thereon for performing methods and/or operating user interfaces relating to the monitoring of player performance during athletic activities (*e.g.,* during a game, a practice session, a workout, etc.), including team oriented athletic activities.

Additional aspects of this invention relate to systems and methods for sensing and monitoring various athletic performance metrics, e.g., during the course of a game, a practice, a training session, training drills, and the like.

Systems in accordance with at least some examples of this invention may include systems for monitoring performance parameters of one or more athletes in a team sport setting. Such systems may include one or more of: (a) a sensor system for monitoring one or more of: (i) a first parameter correlated to a first player's movement speed during a first time period, (ii) a second parameter correlated to a determination of when the first player possesses the ball and when the first player does not possess the ball during the first time period, and (iii) a third parameter correlated to the first player's ball transfer speed, force, or power during the first time period; (b) a data storage system for storing data collected by the sensor system relating to the first, second, and third parameters; (c) a processor system for receiving and processing data stored in the data storage system; and (d) an output device for outputting user perceptible output including athletic performance metric information based on the collected and stored data.

As some additional examples, athletic performance monitoring systems according to at least some examples of this invention may include: (a) at least one sensor system selected from the group consisting of: a RADAR-based sensor system, a radio or radio frequency based sensor system, a global positioning satellite based sensor system, a magnet based sensor system, a magnetic coil based sensor system, a pressure sensor system, an accelerometer sensor system, a gyroscope based sensor system, a time sensor or clock, and a compass, wherein at least one of the at least one sensor system is provided in or on an article of apparel, an article of footwear, a ball, or a hockey puck; (b) means for receiving output from the at least one sensor system; and (c) processing means program and adapted to determine or sense, based on the output received at the means for receiving, data relating to at least one event, metric, or feature selected from the group consisting of: a player receiving possession of the ball or puck; player possession of the ball or puck; a player's speed while in possession of the ball or puck; one or more characteristics of dribbling the ball; a knock on and sprint event; close control of the ball or puck; dribble foot distribution; control of an incoming ball or puck; a one touch pass event; a tackle avoided event; a successful tackle event; a skin event; a ball or puck possession or proximity heat map; a player intensity metric; boot kick zone information; ball or puck flight path distribution or information; longest kick or hit information; ball or puck flight elevation angle information; kick type distribution information; kick or shot power information; kick or pass style information; kick or shot power information at a predetermined threshold movement speed; pass accuracy information at a predetermined threshold movement speed; volley information; a free kick award event; information distinguishing a free kick from a penalty kick; a set piece event; a set piece save event; information for determining whether a set piece kick is on goal; player movement direction information based on body angle; a player turn in event; a player turn in event when in possession of or proximity to the ball; player movement direction or type; information regarding an amount of time a player spends on his or her toes; player posturing or player facing direction; man-to-man opposing position information; information relating to a player's ability to draw opposition; information regarding a player's speed in breaking away from defensive players; a successful pass event; a pass interception event; a give and go event; information relating to a ball or puck pass that passes in proximity to a defensive player but continues on to complete a successful pass; pass direction distribution information; pass player distribution information; information indicating an out of bounds event; information indicating an intentional out of bounds event; information identifying a goal keeper; information identifying a scored goal; information identifying a save; information identifying a keeper parry event; information identifying a keeper parry event with respect to a ball speed above a predetermined threshold amount; information identifying a keeper advance or tackle event; information identifying a player dive or a player jump event, and optionally, a jump height associated with the jump event; information identifying a drop kick event; information identifying a shot on goal that goes out of bounds; information identifying a shot on goal; an automatic pick of team captains; an automatic pick of team goal keepers; an automatic pick of teams; information indicating a game start; information for automatically identifying a direction of play for a team or an individual player; information for automatically identifying an individual player's teammates or an entire team based on pass distribution information; information for automatically identifying an individual player's teammates or an entire team based on player orientation; information for automatically identifying an individual player's teammates or an entire team based on an object's orientation, wherein each player carries an object that is oriented in a first predetermined manner to indicate players on one team; team identification using pre-game ball proximity or passing information; information regarding magnetic characteristics of a ball; ball juggling information; ball pressure information as a function of magnetic field strength associated with the ball; ball proximity to an article of apparel; information for changing a state of magnetic fluid contained in a shoe based on proximity to a ball or puck; information for change a state of an article of protective gear based on proximity to a foot; information relating to running state based on magnetic properties of an article of footwear; information regarding entering a playing field based on sensing a magnetic field; ball possession time information based on reaction of a magnetic switch sensor states within a shoe to a magnetic field generator within a ball or puck; information relating to a player on-field location heat map; information relating to player explosiveness; and information relating to whether a ball is being thrown or kicked.

Additional aspects of the invention relate to estimating the distance between a player and a ball by transmitting a chirp (that may be referred as a sweep signal) to a tag located on the ball. During the chirp, the frequency of the transmitted signal is changed in a predetermined fashion. The tag doubles the transmitted frequency and returns the processed signal to a transceiver typically located on the player. The currently transmitted frequency is compared with the received frequency to obtain a difference frequency from which an apparatus may estimate the distance and the velocity. The apparatus may simultaneously receive the processed signal from the tag while transmitting the sweep signal.

Additional aspects of this invention relate to methods of operating athletic performance monitoring systems of the types described above, as well as to athletic performance monitoring methods that determine or sense data relating to at least one event, metric, or feature described above, e.g., using the various systems described above. Still additional aspects of this invention relate to user perceptible output systems, including graphical user interfaces displayed on computer devices, that provide output information to users of systems and methods according to this invention.

## BRIEF DESCRIPTION OF THE DRAWINGS

The present invention is illustrated by way of example and not limited in the accompanying figures, in which like reference numerals indicate the same or similar elements throughout, and in which:

Fig. 1 generally illustrates the components and features of one example athletic performance monitoring system in accordance with this invention;

Figs. 2A through 2E illustrate example features of various products that may be used in athletic performance monitoring systems and methods in accordance with this invention;

Fig. 3 illustrates a schematic view with a more detailed depiction of certain components of Fig. 1;

Figs. 4 and 5 illustrate features of an alternative example athletic performance monitoring system in accordance with this invention;

Figs. 6 and 7 illustrate various potential features useful in determining ball "possession" or ball "proximity" in accordance with at least some examples of this invention;

Figs. 8A through 8C illustrate variations in radio frequency identification ("RFID") systems that may be used for "proximity" or "possession" determinations in athletic performance monitoring systems and methods in accordance with this invention;

Fig. 9 illustrates example features and components of a semi-passive RFID based "proximity" or "possession" determination system that may be used in systems and methods in accordance with this invention;

Fig. 10 illustrates example features and components of a digital radio packet based "proximity" or "possession" determination system that may be used in systems and methods in accordance with this invention;

Fig. 11 illustrates an example passive frequency doubler system that may be used in "proximity" or "possession" determination systems and methods in accordance with this invention;

Fig. 12 illustrates example features and components of a RADAR based "proximity" or "possession" determination system that may be used in systems and methods in accordance with this invention;

Fig. 13 is a diagram that aids in the discussion of multi-player concurrent usage of systems and methods of the invention and "data collisions;"

Fig. 14 is a diagram that aids in the discussion of multi-player concurrent use of systems and methods in accordance with this invention;

Figs. 15-18 illustrate example features of user interfaces that may be provided by systems and methods according to examples of this invention;

Figs. 19A and 19B illustrate various features that assist in explaining differences in detector response for throwing actions v. kicking actions in accordance with examples of this invention;

Fig. 20 assists in explanation of detection and/or measurement of an "explosiveness" metric in accordance with examples of this invention;

Fig. 21 assists in explanation of detection and/or measurement of an acceleration metric in accordance with examples of this invention; and

Figs. 22 through 94 assist in explanation of detection and/or measurement of various athletic performance metrics, features, and/or other features of systems and methods in accordance with examples of this invention.

Fig. 95 shows an exemplary embodiment of a passive frequency doubler tag that may be embedded on a puck or ball in accordance with embodiments of the invention.

Fig 96 shows a two-element sinuous antenna that may be incorporated with a frequency doubler tag in accordance with embodiments of the invention.

Fig. 97 shows an antenna plot for a two-element sinuous antenna at 2.45 GHz in accordance with embodiments of the invention.

Fig. 98 shows an antenna plot for a two-element sinuous antenna at 4.8 GHz in accordance with embodiments of the invention.

Fig. 99 shows a crossed magnetic slotted dipole that may be incorporated with a frequency doubler tag in accordance with embodiments of the invention.

Fig. 100 shows an antenna plot for a crossed magnetic slotted dipole at 2.45 GHz in accordance with embodiments of the invention.

Fig. 101 shows an antenna plot for a crossed magnetic slotted dipole at 4.9 GHz in accordance with embodiments of the invention.

Fig. 102 shows a turnstile dipole that may be incorporated with a frequency doubler tag in accordance with embodiments of the invention.

Fig. 103 shows an antenna plot for a turnstile dipole at 2.45 GHz in accordance with embodiments of the invention.

Fig. 104 shows an antenna plot for a turnstile dipole at 4.9 GHz in accordance with embodiments of the invention.

Fig. 105 shows a system for a frequency doubler tag in accordance with embodiments of the invention.

Fig. 106 shows a relationship between the range and signal strength in accordance with embodiments of the invention.

Fig. 107 shows a flow chart that may be performed by the system as shown in Fig. 105 in accordance with embodiments of the invention.

Fig. 108 illustrates an example embodiment where a camera and a transducer are worn by an athlete while participating in an athletic activity in accordance with embodiments of the invention.

Figs. 109A-B illustrate example images generated by a camera worn by a user in accordance with example embodiments..

Figs. 110a-b illustrate example signals output by a transducer in accordance with an example embodiment.

Fig. 111 illustrates an example possession display in accordance with example embodiments.

Fig. 112 illustrates an example of determining contested possession of an object in accordance with example embodiments.

Fig. 113 illustrates an example flow diagram of a method for determining whether a user is in possession of an object, in accordance with example embodiments.

Fig. 114 illustrates an example flow diagram of a method for determining which of multiple users is in possession of an object, in accordance with example embodiments.

Fig. 115 illustrates an example flow diagram of a method for processing transducer data for determining whether a user is in possession of an object, in accordance with example embodiments.

The reader is advised that the various parts shown in these drawings are not necessarily drawn to scale.

## DETAILED DESCRIPTION

The following description and the accompanying figures disclose features of systems, methods, computer-readable media, and user interfaces in accordance with examples of the present invention.

### I. General Description of Systems, Methods, and User Interfaces in Accordance with this Invention

Aspects of this invention relate to systems, methods, and computer-readable media with computer-executable instructions stored thereon for performing methods and/or operating systems and/or user interfaces relating to the monitoring of player performance during an athletic activity (*e.g.,* during a game, a practice session, a workout, etc.), including team oriented athletic activities.

Systems in accordance with at least some examples of this invention may include systems for monitoring performance parameters of one or more athletes in a team sport setting (*e.g.,* in a game, during practice, as part of a workout program, etc.). Any desired type of team sport may be involved without departing from this invention, such as soccer, basketball, American football, hockey, rugby, field hockey, lacrosse, baseball, cricket, volleyball, badminton, tennis, and the like. Such systems may accumulate data relating to one individual on a team, to multiple individuals on one team, and/or to one or more individuals on each participating team.

As some more specific examples, systems in accordance with at least some examples of this invention may include systems for monitoring athletic performance(s) that include: (a) a sensor system for monitoring one or more of: (i) a first parameter correlated to a first player's movement speed during a first time period, (ii) a second parameter correlated to a determination of when the first player possesses the ball and when the first player does not possess the ball during the first time period, and (iii) a third parameter correlated to the first player's ball transfer speed, force, or power during the first time period; and (b) a data storage system for storing data collected by the sensor system relating to the first, second, and third parameters. The term "ball," as used herein, constitutes any item used in sporting activities that is possessed, thrown, batted, kicked, hit, or otherwise propelled by the athletes in order to achieve a desired goal of the game. In addition to objects that are substantially round or spherical, such as soccer balls, basketballs, field hockey balls, lacrosse balls, baseballs, volleyballs, tennis balls, and cricket balls, the term "ball," when used generically herein, further includes other sport related objects, such as hockey pucks, America footballs, rugby footballs, badminton birdies, and the like.

Systems in accordance with at least some examples of this invention further may include: a processor system for receiving and processing data stored in the data storage system; and an output device (such as an audio device; a video device; an alpha-numeric display device; a computer monitor; a display screen from other electronic devices, such as cellular telephones, watches or other wrist borne devices, portable electronic devices, etc.) for generating a user perceptible output.

Based on the sensed data, systems and methods in accordance with this invention may determine any desired data associated with the athletic performance. As some more specific examples, systems and methods in accordance with examples of this invention may determine one or more of the following performance metrics for one or more of the players participating in an athletic activity: a player's maximum movement velocity during a desired time period; a player's average movement velocity during a desired time period; a player's time correlated movement velocity during a desired time period; a number of times that a player's movement velocity exceeded a predetermined threshold value during a desired time period; an amount of time that a player possessed the ball during a desired time period; an amount of time that a player was located within a predetermined distance from the ball during a desired time period; a player's movement velocity when in possession of the ball during a desired time period; a player's maximum movement velocity when in possession of the ball during a desired time period; a player's average movement velocity when in possession of the ball during a desired time period; a player's time correlated movement velocity when in possession of the ball during a desired time period; a player's ball transfer speed, force, or power (e.g., kick speed, pass speed, throw speed, shot speed, etc.) during a desired time period; a player's maximum ball transfer speed, force, or power during a desired time period; a player's overall movement distance during a desired time period; a player's overall movement distance while in possession of the ball during a desired time period; a number of times that a player possessed the ball during a desired time period; a number of times that a player was located within a predetermined distance from the ball during a desired time period; a number of times that a player contacted the ball during a desired time period; one or more performance goals for a player; whether a player has achieved a performance goal; and a revised performance goal for a player.

The output system associated with systems and methods according to this invention may output information relating to a player's athletic performance in any desired form, format, or manner (e.g., in any user perceptible manner). For example, the output system may output audio, video, alpha-numeric, tactile, and/or graphical information (including through a graphical user interface) relating to any of the performance metrics described above.

Methods for monitoring athletic activities of the types described above may include one or more of the following steps: (a) sensing data relating to one or more of: (i) a first parameter correlated to a first player's movement speed during a first time period, (ii) a second parameter correlated to a determination of when the first player possesses the ball and when the first player does not possess the ball during the first time period, and (iii) a third parameter correlated to the first player's ball transfer speed, force, or power during the first time period; (b) storing the data relating to the first, second, and third parameters; (c) calculating or determining one or more athletic performance metrics based on the sensed or stored data; and (d) generating a user perceptible output that includes information relating to one or more of the calculated or otherwise determined athletic performance metrics. The performance metrics may be any of the various types described above. The user perceptible output may be in any of the various forms or formats described above (e.g., audio, video, alpha-numeric, tactile, and/or graphical information).

When the athletic performance of multiple participants is tracked, the "time periods" for the tracking may be the same or different without departing from this invention. For example, the sensors may collect data for each player only during the time period that the player is actually actively in the game (e.g., when the player is not on the bench). The time period(s) may span one or more games or practice sessions, or they may involve only portions of games or practice sessions. Also, the time period may involve continuous or discontinuous blocks of time (e.g., if a player goes in and out of a game, the sensors may sense the player's activity over the course of the entire game as a single "time period," but only while the player is actively involved in the game).

Additional aspects of this invention relate to generating user perceptible output relating to athletic performance metrics measured and/or determined by systems and methods in accordance with this invention. In some examples, this output may be in the form of a graphical user interface generated on a computer-controlled display device (such as a computer monitor, a display screen for a cellular telephone or other portable electronic device, other audio and/or video display devices, etc.). Such aspects of the invention may include computer-readable media (such as a computer memory, like a hard disk drive, a portable computer memory device, and the like) including computer-executable instructions stored thereon for generating a graphical user interface on a display device, wherein the graphical user interface includes one or more of: (a) a display portion containing information relating to a player's movement speed during a desired time period of an athletic performance activity; (b) a display portion containing information relating to a player's ball possession during a desired time period; (c) a display portion containing information relating to a player's ball transfer speed, force, or power during a desired time period; (d) a display portion containing information relating to a player's maximum movement speed during a desired time period; (e) a display portion containing information relating to a player's maximum movement speed while in possession of the ball during a desired time period; (f) a display portion containing information relating to a number of times that a player's movement speed or power exceeded a predetermined threshold during a desired time period; and (g) a display portion containing information relating to a number of times that a player possessed or contacted a ball during a desired time period. Two or more of the various display portions may be displayed simultaneously, or one may access information contained in some of the display portions through interaction with an element provided in another of the display portions.

Given the general description of various example features and aspects of the invention provided above, more detailed descriptions of various specific examples of athletic performance monitoring systems, methods, computer-readable media, and user interfaces according to the invention are provided below.

## II. Detailed Description of Specific Examples of Features of Athletic Performance Monitoring Systems and Methods According to the Invention

The following discussion and accompanying figures describe various example systems, methods, and computer-readable media with computer-executable instructions stored thereon for performing methods, operating systems, and generating user perceptible output relating to the monitoring of player performance during an athletic activity (*e.g.,* during a game, a practice session, a workout, etc.), including team oriented athletic activities. When the same reference number appears in more than one drawing, that reference number is used consistently in this specification and the drawings to refer to the same or similar parts or elements throughout.

Initially, example hardware for operating systems and performing methods in accordance with this invention will be described. Then, a more detailed explanation of examples of performance monitoring and performance metric determination will be described. Example features of use of systems and methods in accordance with this invention in a multi-user atmosphere will be described. Additionally, features of an example user interface for providing user feedback and information will be described.

### A. Example Hardware Systems

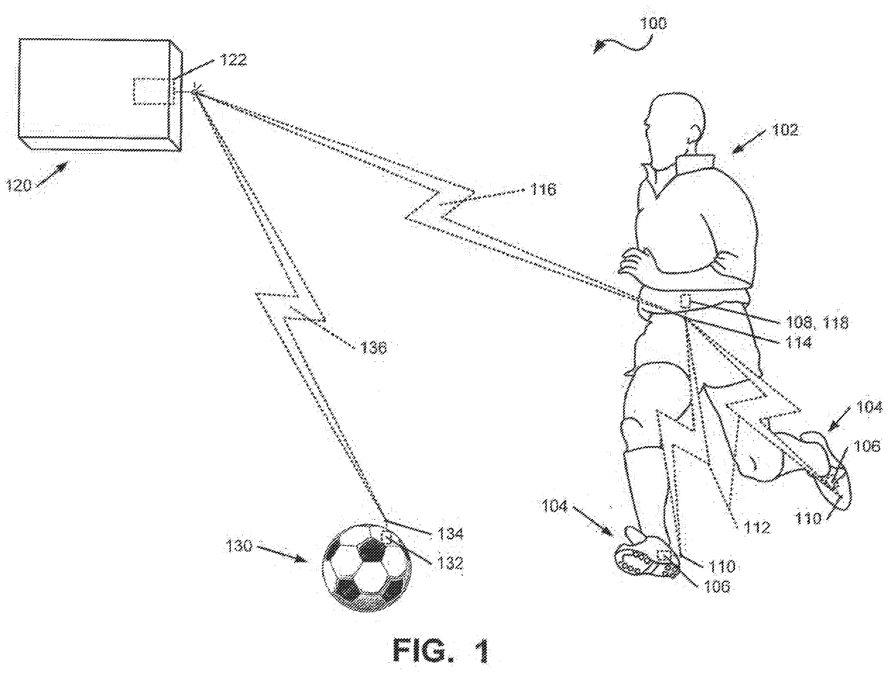

Fig. 1 generally illustrates features of example hardware components that may be included in an athletic performance monitoring system 100 in accordance with this invention. First, the system 100 may include one or more sensors that are carried by the athlete 102 during the course of the game, practice session, or the like (generically referred to herein as an "athletic performance" or "athletic activity"). As some more specific examples, one or more of the athlete's shoes 104 may carry a sensor 106 therein. As will be described in more detail below, the shoe sensors 106 may be used, at least in part, to measure various athletic performance metrics, such as movement speed, movement distance, on ball movement speed, on ball movement distance, off ball movement speed, off ball movement distance, ball possession time or count, kick speed, etc. The shoe based sensors also may be used to provide a record or identify the player that kicked the ball (optionally while also using data from a ball based sensor). In some example systems and methods according to this invention, the shoe 104 based sensors 106 may measure speed and distance in a manner akin to the measurement of speed and distance in NIKE+™ athletic performance monitoring systems available from NIKE, Inc. of Beaverton, Oregon (e.g., pedometer based speed and/or distance type information).

If desired, the foot mounted sensors 106 may transmit relevant data back to a receiver 108 also worn by the athlete 102. While the data may be transmitted in any desired manner, Fig. 1 generally illustrates a wireless type transmission, as shown by transmission elements 110, transmission icons 112, and receiver element 114. Any desired wireless or wired transmission system and method may be used without departing from this invention, including the use of any desired wired or wireless data transmission format or protocol, including the transmission systems and protocols currently in use in NIKE+™ athletic performance monitoring systems.

The receiver 108 receives the data from the one or more shoe mounted sensors 106 and stores this data and/or transmits it to an input system 122 provided in a remote computer system 120. This can be accomplished in real time, during the athletic performance, if desired. Fig. 1 illustrates that the receiver 108 includes a transmission system (i.e., transceiver element 114), and the actual data transmission procedure is represented in Fig. 1 by transmission icon 116.

The remote computer system 120 may be any desired type of computer system, at any desired location, without departing from this invention. For example, the transmission system 114 may transmit over the internet to a remotely located server or other computer system 120, *e.g.,* via cellular telecommunications systems or other wireless publicly or privately available data transmission systems. As other examples, the transmission system 114 may transmit to a sideline based or coaches' box based computer system 120, including to hand-held or portable computer systems 120, like those available in cellular telephones, personal digital assistants, and the like. In this way, the coach, trainer, or athlete 102 (or others) can readily have the collected data available for review and use, even in real time during the athletic performance.

The on-body receiver 108 further may include one or more sensor devices 118, if desired. For example, as will be explained in more detail below, the sensor device(s) 118 may constitute a body core mounted accelerometer that may be useful in determining player acceleration, player movement velocity, player movement distance, on ball movement speed, off ball movement speed, vertical displacement (up or down), and the like. The on-body receiver 108 sensor device(s) 118 also may be useful for sensing the ball, for determining metrics like ball proximity/possession time, on ball speed, on ball acceleration, off ball speed, off ball acceleration, etc. If desired, the body core sensor device(s) 118 may be utilized and the shoe based sensor device(s) 106 may be eliminated (or *vice versa*). As another example, if desired, the shoe based sensor device(s) 106 may directly transmit to computer system 120, without the intermediate transmission to an on-body receiver 108.

In systems and methods in accordance with at least some examples of this invention, the ball 130 also may include one or more sensors 132, a data transmission system 134, or other electronic capabilities (both active and passive). As shown in Fig. 1, the data transmission system 134 of the ball 130 also may transmit data to the remote computer system 120 (*e.g.,* as shown through transmission icon 136). Again, any desired type of transmission system may be used, such as wireless transmission and wireless transmission protocols. As will be described in more detail below, the ball sensor system 132 may be used to provide information useful for determining various metrics such as ball speed, ball location, ball possession (*e.g.,* by ball contact with or proximity to a player), kick speed, kick force, and the like. The ball sensor(s) 132 may include, among other things, one or more accelerometers, gyroscopes, pressure sensors (*e.g.,* piezoelectric sensors, force sensors, etc.), RFID tags, etc. If desired, the ball transmission system 134 could transmit to the receiver 108 in addition to or in place of the transmission to the remote system 120.

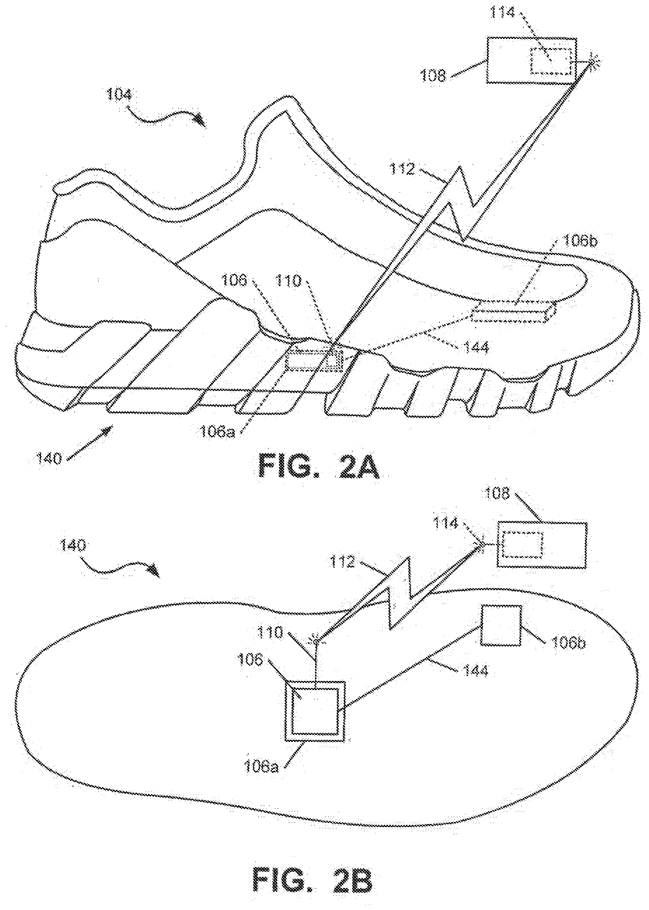

Figs. 2A and 2B illustrate features of a shoe 104 that may include one or more sensors 106 in accordance with at least some examples of this invention. As shown in these figures, the sole 140 of one or both shoes 104 may include a centrally located housing 106a in which sensor 106 is mounted. As noted above, this sensor 106 may be an accelerometer or a pedometer based speed and/or distance type sensor (*e.g.,* a piezoelectric sensor, a force sensor, etc.), and the mounting location and structure may be akin to the mounting of the sensors in NIKE+™ athletic performance monitoring systems available from NIKE, Inc. of Beaverton, Oregon (*e.g.,* mounted generally in the arch area of the sole 140, within a housing 106a defined in the midsole structure and underneath a sock liner or insole member of the shoe 104). Other mounting locations, structures, and arrangements on a shoe 104 (or other foot or leg borne equipment, such as a sock, shin guard, etc.) are possible without departing from this invention.

As further shown in Figs. 2A and 2B, the shoe 104 may include other sensors, such as sensor 106b. This sensor 106b (or sensors) may be provided for other purposes, such as detection of contact with the ball 130 (which may correlate to ball possession), detection of kick force, detection of foot acceleration (which may correlate to kick force, ball speed, etc.), or the like, and it may be provided at any desired location on the shoe 104 (*e.g.,* on the exterior, within the construction, on or incorporated into the upper, etc.). The sensor 106b may be an accelerometer, a force sensor, a pressure sensor (*e.g.,* a piezoelectric sensor), or the like. Other sensors also may be provided on one or both shoes 104 worn by the athlete 102 without departing from this invention. When sensors are provided on both shoes, these sensors may measure the same or different parameters.

Figs. 2A and 2B illustrate that the sensor 106b is connected to sensor 106 via connection 144, and in this manner, data from both sensor 106 and 106b is transmitted to the receiver 108 via transmission system 110, 112, and 114. This is not a requirement. For example, if desired, sensor 106b could include its own data storage and/or transmission system for storing data and/or transmitting it to the receiver 108 (or to another remote system, such as remote system 120). Other data storage and/or transmission arrangements also are possible without departing from this invention.

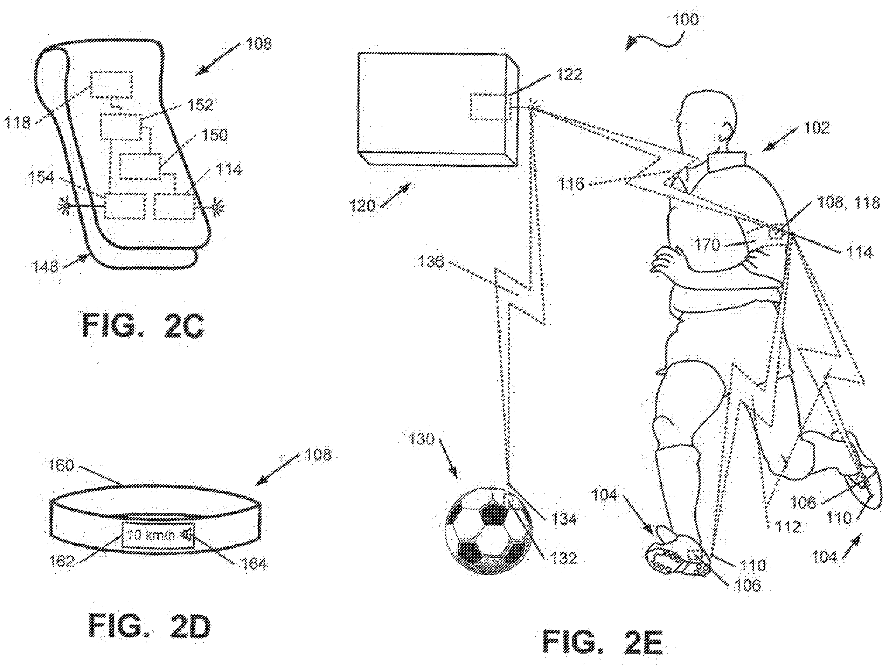

Fig. 2C schematically illustrates an example on-body receiver 108 that may be included in systems and methods in accordance with at least some examples of this invention. The receiver 108 of this example includes the data input device 114 that receives data transmissions from the shoes 104 or other remotely located sensors (*e.g.,* sensors 106, 106b, 132, etc.). This remotely generated data may be stored in a memory device 150, further processed by a processor system 152, and/or immediately transferred to output system 154 (*e.g.,* for transmission to another remote system, such as system 120). As mentioned above, receiver 108 further may include one or more of its own sensors 118, such as an accelerometer, a ball proximity detector, or other desired sensor element.

Fig. 2C illustrates the receiver 108 having a separate input device 114 and an output device 154. This is not a requirement. If desired, input may be received in and output may be transmitted from the receiver 108 using the same system (*e.g.,* an input/output system, such as a wireless transceiver). When present as a separate system, the output device 154 may take on any desired form, such as a wireless transmitter (using any desired wireless transmission technology or protocol), a computer connection port (such as a USB port or other computer connection port), or the like.

On-body receiver 108 may take on a variety of different forms without departing from this invention. For example, Fig. 2C illustrates the receiver 108 in the form of a clip 148 that may be attached, for example, to the waist band of the athlete's shorts (*e.g.,* as shown in Fig. 1). The receiver 108 also may be in the form of a wrist band, such as a watch or other wrist borne data receiving device 160, like that shown in Fig. 2D. Optionally, if desired, the receiver 108 may include an output device that provides feedback to the athlete 102 in real time, as the athletic performance is taking place (such as a display monitor 162 for alphanumeric, video, or textual output; audio output (such as speaker 164, headphone, ear bud, etc.); etc.), as shown in Fig. 2D. As another option, the output device 154 may provide output to a device for providing real time feedback to the athlete 102 (such as a display, a speaker, an earphone, etc.).

Fig. 2E shows an overall system similar to that of Fig. 1, except in Fig. 2E the receiver 108 is formed as part of an armband 170, which may be worn inside the athlete's shirt or outside the shirt. Other arrangements and mountings for sensors, such as sensors 106, 106b, and/or 118, and/or receiver 108 (if present or necessary) are possible without departing from this invention. For example, one or more of the sensor(s) or receiver may be integrated into the clothing of the wearer, such as formed in or housed within a pocket provided in the waistband of the shorts or elastic of the jersey, as part of a belt structure, etc. As additional examples, a player's shin guard may include a sensor and/or a receiver device (*e.g.,* for sensing the same type of data as sensed by the shoe borne sensor(s), such as step count, pedometer type speed and distance information, accelerometer data, ball contact data, ball proximity data, kick force, etc.). As another example, the receiver 108 or sensor(s) 118 could be included as part of a neckband, headband, or other apparel. Preferably, any body mounted sensors and/or receivers will be lightweight, durable, and positioned so as to have little or no impact on the player's performance or play and so as to have little or no possibility of injuring the player or others.

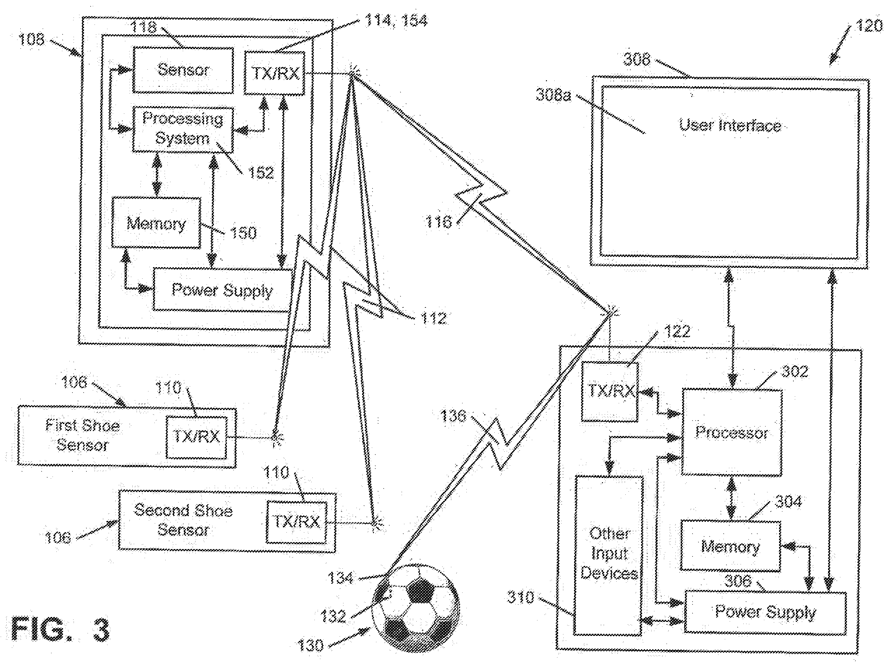

Fig. 3 illustrates additional features that may be included in systems and methods in accordance with at least some examples of this invention. In addition to the two foot mounted sensors 106 and the body mounted sensor 118 and receiver 108, Fig. 3 illustrates additional details of an example remote system 120 that may receive data transmitted from the receiver 108 and/or the ball 130 (*e.g.,* via connections 116 and 136, respectively). In addition to transmitting data from the sensors 106, 118, and/or 132, transmission connections 116, 136, and/or 112 also may be used to transmit data from the remote system 120 to the receiver 108, ball 130, and/or shoes 104, respectively (*e.g.,* to vary or control aspects of the sensors or other electronics provided in the receiver 108, ball 130, and/or shoes 104).

The remote device 120 may be, for example, portable audio and/or video players, cellular telephones, personal digital assistants, pagers, beepers, palm top computers, laptop computers, desktop computers, servers, or any type of computer controlled device, optionally a computer controlled device that generates or displays a human perceptible output and/or interface. The example remote device 120 shown in Fig. 3 includes a processor system 302 (which may include one or more processors or microprocessors), a memory 304, a power supply 306, an output device 308, other user input devices 310, and data transmission/reception system 122 (*e.g.,* a wireless transceiver). The transmission/reception system 122 is configured for communication with the receiver 108, ball 130, and/or shoe sensors 106 via transmission/reception systems 114, 134, and/or 110 through any type of known electronic communication, including contacted and contactless communication methods, such as RFID, Bluetooth, infrared transmission, cellular transmissions, etc. The output device 308 may constitute any desired type of output device that includes a human perceptible interface and/or that generates output, such as portable audio and/or video players, cellular telephones, personal digital assistants, pagers, beepers, palm top computers, laptop computers, desktop computers, buzzers, vibrators, and the like. In this illustrated example, the output device 308 includes a user interface 308a that may be in the form of a graphical user interface, such as an interface illustrating an internet website page or similar graphical depiction of data or information.

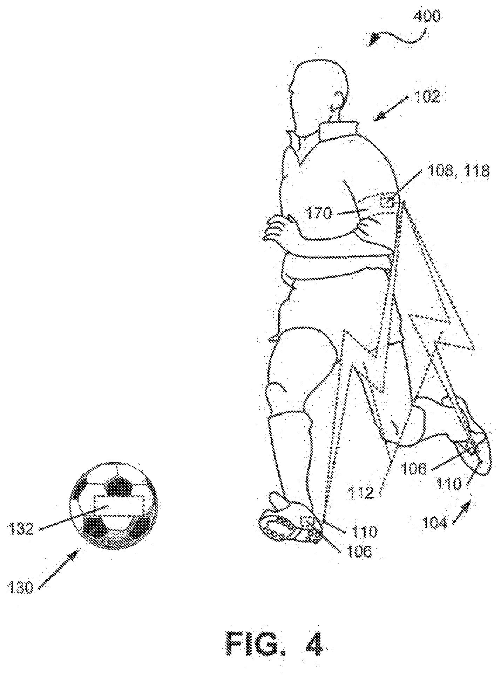

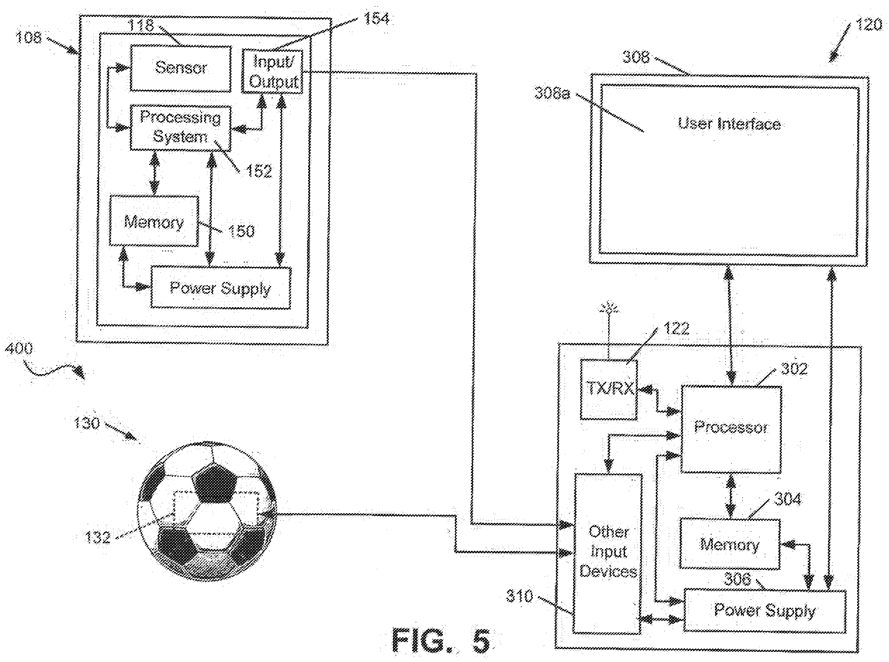

The systems illustrated in Figs. 1 through 3 are potentially active, real-time transmitting systems that provide data to the remote system 120 as the athletic activity is taking place. This is not a requirement. For example, the system 400 of Figs. 4 and 5 is much more passive than the systems of Figs. 1 through 3. As far as the hardware systems, the system of Fig. 4 is similar to those of Figs. 1 through 3 except that transmission systems 114 and 134 are removed, and receiver 108 and ball 130 function more like data loggers. More specifically, receiver 108 and ball 130 store data from sensors 106, 118, and/or 132 while the athletic activity takes place and save it for later transmission to a remote system 120, *e.g.,* for post activity analysis, review, etc. If desired, even the data transmissions 112 from the shoes 104 to the receiver 108 may be omitted, and the shoe based data could be stored locally with the shoe sensors 106 for later download.

Optionally, if desired, the receiver 108 may include some sort of display (*e.g.,* like that shown in Fig. 2D) or other output device to provide the athlete with some real-time performance feed back while the athletic performance is taking place (*e.g.,* current speed, current distance traveled, minutes played, time in possession, on-ball speed, off-ball speed, a "pick up your pace" indication or other motivation or rewards, etc.).

After play is completed, the receiver 108 and the ball 130 (or an electronic component removed therefrom including their data log) may be plugged into a remote system 120, like those described above. See Fig. 5. Any type of connection system may be used without departing from this invention, including a wireless connection, a hardwired connection, connection via an input port (such as a USB port, or the like), etc. The remote system 120 may be located on the sidelines, in the locker room, in a player's home, or at any desired location, and it may be portable or non-portable.

Given the above example hardware descriptions, now additional details of example metrics that may be measured and the use of such hardware systems will be described in more detail.

### B. Player Acceleration, Speed, and/or Movement Distance Sensing

Movement speed is one metric that is particularly important for gauging an athlete's performance. Systems and methods in accordance with at least some examples of this invention may measure the player's movement speed in various ways. For example, the sensor 106 in one or more of the athlete's shoes 104 may be adapted to measuring acceleration, speed, and/or distance information, *e.g.,* in a manner akin to the way NIKE+ athletic performance monitoring systems and other pedometer based sensor systems monitor speed and distance information. For example, the sensor 106 may be an accelerometer, a pressure sensor (*e.g.,* a piezoelectric sensor), or other force sensor that determines each time the player's foot hits the ground or other data associated with foot motion. By assuming that each foot contact constitutes a step, and by assuming each step covers a specific distance, the number of foot contacts may be correlated to an overall distance the athlete traveled. If desired, the distance for each step also may be adjusted based on various sensed factors, such as foot loft time between ground contacts, foot impact force, and the like, *e.g.,* in manners that are known and used in the pedometer art. Also, by monitoring the time associated with the movements (*e.g.,* by including a time stamp with each monitored foot contact, by tracking overall use time, etc.), the overall athlete's speed may be determined.

Pedometer based speed and distance measurement, however, may not always provide the desired degree of accuracy for use in many team oriented sports. For example, in soccer, football, basketball, rugby, and the like, athletes tend to move at widely varying speeds over the course of a game or practice session. They also tend to frequently jump vertically, dive, and otherwise leave their feet during play. Moreover, their feet are exposed to forces from sources other than contact with the ground, such as kicking the ball, kicking and hitting another object, etc. These additional features of many team sports may limit the accuracy of pedometer based speed and distance measuring systems.

Accordingly, systems and methods in accordance with at least some examples of this invention may include a body core mounted speed and/or distance measuring device. This may come, for example, in the form of an accelerometer mounted at the core of the athlete's body, such as in a waist band mounted accelerometer sensor (*e.g.,* a two or three axis accelerator sensor 118, which may be included as part of receiver 108 to determine motion in two or three dimensions). Data generated by an accelerometer sensor 118 *(i.e.,* the acceleration of the player at the location of mounting, such as the body's core or waist) may be integrated to provide the athlete's movement speed information, and it may be integrated again to provide the athlete's movement distance information. A body mounted sensor of this type may provide more accurate determination of the body's motion, *e.g.,* when moving side to side, dancing around the ball, etc. Systems and methods for measuring acceleration and integrating the data obtained from an accelerometer are known.

Acceleration, speed, and/or distance determinations may provide useful data and information in several ways and for several performance metrics in systems and methods in accordance with this invention. For example, this data may be useful in determining the following metrics, which may be of interest to participants in team sports, such as soccer, basketball, American football, rugby, and the like: overall top acceleration, average acceleration, overall top running speed, average running speed, overall top running speed when in possession of the ball, average running speed when in possession of the ball, overall top running speed when not in possession of the ball, average running speed when not in possession of the ball, number of times speed exceeded a predetermined speed threshold (*e.g.,* the number of times the athlete sprinted), overall distance traveled during the game, etc. This data can help the players (and/or their coaches) evaluate how hard the athlete is working, how much effort he or she is putting in to the game, how they are improving over time, the extent of recovery from injury, etc. This data also can be used to foster competition among individuals, such as team members, *e.g.,* to provide motivation to work harder, improve, beat the other player's metrics, etc.

If desired, the body core based sensor (*e.g.,* sensor 118 as part of receiver 108) may be the only sensor necessary for determining acceleration, speed, and/or movement distance determination. Therefore, if desired, the foot based sensors 106 could be eliminated. Nonetheless, if desired, the foot based sensors 106 could be used to provide secondary data for speed and/or distance measurement, such as data to help confirm the body core based sensor data, data to adjust or correct the body core based sensor data, and/or data to be used when the body core based sensor data is unavailable or seemingly unreliable. Additionally or alternatively, if desired, the shoe based sensor(s) 106 could be used to help eliminate drift of the body mounted accelerometer (*e.g.,* if the shoe based data indicates that the player is stationary, this information could be used to calibrate or re-zero (*e.g.,* eliminate drift from) the two or three axis body based accelerometer). The relative difference in acceleration measurements between a body core based accelerometer and a foot based accelerometer also may be determined.

As another alternative, at least some systems and methods in accordance with this invention may include a means of detecting the player's orientation or "mode of moving" when moving. For example, if desired, an electronic compass or a rotational sensor may be incorporated into the system, *e.g.,* to aid in detecting a player's direction of movement and/or to provide additional details regarding the characteristics of the player's mode of movement (*e.g.,* running forward, running at a side step, running backward, etc.). An accelerometer also can provide useful information regarding the direction of movement, if the accelerometer has a predetermined orientation at the start (*e.g.,* with one axis of a two or three axis accelerometer facing the forward direction of motion). A determination of the amount of time or distance that a player runs forward, sideways, or backward could be a useful metric for measuring performance, at least in some sports. Also, if desired, different pedometer based speed and distance determination algorithms may be used, depending on the player's mode of movement (forward, backward, sideways, etc.), which may enable a more accurate determination of the player's overall movement speed or movement distance. More specifically, one algorithm may be appropriate for determining speed or distance (*e.g.,* based on foot loft time, etc.) when a player is running forward, but a different algorithm may be better when running sideways, and even a different algorithm may be better when running backward.

In one more specific example of systems and measurements in accordance with this invention, one footpod (e.g., element 106, optionally one in each shoe 104) measures speed and distance of each step, e.g., utilizing a 3-axis accelerometer, and the collected data may be stored on the footpod 106 during a match or training session. A separate controller or a mobile phone (or other suitable device) may be used to communicate with the footpod 106, e.g., for the purpose of ascertaining footpod status, for starting/pausing/stopping recording of a session, and for initiating an upload of data (e.g., to computer system 120). In systems where a separate controller is used for these purposes, the user would need to connect the controller to his/her computer to upload their data, e.g., to a website service. In the case of a mobile phone (or other similar device) functioning as the controller, the phone could temporarily store the data and/or send the data directly to a web server wirelessly. Variations in these potential systems also are possible without departing from this invention.

Notably, for purely determining an athlete's acceleration, speed, or movement distance, no sensors, electronics, or other special features are needed in the ball. Therefore, if desired, a conventional ball could be used in such situations. In other situations and/or for measuring certain metrics, which will be described in more detail below, it may be advantageous to provide sensors, electronics, and/or other specialized structures in the ball.

### C. Player Ball "Possession" and "Proximity" to the Ball Detection

Another useful piece of information for many types of team sports relates to a player's ball possession time. This may be measured, for example, by detecting an athlete's contact with the ball (*e.g.,* by a hand, foot, or other body part), an athlete's close proximity to the ball, or in other manners. Determination of ball possession or proximity to the ball also can be an important part of other interesting or desired metrics, such as possession time, overall top running speed when in possession of the ball, average running speed when in possession of the ball, overall top running speed when not in possession of the ball, average running speed when not in possession of the ball, number of times near the ball, number of ball contacts or "touches," kick force, etc. This data can help the players (and/or their coaches) evaluate how hard the athlete is working, how much effort he or she is putting in to the game, which players are the most effective with the ball, which players work hardest to stay near the ball, the strongest defenders, the ball "hogs," etc. This data also can be used to foster competition among individuals, *e.g.,* to provide motivation to work harder, improve, beat the other player's metrics, etc.

In some team sports where the ball is held throughout at least most of its possession, ball possession for an individual player can be relatively easy to determine, *e.g.,* by determining which player is contacting the ball and/or by determining how long the player held the ball. One example is American football or rugby. Similarly, in lacrosse, the ball tends to rest in the head of the player's stick throughout the majority of the player's possession time. For such sports, appropriate sensors in the ball and/or on the player and/or on their equipment can relatively easily determine who has possession and the length of time of that possession. As one more specific example, RFID receivers or readers in an athlete's clothing or equipment (such as gloves, a jersey, helmet, pads, stick, shoes, etc.) may be triggered by an RFID transmitter tag mounted in or on the ball, and electronics included with the athlete's clothing or equipment may log how long each individual possession lasts. By time stamping or otherwise providing time data associated with this possession data, the possession data could be correlated to acceleration, speed, and/or movement distance data (*e.g.,* determined as described above), to allow systems and methods in accordance with this invention to determine more specialized metrics, such as overall top running speed when in possession of the ball, average running speed when in possession of the ball, overall top running speed when not in possession of the ball, average running speed when not in possession of the ball, etc. While other players also may come in contact with the ball during an individual play, this contact typically is relatively short term, and it typically is overlapped by and/or surrounded on each end by contact with the main player in possession. Therefore, the data can be easily analyzed to determine which contacts simply constituted a fleeting, non-possessory contact and which contacts actually demonstrated possession of the ball. Alternatively, if desired, multiple players could be considered to simultaneously have "possession" of the ball by systems and methods according to this invention (*e.g.,* if "possession" is simply equated to any contact with the ball).

In other sports, however, continuous contact with the ball is not a feature of ball "possession." For example, in soccer and basketball, a player in "possession" "dribbles" the ball to move it up and down the field of play, resulting only in occasional contact with the ball. The ball is not typically held for long periods of time or carried for long distances in such sports. In hockey and field hockey, the ball (*e.g.,* including a hockey puck) may repeatedly come into and out of contact with the player's stick while the player in possession of the ball moves down the field of play. Also, a player in "possession" of the ball may only make contact with the ball once, sometimes for only a very short time period (*e.g.,* when a quick pass or shot is made). Moreover, in all of these sports (*e.g.,* soccer, basketball, hockey, field hockey), players on the opposing teams may attempt to steal the ball or puck throughout a player's possession. Such features of play make ball "possession" somewhat more difficult to determine using sensors.

Systems and methods in accordance with at least some examples of this invention may approximate a player's ball "possession" using various features of proximity of the player to the ball. While the description below primarily focuses on possession determination in the context of soccer, those skilled in the art, given the benefit of this disclosure, would be capable of extending features of this description for use in other sports, such as basketball, hockey, field hockey, American football, rugby, lacrosse, and the like.

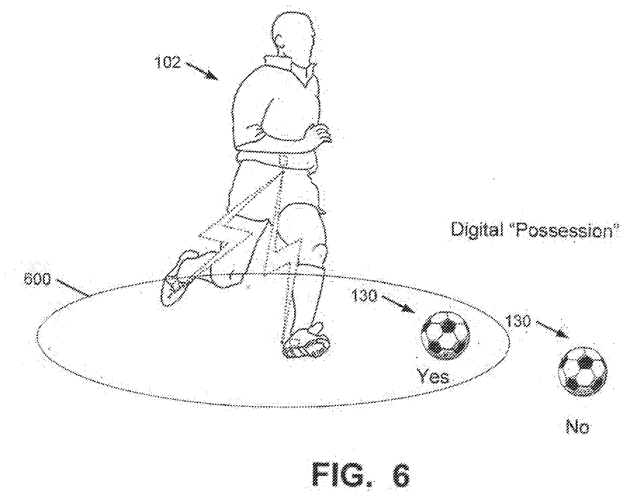

Determination of "possession" may include various features. For example, systems and methods in accordance with at least some examples of this invention may determine that "possession" exists whenever a player contacts or comes within a certain threshold distance from the ball (*e.g.,* within one meter). As illustrated in Fig. 6, such systems may be thought of as "digital" possession determining systems, where a player either has possession or does not have possession. More specifically, when the ball 130 is within a one meter distance of the player 102 (inside ring 600), the player 102 may be considered as having "possession." When the ball 130 is more than a one meter distance from the player 102 (outside ring 600), the player 102 may be considered as not having "possession." In such systems and methods, multiple players may be considered as having "possession" at a single time (when each player is within close proximity to the ball). When multiple players from different teams are located in proximity to the ball, this also may be considered "contested time," as is described in more detail below.

Optionally, if desired, a positive determination of "possession" may require at least one contact with the ball (and optionally, the "possession" determination may start at that contact). As another option, systems and methods according to the invention may track both "possession" (*e.g.,* requiring at least some contact and/or continuing contact with the ball) and "proximity" (*e.g.,* when there has not been contact but the player is close to the ball or when a different player has made an intervening ball contact but the first player remains close to the ball, etc.). If desired, a new "possession" determination may be made each time a different player contacts the ball (although the previous player in contact may remain close to the ball and his or her "proximity time" may continue to accumulate). As noted above, "proximity" may be simply equated to "possession" in some systems and methods according to this invention.

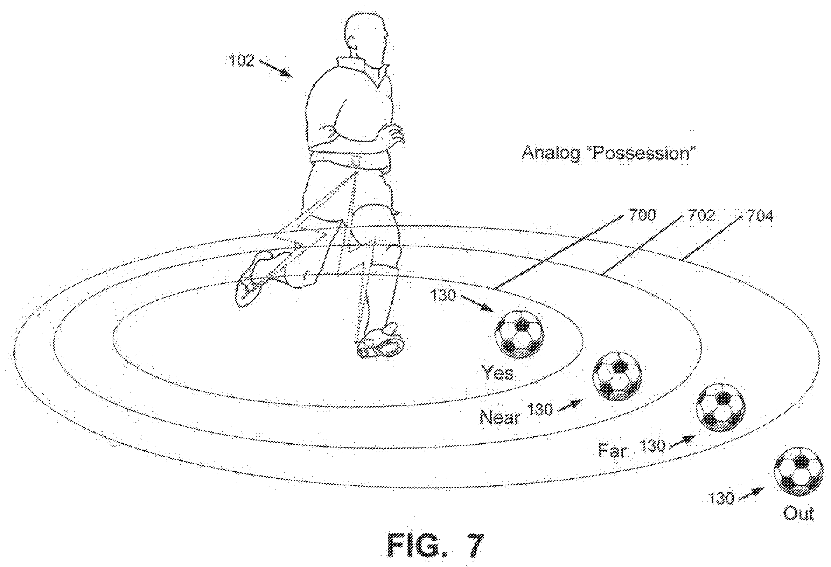

"Possession" also may be considered as more of an "analog" parameter. For example, systems and methods may be produced to provide a more detailed determination of the proximity of a player to the ball. For example, as shown in Fig. 7, determination of the player's distance from the ball may be more closely determined, to better enable a determination of "possession." For example, when the player 102 is very close to the ball 130 (*e.g.,* within inner ring 700), that player may be considered in "possession" of the ball 130 (if desired, multiple players may have "possession" at one time). When the player 102 is relatively close to the ball 130 (*e.g.,* within ring 702 but outside ring 700), the player 102 also may be considered to be in possession of the ball, optionally, if other parameters are met (such as if the player 102 was the last person to touch the ball 130 or the player 102 is the closest player to the ball 130, and there has been no intervening ball contact by another player, etc.). When the player 102 is somewhat close to the ball 130 (*e.g.,* within ring 704 and outside ring 702), the player 102 also may be considered to be in possession of the ball 130, optionally, if other (optionally, more stringent) parameters are met (such as if the player 102 was the last person to touch the ball 130, the player 102 is the closest player to the ball 130, and there has been no intervening ball contact by another player, etc.). Any desired possession parameters may be developed without departing from this invention. When the player is too far away from the ball 130 (*e.g.,* outside ring 704), systems and methods according to at least some examples of the invention may determine that the player 102 does not possess the ball 130. Optionally, systems and methods according to at least some examples of this invention may determine that a player remains in "possession" of the ball until a new player contact with the ball is ascertained, irrespective of the previous player's location with respect to the ball.

**1. RFID Technology**

One potential way of determining ball possession or proximity is through the use of RFID (radio frequency identification) technology. RFID systems use coupled energy to transmit a small amount of data between an interrogator (also known as a "reader") and a remote, inexpensive tag. The tag can be stationary or in motion with respect to the reader. Such RFID systems can be categorized according to two main criteria, namely: the means of powering the tag (*e.g.,* passive, semi-passive, or active) and the energy coupling mechanism (*e.g.,* inductive or radiative).

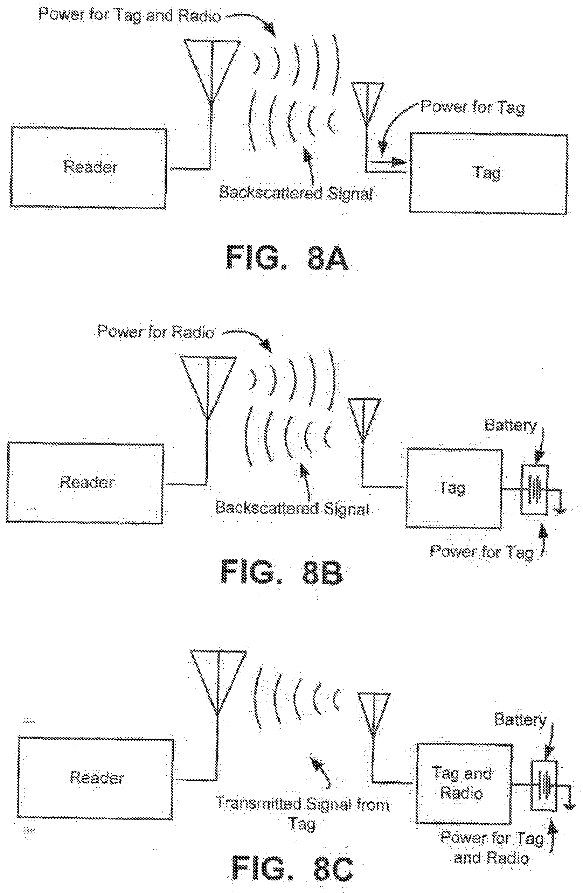

Figs. 8A through 8C schematically illustrate various RFID technologies. In the "passive" RFID system illustrated in Fig. 8A, power for both the tag and the return radio signal *(i.e.,* the "backscattered signal" in Fig. 8A) generated by the tag are provided by energy recovered from the reader signal. Such a completely "passive" system may be advantageous in the environment of this invention because it could eliminate the need for a power source (e.g., a battery) on the ball. In "semi-passive" RFID systems, as illustrated in Fig. 8B, power for the return radio signal is provided from recovered reader energy signal, but the tag electronics are powered by a small battery included with the tag. The "active" RFID system illustrated in Fig. 8C is really akin to a traditional radio system. The tag radio signal and the electronics are both powered by a local battery provided with the tag (and the reader's electronics are powered by its own separate power source).

Radio tag frequencies range from a few hundred MHz to several GHz. In this spectrum, wavelengths become comparable to the mechanical scale of personal electronics and more specifically, full wavelength antenna sizes. Such features allow far-field operations where power varies inversely with the square of the distance from the source.

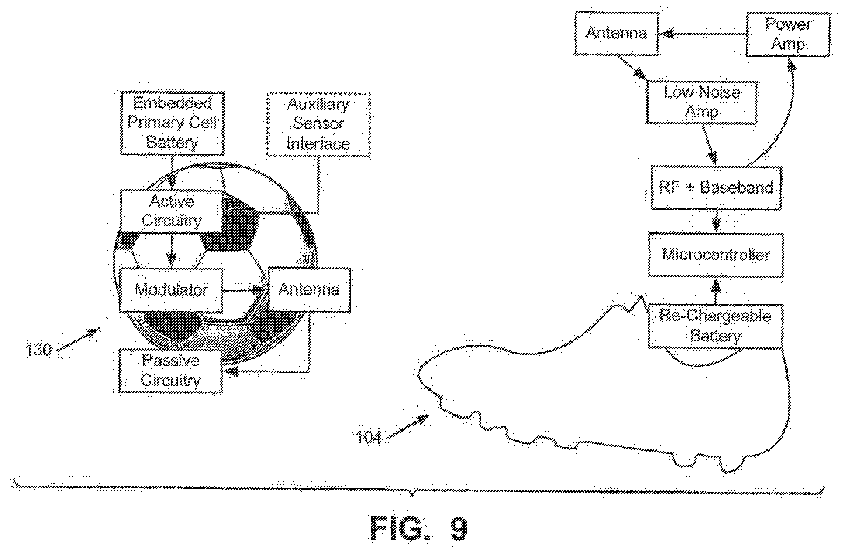

Fig. 9 illustrates one example of the hardware and equipment that may be used in a semi-passive RFID system to detect player proximity for soccer or other sports. Notably, in the system illustrated in Fig. 9, the ball 130 includes the RFID tag and its associated antenna and other electronics, and the shoe 104 (or other article of the player's equipment, such as a shin guard, sock, receiver 108, etc.) includes the RFID reader and its associated antenna and other electronics. More specifically, the ball 130 of this example carries an embedded primary cell battery, an auxiliary sensor interface, active circuitry, a modulator, passive circuitry, and an antenna. The player (*e.g.,* the shoe 104) in this example system carries a re-chargeable battery, a microcontroller, an RF + baseband component, a low noise amp, a power amp, and an antenna. The battery assist on the ball mounted tag permits a relatively low-received power density, which effectively lowers the transmission power required on the player (and lowers the mass of the necessary battery and other electronic equipment to be carried by the player). A single ball 130 may include multiple tags on the ball (*e.g.,* to assure that a tag antenna is always facing the player's reader, to enable more sensitive distance measurement, such as for analog possession determinations, etc.). RFID tag and reader equipment of this type is conventionally known and commercially available.

Proximity detection of this type may be combined with data relating to foot contact with the ball, if desired, to distinguish between ball possession and ball proximity. Alternatively, as noted above, possession may simply be equated with proximity, if desired.

**2. DPR Technology**

Digital packet radio ("DPR") also may be useful in determining ball proximity and/or "possession" (optionally, in conjunction with other data, such as foot and/or ball contact data) in systems and methods according to at least some examples of this invention. Many NIKE+ athletic performance monitoring products (available from NIKE, Inc. of Beaverton, Oregon) use DPR for wireless data communications (*e.g.,* in the 2.4 GHz band). DPR also is used in many commercially deployed networks, such as cellular networks, WiFi (802.11), ZigBee, and PCS. Two example chipsets that may be used for implementing DPR based proximity and/or possession determinations in systems and methods according to this invention include chipsets available from Nordic Semiconductor Inc. of Sunnyvale, California and ANT Wireless of Cochrane, Alberta, Canada. Both companies make ultra low-power radio silicon chipsets that can be used in a variety of applications. The radio chipsets can be powered by a standard coin cell type battery with excellent device lifetimes.

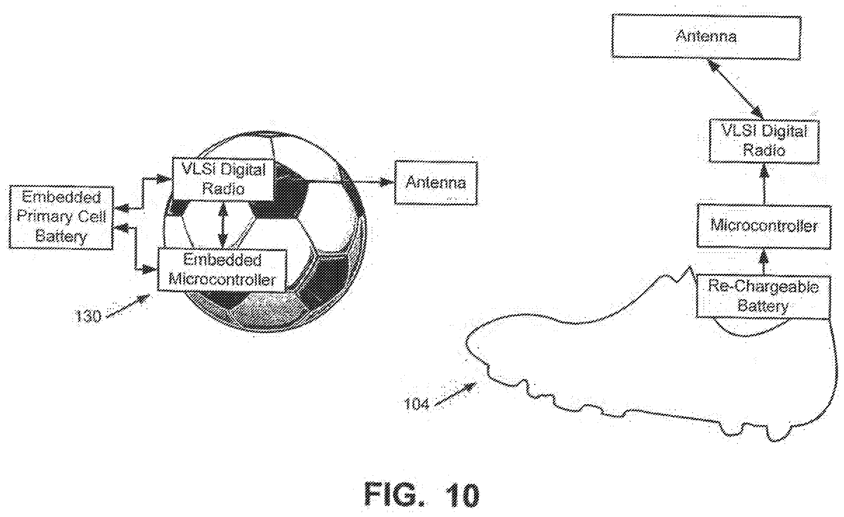

DPR implementations for proximity and ball possession determinations offer low-power, high range systems and methods. Fig. 10 illustrates one example system. Notably, while these systems and methods are low power and high range, they still require an active receiver end *(i.e.,* some electronics and/or power on the ball 130), as shown in Fig. 10. In the DPR system of Fig. 10, the ball carries an embedded power source (*e.g.,* primary cell battery), an embedded microcontroller, a very large scale integration ("VLSI") digital radio system (*e.g.,* a chip), and an antenna. The athlete (*e.g.,* as part of the shoe 104 or receiver) carries a re-chargeable battery, a microcontroller, a VLSI digital radio system (*e.g.,* a chip), and an antenna. The DPR system may operate on any desired frequency, such as 915 MHz or 2.4 GHz. Such hardware systems are known and are commercially available, as noted above.

In the ball 130, the small radio and the microcontroller trigger radio bursts that send out unique identifying data packets. The trigger for each radio burst could be periodic (*e.g.,* every 50 ms, every second, etc.). On the other hand, the trigger could be aperiodic, such as in response to an actual event trigger, like motion, contact, impact, etc. These packets allow a body-worn receiver on the player 102 (*e.g.,* in boot 104, in a body core worn element, etc.) to log received data that directly correlates to how long the ball 130 spent within proximity to the receiver. This proximity may be correlated to ball possession (optionally, if another metric is logged, such as contact between the player's foot and the ball 130, as determined by a shoe based sensor 106b). This is a very "digital" possession type determination system. If desired, as noted above, possession may be equated to proximity.

DPR also may be used to provide more analog possession information. In such a system, the ball 130 may serve as the receiver, and the body worn device may provide the bulk of the transmissions. In such as system, the ball 130 would periodically listen for a radio packet broadcast from the body worn transmitter. The body worn-transmitter could send out bursts of packets at different set output powers. The ball 130 would only receive packets from the weakest transmitted signals when it is in close proximity to the player 102. The number of signals received by the ball 130 will decrease the further that the ball 130 is away from the player 102 sending the signals until it is receiving only the strongest signals or none at all. The ball 130 may respond to any received packets by transmitting back with a unique identifier derived from the packets it received (*e.g.,* an identifier indicating the transmission power). This arrangement allows the body worn receiver to determine how far away the ball 130 is based on the weakest signal that is received at the ball 130 and for which a response was sent. Alternatively, if desired, the ball could send out the bursts of packets at different output powers and the body worn sensor could receive these packets and determine the relative distance between the ball and the body sensor based on the detected signals (and their corresponding power levels).

With DPR systems, because there is an active radio at each end, *i.e.,* at the ball 130 and at the player 102, the transmission power can be quite low (and smaller than other technologies), but, as noted above, it does require some power source on the ball. DRP also provides the ability to dynamically vary output power, giving systems and methods in accordance with at least some examples of this invention the ability to estimate the range between the ball 130 and the player 102, and/or even the ability for the player's system to acquire the ball outside of some predetermined "possession" distance (*e.g.,* one meter).

### 3. RADAR Technology

Ball possession and/or player proximity to the ball also may be detected in some example systems and methods according to this invention by RADAR technology ("RAdio Detection And Ranging"). RADAR systems use reflected radio "ping" energy to identify and locate target objects by analyzing their reflected "signature." RADAR systems do not require active transmission in two directions, which means that the ball need not include an active transmitter or a power source in at least some RADAR based proximity or possession determination systems and methods in accordance with this invention. If desired, however, RADAR based systems could rely on an active (power utilizing) systems as part of the ball to generate a radio "ping" for the mobile detector to recognize, or they may in some way (*e.g.,* actively powered or passively unpowered) enhance or distinguishingly mark the reflected energy to ease the mobile transponder's job of identifying the ball from the clutter of background noise, other reflections, and/or miscellaneous distortions in the signal. Additionally, the RADAR may apply modulations and other processing techniques to the transmitted energy and reflected signature to measure other attributes of the target object, such as the velocity of the target object (*e.g.,* by using Doppler techniques).

Passive RADAR systems, in which the ball does not include a power source, can rely on one or more other RADAR reflection techniques to increase the "visibility" of the reflected signal from the ball (*e.g.,* by increasing its gain or coherence). One example of such a technique would be to provide a reflective device on the ball, such as a corner reflector. As one example, the corner reflective material could be provided within one or more seams of the ball, or optionally in an interior layer of the ball (if the impinging radiation is capable of penetrating the ball's exterior cover). Corner reflectors are known in the RADAR and other art, and these devices reflect radiation outward from the reflector in substantially the opposite direction from which it entered the reflector (*i.e.,* directly back toward the radiation source and/or parallel to its incoming direction). Another example technique would be to provide "chaff" on or in the ball structure.

"Chaff" constitutes specifically sized small pieces of RADAR reflective material organized in a unique pattern on the ball that is easily recognized by the RADAR detection system. Such reflectors and chaff are well known in the RADAR field, and are sized and shaped in suitable configurations so as to be capable of incorporation into the structure of a ball (such as a soccer ball, hockey puck, basketball, or the like). These features increase the RADAR reflection signature from a ball and make the ball better stand out among the other RADAR radiation reflected from other objects in the area (such as other players, other equipment on or near the field, goal posts, etc.).

Another technique for helping a passive (non-powered) ball's radiation reflection signature stand out among other objects involves the use of a passive frequency doubler structure on the ball. A passive frequency doubler works using a principle similar to "square law" detectors. Non-linear devices can generate frequency harmonics when stimulated with a signal. A diode, at small signal levels (*e.g.,* equivalent to less than -20dBm) has a VI relationship that is roughly $I = k*V^2$, where k is some constant. Such a device is capable of generating a frequency harmonic that is twice that of what is used to drive the diode, by the equation: $\cos(f_0)^2 = ½ + ½*\cos(2*f_0)$, where $f_0$ is the input frequency. This frequency harmonic can be radiated out of the same antenna that received the fundamental frequency. Harmonics other than the doubled frequency ($2^{nd}$ harmonic) will also be generated by the non-linear device, so this application of detecting frequency harmonics is not limited to only the doubled frequency, but can also detect 3 times the frequency, 4 times the frequency, 5 times the frequency, etc.

By providing a passive frequency doubler structure on the ball in such a RADAR system, the reflected radiation detector or receiver only needs to listen for a signal at twice the carrier frequency that its associated transmitter radiated. This doubled frequency signal will be known to be unique to the object carrying the passive frequency doubler *(i.e.,* the ball in this arrangement). In addition, the frequency doubler also generates a DC component, which may be used to power a small amount of electronics on the ball. These electronics could modulate the signal that the frequency doubler radiates, essentially giving the ball a unique ID. On the other hand, the signal that is radiated can be coded (with a barker code or a pseudorandom sequence), and then auto-correlated with the returned signal for an additional signal processing gain. Another simple method that may be used for processing gain would be frequency chirping.

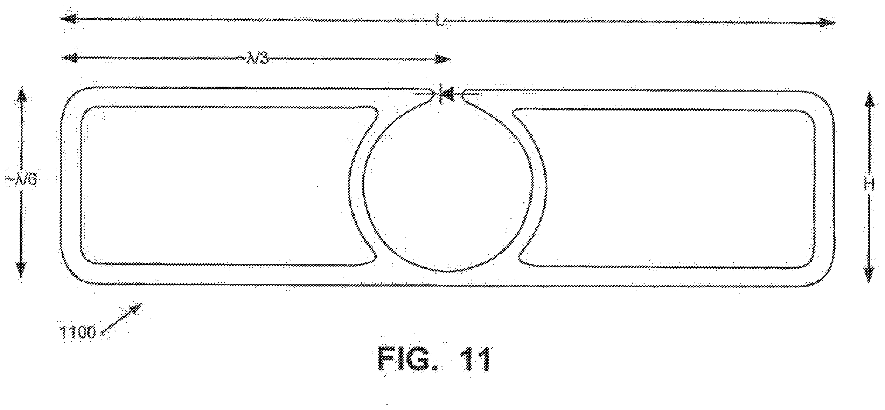

Frequency doubler antennas of the types described above are known, as described for example, in U.S. Patent No. 4,890,111, which patent is entirely incorporated herein by reference. One example antenna 1100 as described in this patent is illustrated in Fig. 11. The dimensions of such an antenna may be about 2/3 of the wavelength $\lambda$ of the transmitted and incident radiation frequency in the length dimension L and about 1/6 that wavelength A in the height dimension H. With such an antenna incorporated in to the structure of a soccer ball (*e.g.,* on its exterior surface, between layers of the ball, within the ball interior, etc.), the mobile receiver could be configured to "listen" for a specific carrier frequency (*i.e.,* twice the transmitted frequency) to detect the presence of the ball, *e.g.,* when enough energy is present in both the forward and return path from the player mounted radiation transmitter, to allow the radiation to reach the ball and bounce back to the player mounted radiation detector. As some more specific examples, the initially transmitted RADAR frequency may be 915 MHz, and the reflected frequency may be doubled to 1830 MHz. Another good candidate is 433 MHz (doubled to 866 MHz). The use of other frequencies also is possible without departing from this invention.

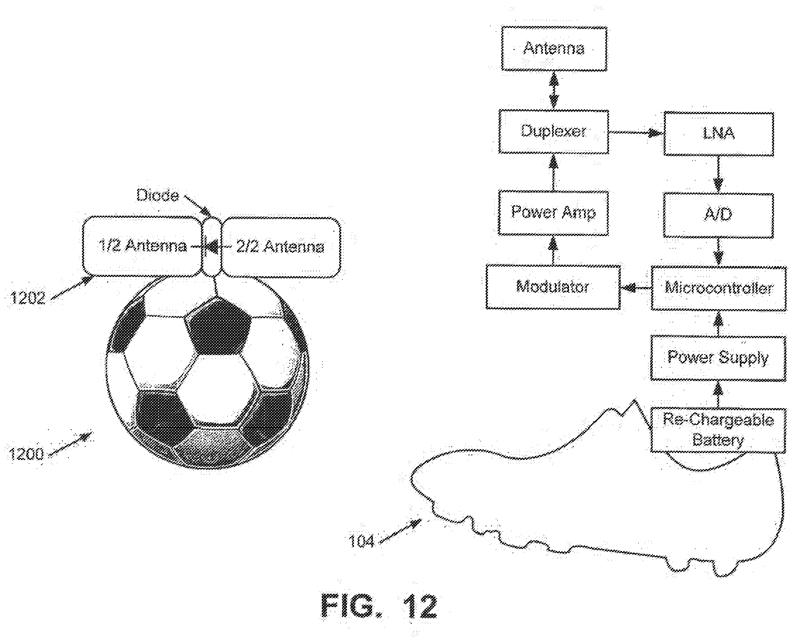

Fig. 12 illustrates example structures that may be provided on both the ball 1200 and the player 102 (e.g., as part of the player's shoe 104, as part of receiver 108, etc.) in accordance with at least some RADAR based proximity detection systems in accordance with this invention. As shown in Fig. 12, the ball 1200 includes an antenna structure 1202 like that described above in conjunction with Fig. 11. The shoe 104 (or other player borne component) includes a rechargeable battery and/or other power supply, a microcontroller, a modulator, a power amp, a duplexer, an antenna, a low noise amplifier (LNA), and an analog to digital converter (A/D). The shoe 104 transmits radiation toward the ball 1200 at a first frequency (e.g., 915 MHz), and the ball 1200 doubles the frequency through antenna 1202 and reflects the radiation back toward the shoe 104, where it can be detected. The ball 1200 may include plural antennas all around the ball structure to assure that at least one antenna faces the receiver on the player.

As an alternative, if desired, one or more RADAR radiation sources may be independent of the player (e.g., located on the sidelines or at other locations, to cover the entire field, etc.). In such a system, the player 102 need only carry the reflected radiation detector (and its associated power source and electronics), and not the radiation transmission source. The ball electronics may be configured to send out radiation only at a desired power level so that a player mounted detector would only detect the reflected radiation from the ball when in relatively close proximity to the ball (e.g., within 1 meter, etc.).

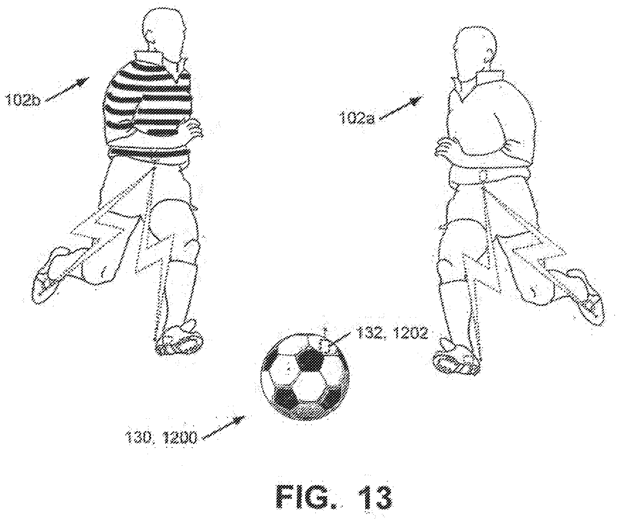

Various features may be provided to help prevent "packet collisions" when multiple players are using systems and methods in accordance with this invention, e.g., to help prevent one player from detecting radiation reflected from the ball transmitted by a different player. For example, as shown in Fig. 13, two players, one player 102a from one team and one player 102b from the other team (or even more players), may approach the ball 1200 simultaneously. If each player is equipped with actively transmitting RADAR or other data transmitting systems and methods according to the invention, the various detectors or sensors could easily read the wrong data and incorrectly determine position or proximity data. Such data "packet collisions" should be avoided to provide more reliable and usable data for systems and methods according to this invention.

One way of limiting or eliminating "packet collisions" constitutes a timing plan where each player's device transmits at a random interval, with a standard mean interval in place. This would make it unlikely that any two or more players would be transmitting at a given time in proximity to the ball, but that all players would have the same overall transmit rate. In such a system, a player's reflected radiation detection system could be activated only for a short time after his or her device transmits its radiation "ping" or data transmission, to help prevent unwanted data reception and sensor activation. One potential downside, however, would be that such a technique could potentially limit a single device's ability to detect the ball quickly, due to limitations on the average transmit rate (i.e., due to delays between transmissions).

Another method for limiting or eliminating "packet collisions" would be to "channelize" the devices on each player. Because the passive frequency doubler can operate on many frequencies in a narrow band, each player could use a slightly different frequency within the same broader band. Then, each player's detector could be tuned to "look" within a narrow band around two times the transmitted frequency. Such "channelization" also could be used to distinguish one team's data from the other team's data during the game or other activity. Other "collision avoidance" techniques also may be utilized without departing from this invention. Collision avoidance features also may be used with other proximity and possession systems and methods described above, if desired, without departing from this invention.

**4. Other Potential Proximity/Possession Detection Technology**

Other sensing systems and detection arrangements may be used for determining proximity and/or ball possession without departing from this invention. For example, ultrasound based proximity detection may be utilized, particularly for very close range ball proximity detection applications. Ultrasound systems may work using reflected radiation techniques similar to the RADAR techniques described above. Infrared radiation detection systems (both passive and active systems) may be utilized to detect ball proximity. Micro-Electro-Mechanical ("MEMs") devices, such as accelerometer and/or gyroscope devices (e.g., fabricated using semiconductor lithographic processes) also may be incorporated into a ball. Hall-effect sensing may be used with magnets in either the ball or shoe to detect proximity, particularly for short range applications. The inclusion of such devices in a ball may be particularly useful as adjunct sensors, e.g., to help determine when a ball has been kicked, and optionally, which player made the kick (e.g., by time stamping the data relating to the sensed contact in both the ball data and the various player's shoe data, etc.), kick force, kick speed, etc.

Some more specific examples are described below. One or more of these possession determination systems and methods may be used in conjunction with one or more of the speed/distance measuring systems described in more detail above.

In some systems and methods in accordance with examples of this invention, a magnet may be suspended in the center of the ball 130, and the footpod 106 may be equipped with a magnetometer (e.g., a compass sensor that measures Earth's magnetic field). This system may function, for example, by detecting small changes in the Earth's magnetic field due to the magnetic field emitted by the ball 130, which indicates the ball 130 is within a certain distance of the player's foot (and hence a certain distance from the magnetometer of the footpod 106). From this type of detection, physical contact with the ball and/or close proximity of the athlete to the ball may be inferred.

As another more specific example, one or more small tags may be built into the construction of the ball 130. A signal would be emitted by a sensor/receiver on the player. When the tag in the ball 130 receives the signal, it bounces it back at exactly double the frequency (e.g., using the frequency doubler features built into the ball as described above). The receipt of this doubled frequency signal by the sensor/receiver (e.g., in the footpod 106) indicates the ball 130 is within a certain range of the sensor/receiver (e.g., dependent on the strength of the initial signal). As some even more specific examples of this aspect of the invention, a sensor/receiver may be placed in both of the player's shoes 104 and have a short required working range (e.g., about 30cm). In this case, each detection of the ball 130 would infer a physical contact with the ball 130 by the player's shoe 104. Alternatively, if desired, a sensor/receiver may be located in just one of the player's shoes 104 or on the player's body (such as waist-worn component 108) and have a larger working range (e.g., about 1-2m). In this case, each detection would infer proximity of the player to the ball 104, or that the player is in possession and control of the ball 104.

Moreover, in a similar manner to the way player-to-ball proximity may be measured, systems and methods according to at least some examples of this invention may determine player-to-player proximity. As another more specific example, modules carried by each player may wirelessly communicate with one another when within a predetermined distance or range from one another (e.g., via a peer-to-peer network) to provide an indication of player-to-player proximity.

**D. Ball Motion Related Metrics**

Other useful metrics for many types of team sports relates to the speed at which the ball moves during play, *e.g.,* as a result of a kick, throw, hit (*e.g.,* with a bat, stick, arm, foot, racket, etc.), etc. More specific types of metrics that may be of use include, for example, ball speed, ball spin, linear ball speed, spin speed, spin direction, ball transfer speed (the term "transfer," as used in this context, generically means movement of the ball due to athlete interaction, such as a kick, throw, hit, header, etc.), ball transfer force, etc. Combining ball oriented metrics like these with various player oriented metrics (e.g., due to shoe or player body oriented sensor data and interaction between shoe or body oriented electronics and ball oriented electronics, as described above) or other data, such as possession, speed, time, etc., can provide other useful information, such as the identification of the player that kicked or otherwise propelled the ball, number of ball "touches" or contacts for various specific players, goal success and credit to the appropriate player, pass attempt success (e.g., whether the pass successfully reached a player on the same team), steals, missed passes, turnovers, etc.

Providing sensors in various types of balls, such as soccer balls, is known in the art. For example, various electronically enhanced balls that measure metrics, such as spin, speed, curve, trajectory, pressure, contact, and the like, are described in patent applications owned by Cairos Technologies, AG and in patents naming David J. Martinelli as the inventor. These patents include: U.S. Patent No. 6,073,086; U.S. Patent No. 6,157,898; U.S. Patent No. 6,148,271; U.S. Patent No. 6,151,563; U.S. Published Patent Appln. No. 2007/0191083; U.S. Published Patent Appln. No. 2007/0059675; U.S. Published Patent Appln. No. 2007/0060425; U.S. Published Patent Appln. No. 2007/0299625; U.S. Published Patent Appln. No. 2008/0085790; U.S. Published Patent Appln. No. 2008/0084351; U.S. Published Patent Appln. No. 2008/0088303; U.S. Published Patent Appln. No. 2008/0090683; PCT Published Patent Appln. No. WO2008/080626; PCT Published Patent Appln. No. WO2008/104,247; and PCT Published Patent Appln. No. WO2008/119479. Each of these patent documents is entirely incorporated herein by reference. The various ball oriented sensors or other electronics or structures described in the possession/proximity discussion above may be incorporated into a ball structure in the same manner as described in these various patents and publications.

### E. Example Team Features

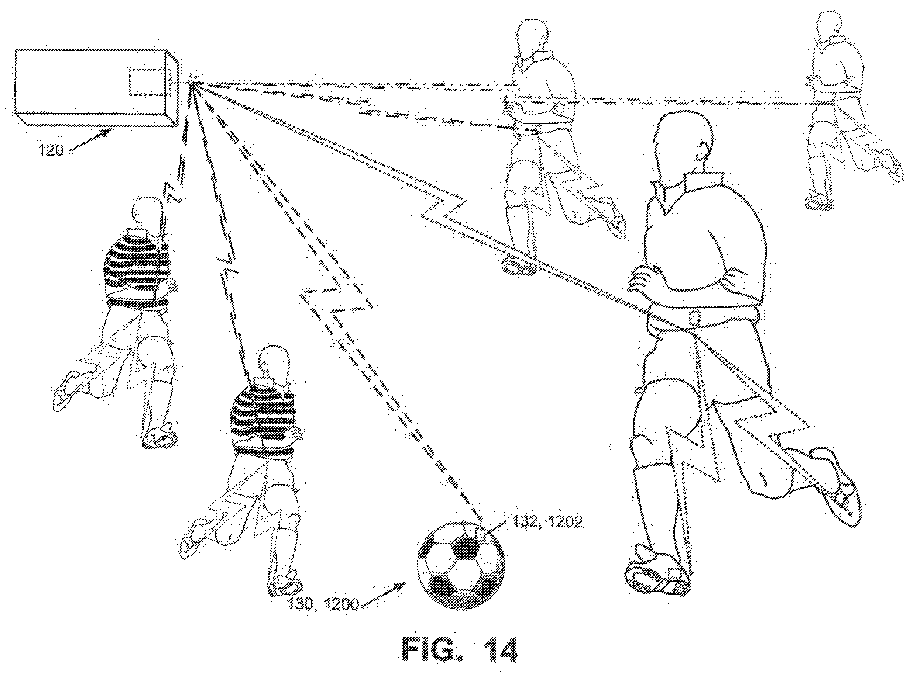

As illustrated in Fig. 13 (and as alluded to above) and Fig. 14, systems and methods in accordance with this invention are not limited for use with a single player. Rather plural players, optionally on both teams, may be equipped with active transmitters and/or receivers that interact with the transmitting, receiving, and/or reflecting equipment provided with the ball 130 or 1200. When plural players on a team are equipped with appropriate electronic equipment as described above, it can be determined when the ball 130, 1200 moves from one team member to another. Such systems and methods can be useful for providing various team metrics, such as team possession time, passing streaks and efficiency, pass accuracy, turnovers, steals, tackles, etc. All data (e.g., from the players on both teams, from the ball 130, 1200, etc.) can be transmitted to a single remote computer system 120, or optionally, if desired, to different remote computer systems 120 (e.g., one for each team, one for each player, etc.). As yet another example, if desired, the data can simply be logged during the game or practice session (as described in conjunction with Figs. 4 and 5 above) and later downloaded or otherwise accessed for use by the individual players, coaches, etc. The various player's data also could be intercommunicated to one another via peer-to-peer networking so that players could compare performances quickly and easily, e.g., on the sidelines, in the locker room, etc.

Team oriented metrics also allow team players and coaches to look at both the individual and team data and determine various features or characteristics of play, such as which players play best together, the strengths and weaknesses of individuals, the strengths and weaknesses of various groupings of players, who is ball "hogging," who is insufficiently involved in the game, who is loafing, etc. The coaches and/or team members can evaluate the data in real time (e.g., on the sidelines, in the coach's box) during the game or practice session to better understand whether a combination of players is working (or, potentially, to discover an injury or other need for substitution by noting that a player's performance has suddenly fallen off). Also, the team data can be used to motivate the individuals to challenge one another and/or to motivate them to make efforts to improve the overall team statistics.

### F. Example Website Features

Additional aspects of this invention relate to the presentation of data to the player, coach, trainer, or other person(s). Such systems help the player measure and track his or her capabilities, mark improvements over time, determine areas that require additional work, etc. Data can be collected over single games, portions of games, single practices, portions of practices, multiple games (or portions thereof), multiple practices (or portions thereof), multiple seasons (or portions thereof), etc.

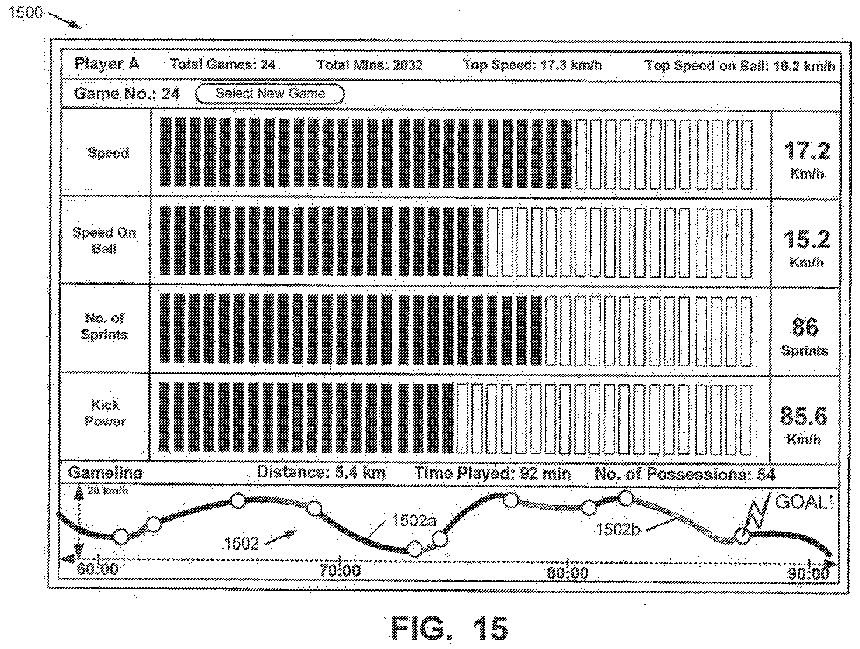

Fig. 15 illustrates an example user interface screen 1500 that may be used in systems and methods in accordance with at least some examples of this invention. As shown in Fig. 15, the interface screen 1500 may present much information to the player, including information relating to a specific game or practice session, as well as information relating to more long term use of systems and methods in accordance with this invention. For example, as shown in Fig. 15, user interfaces 1500 in accordance with this invention may provide information relating to the overall total number of games played by the player, the total overall minutes logged by the player using the system, the player's top speed over that time period, and the player's top speed while in possession of the ball (e.g., while he was personally in possession of the ball or within close proximity to it, not while the team was in possession).

The interface screen 1500 also provides information for an individual game (with the ability to select among the various stored games on the system). As illustrated in Fig. 15, in this example interface, the screen 1500 displays information relating to the player's movement speed during this specific game (i.e., Game 24), movement speed while in possession of the ball during this specific game, the number of "sprints" during the game (e.g., the number of times that the player's movement speed exceeded a predetermined threshold, such as 75% of their top speed), and the player's highest "kick power" during the course of the game (e.g., the highest ball speed logged from the player's kick). Also, if desired, the user interface could be adapted to allow user selection of various different metrics or information to be displayed.

The "Gameline" portion of this example interface screen 1500 includes information relating to the specific game displayed. For example, in the illustrated screen 1500, the Gameline includes information indicating the entire distance that the player moved during the game, the number of minutes played, and the number of "touches" or times that the player had "possession" of the ball. Additionally, in this example, the Gameline includes information regarding the user's speed over the course of the game, as well as the times that the player's team had possession of the ball. In this example, the dark black portions 1502a of the player's movement velocity line 1502 indicate when the player's team did not have possession of the ball and the lighter gray portions 1502b of the player's movement velocity line 1502 indicate when the player's team had possession of the ball. The visible portion of the movement velocity line 1502 can be changed so that any desired portion of the game can be displayed (the 60 to 90 minute time period is displayed in this illustrated example), or an entire game (or the portion in which the player played) can be displayed in a single view, if desired. Other metrics may be displayed in the Gameline portion of the interface 1500, if desired, without departing from this invention, either in place of this movement velocity time line 1502 or in addition to it (such as the times when the player kicked the ball, the player's goals (as shown), the player's successful passes, the team's goals, etc.). Also, if desired, user interfaces according to the invention could be designed to allow user selection of various different metrics in the Gameline portion.

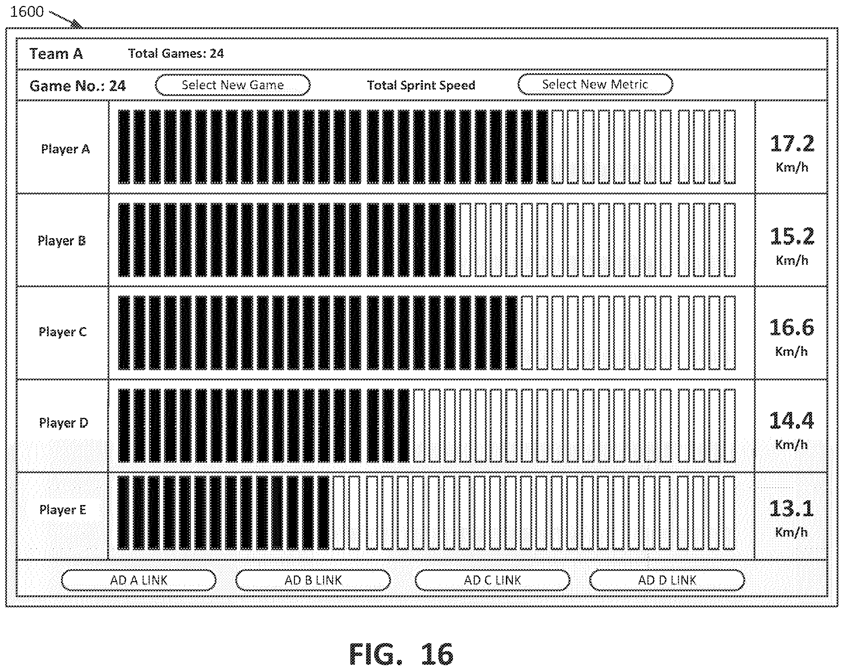

User interfaces in accordance with at least some examples of this invention also may display team information (or even competitor team information), if more than one player is equipped with the sensors and detectors in accordance with this invention. Fig. 16 illustrates an example user interface screen 1600 in which data from five players are displayed in a single screen. In this example, the player movement velocity data (e.g., top sprint speed) for five players that participated in a single game (Game 24) is displayed, e.g., so that the players or coaches can compare performance characteristics. Furthermore, in this example interface screen 1600, data for other games can be selected, or data for other measured metrics may be displayed in this plural player comparative manner (e.g., speed on ball, number of sprints, number of touches, kick power, number of successful passes, number of steals, number of turnovers, etc.). Other team data or other measured metrics also may be made available and displayed in this type of user interface screen without departing from this invention.

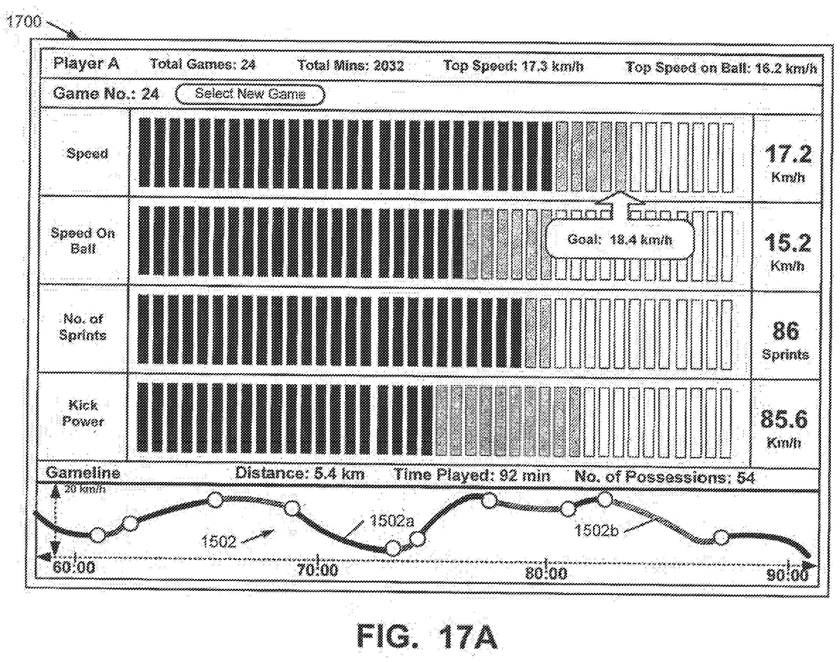

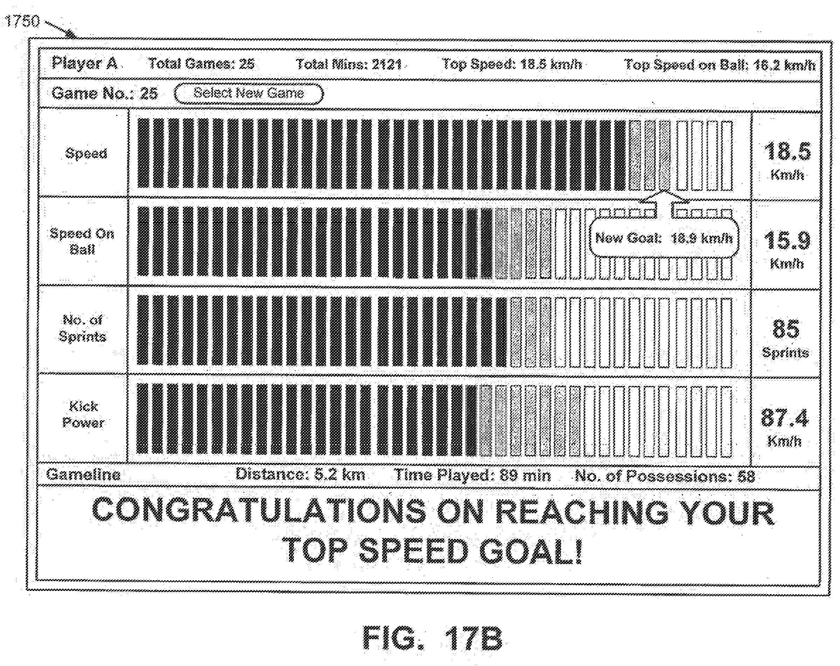

Systems and methods in accordance with at least some examples of this invention may include "goals" or "challenges." While the goals may be set by the individual player for himself or herself, optionally, the goals or challenges may be set by a coach, a teammate, a competitor, etc. Figs. 17A and 17B illustrate an example. Fig. 17A illustrates a user interface screen similar to that of Fig. 15, but in this example, each data metric further includes "grayed out" blocks that represent a player's "goal" or "challenge" for that metric. For example, in Fig. 17A, the data from Game 24 is displayed with an indication of the player's performance in that game (the blackened in boxes) and an indication of where the player's performance stood with respect to their "goal" or "challenge" levels (the grayed out boxes). The specific metric for the "goal" or "challenge" may be displayed in any desired manner, e.g., by clicking on the last box associated with the goal or challenge, by hovering over a grayed-out box, through a continuous display, etc. Notably, in this illustrated example, the system indicates that the player's overall top "speed" goal or challenge is 18.4 km/h, while in the present game they had only run at a top speed of 17.2 km/h.

In the next game (Game 25), however, as illustrated in the user interface screen 1750 of Fig. 17B, Player A achieved his or her speed goal by running 18.5 km/h. In this instance, systems and methods in accordance with at least some examples of this invention may provide a congratulatory message (e.g., textually, visually, audibly, etc., note the changes in the Gameline portion of Fig. 17B as compared to Fig. 17A). Furthermore, if desired, in an effort to keep the player motivated, a new "goal" or "challenge" can be calculated and displayed for the player. Also, if desired, when presented as a challenge from a third party, systems and methods in accordance with at least some examples of this invention may send a message to the challenger (or offer to let the player compose a message to his or her challenger) to advise that the challenge had been met. Other "rewards," motivational information, or other interaction may be provided, if desired, without departing from this invention.

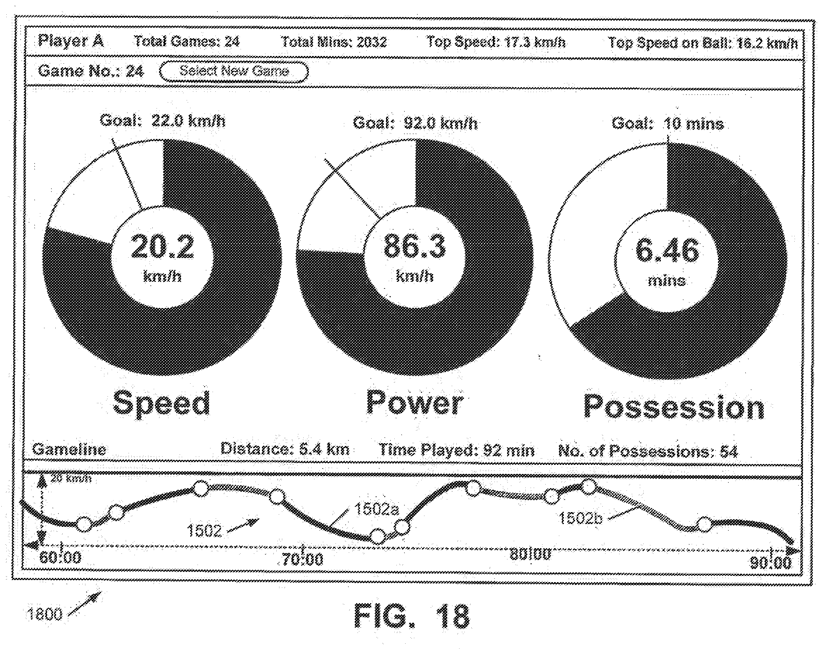

User interfaces for athletic performance monitoring systems and methods in accordance with this invention may take on a wide variety of forms and formats and provide a variety of different types of displays and information without departing from this invention. Fig. 18 illustrates another example user interface screen 1800 in which player speed, kicking power, and individual possession information is displayed on a more circular graph (as compared to the linear graphs of Figs. 15-17B). Fig. 18 also shows a player possession time metric as opposed to the speed on ball and number of sprint metrics provided in Figs. 15, 17A, and 17B. Displays of other metrics or combinations of metrics are possible without departing from this invention. Other graphical or other displays of the desired metric information also may be provided without departing from this invention.

**G. Throwing v. Kicking Determinations**

In at least some example systems and methods according to this invention, it may be desirable to distinguish between situations in which a ball or piece of sporting equipment has been thrown and when it has been kicked. This may be useful in various sports, such as soccer (e.g., to determine when play resumed and how it resumed, as will be described in more detail below) and basketball (e.g., to determine whether possession should be awarded to the other team). A determination of throwing v. kicking also may be useful for determining other metrics, such as possession time in soccer, as the throwing v. kicking determination may be useful in helping to determine when a ball goes out of bounds (e.g., on the side) during a soccer game (e.g., time between a throwing action and a previously determined kicking action may be considered "out of bounds" time in soccer (as a throwing action often is used to restart play from an out of bounds condition), and that amount of time may be deducted from a team’s determined ball possession time). Aspects of this metric also may be useful in basketball, for example, to determine when the ball struck the ground (more like a "kicking action" sensor response, as described below) as opposed to being pushed with a hand (such as for a shot or pass).

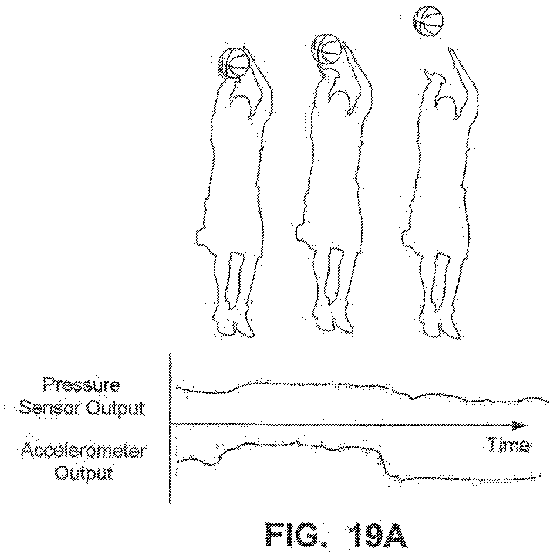

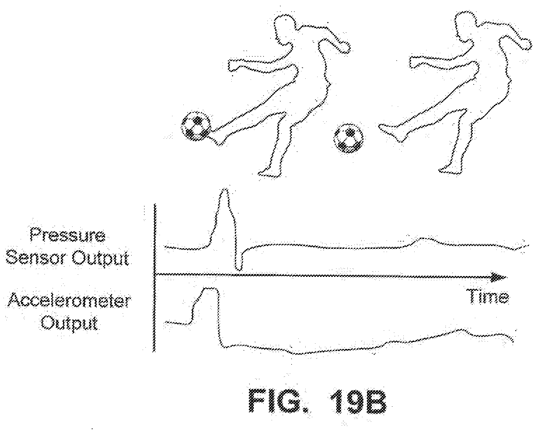

In accordance with at least some examples of this invention, as illustrated in Figs. 19A and 19B, output from one or more pressure sensors (e.g., a ball mounted pressure sensor and/or a foot mounted pressure sensor) and/or one or more accelerometers (or other inertial sensing device) (e.g., ball mounted and/or foot mounted) may be used for determining whether a ball has been thrown or kicked. Fig. 19A illustrates the ball sensor responses during a typical throwing action (such as a throw-in in soccer, a shot in basketball, etc.) and Fig. 19B illustrates the ball sensor responses during a typical kicking action (or a dribble off the floor in basketball). As shown in Fig. 19A, the output from both a pressure sensor and an acceleration sensor during a throwing action will tend to be a slow, long signal (or, depending on the throw, there may be little to no pressure signal at all from a simple throwing action). During a kicking action, however, as illustrated in Fig. 19B, a relatively short and strong impulse signal is generated by both the pressure sensor and the accelerometer sensor followed by a low-rate slowdown of the ball (e.g., due to aerodynamics, gravity, etc.). The pressure change inside the ball (or other object) is much slower when thrown as compared to when kicked, but the pressure change may last a longer time during the course of a throw event. Additionally, the accelerometer output will tend to constitute a much longer signal and lower level of acceleration from a throw as compared to a kick. These differences in sensor output between Figs. 19A and 19B will allow systems and methods in accordance with examples of this invention to distinguish between throwing actions (such as throw-ins in soccer, shots or passes in basketball, etc.) and kicking actions (or other similar actions that will generate a similar pressure and accelerometer output spike, such as ball contact with ground (e.g., a dribble), ball contact with a basketball rim, ball or puck contact with a goalpost or hockey stick (e.g., in football, hockey, soccer, etc.)).

**H. "Explosiveness" Determinations**

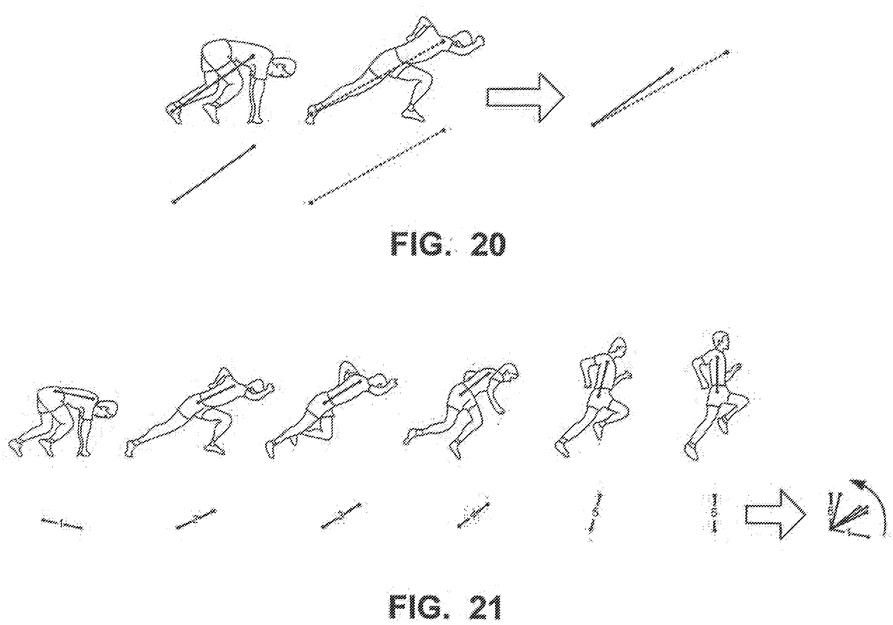

Fig. 20 illustrates an example of features that may be involved in determination of an "explosiveness" or "power" metric. Some metrics that may be useful in athletic performance monitoring systems and methods according to at least some examples of this invention relate to ways of determining how hard an individual is working over the course of a game or practice session. Fig. 20 illustrates various features involved in determining one example "explosiveness" metric. When athletes are in a crouch position (e.g., as shown in Fig. 20, such as sprinters, football linemen, backs, or other players, etc.), their effectiveness at the start of the activity is often determined by how quickly they spring into action (e.g., get out of the starting blocks, get out in front of rushing defensive players to make a block, etc.). As shown in Fig. 20, determination of the distance between the athlete’s feet and his/her upper body or torso, and the rate of change of this distance, can be used to determine an "explosiveness" metric that may be a gauge of the athlete’s performance. Note, for example, the differences in orientation and length between the foot based module and the torso based module from the crouch position (the solid line) and the initial "explosion" position (the broken line). Measuring and tracking the distance and/or angle and their rates of change may be used to determine various features or other metrics, like initial explosiveness, explosiveness over the course of a game or training sessions, improvements in explosiveness, effectiveness of training or conditioning, etc.

This measurement system may utilize two sensors (e.g., wireless sensors) or other modules that allow determination of the relative distance between two points (e.g. a foot based point and a torso or body core based point). The two sensors may report their positions to thereby allow their relative positions to be determined, and this information may be stored (e.g., in one of the sensors or modules, on another athlete carried device, such as a mobile phone, watch, PDA, audio/video playback device, MP3 player, etc.), transmitted to another location (such as a remote server, a laptop or other computer, etc.), etc.

Similar explosiveness or power metrics also could be used, for example, tied to a jumping action, such as a jumping action in basketball (or other sports).

Fig. 21 illustrates another potential manner of measuring explosiveness or power metrics by determining the player's acceleration. Generally, as illustrated in Fig. 21, when accelerating (as shown toward the left of Fig. 21), an athlete's center of mass and/or torso are generally located ahead of his/her feet. When a steady state pace is achieved (or when slowing down, as shown more toward the right of Fig. 21), the center of mass and/or torso more closely align vertically. In this example metric, the changing angle of the player's torso is determined, and the rate of change of this angle will provide information as to whether the athlete is accelerating, moving at a steady state pace, or decelerating.

As some more specific examples of the system of Fig. 21, the sensing system may include one or more of: an accelerometer, a gyroscope, or other rotation sensing devices. A sensor may be placed on the upper body and used to measure the rate of change of the angle of the upper body with respect to the body center (e.g., waist or pelvic area) and/or the feet. As another example, if desired, the rate of change of the gravity vector may be measured by an upper body mounted accelerometer. Optionally, if desired, this metric may be used in combination with foot or leg movement metrics to provide additional information or more detailed metrics with respect to specific activities. The foot or leg movement metric(s) may be measured using an accelerometer, a piezoelectric sensor, etc., to measure foot movement speed, foot impact force, foot loft time, etc. Combining the rate of torso angle change with other data, such as one or more of: body weight, height, foot location, foot movement, foot speed, or the like, may allow actual player acceleration to be determined.

## I. Additional Potential Features and/or Metrics that may be Measured in Systems and Methods According to this Invention

As noted above, while much of the above description has been provided in terms of use in a soccer environment, given the benefit of this disclosure, one skilled in the art could readily extend aspects and features of this invention to other team sports, such as basketball, American football, hockey, rugby, field hockey, lacrosse, baseball, cricket, volleyball, badminton, tennis, and the like. Different metrics may be tracked, stored, and/or displayed for different players or for different positions on the team (e.g., goalie versus center versus defensemen, etc.).

A wide variety of parameters or metrics may be measured and determined without departing from this invention. Including the various metrics described above, additional metrics that may be measured in systems and methods in accordance with at least some examples of this invention may include: vertical leap (e.g., with a body core mounted three axis accelerometer); number of leaps; jump height with the ball; jump height without the ball; team pace or match pace (an aggregate measure of speed, distance, and/or other data from all players on the team); on-field position and/or movement; on-field position and/or movement with respect to the ball's location; average speed intervals (on and off ball); top speed intervals (on and off ball); total distance moved (on and off ball); distance intervals; shot power; shots on goal; assists; blocks; saves; game duration; playing time; typical game statistics; etc. Data relating to any of these or the other metrics above may be combined and/or further processed, if desired, to provide other metrics or indices relating to the athlete's performance, such as a "hustle" or "intensity index," the number of shots without a goal, average number of shots between goals, tackles per game, minutes without giving up a goal, shot blocks, etc.

Another useful metric similar to one described above also may be termed "explosiveness," e.g., data and metrics tracking the player's initial movements from a slowed pace or stopped position. For example, this metric may include acceleration information relating to the first two or three steps. Additionally or alternatively, this metric may include information relating to the force applied to the athlete's foot or feet even before the athlete moves (i.e., as he or she prepares or "loads up" to take off).

Another useful metric may involve consideration of the differences in a player's performance over the course of a single game. If a player has a dramatic drop off later in the game, this information could be useful to the coach (e.g., to provide motivation, to induce substitution, etc.) or to the player (e.g., to induce work on conditioning, etc.).

Systems and methods according to this invention also may allow user input of other information relative to the game, such as temperature, humidity, wind conditions, field conditions (e.g., wet, dry, etc.), etc. Tracking these features may be useful to see how players perform under a variety of conditions and determining which players to field under a given set of conditions.

If desired, aspects of this invention also may include various automatic ON/OFF switching features, e.g., to preserve battery power for the actual game time but to assure that the desired data is captured. As one example, a referee, scorer, or coach could include a device that turns all devices ON and OFF from a central location. As another example, if desired, detection of the referee's whistle frequency could be used to turn the devices on and off.

Systems and methods according to examples of this invention also may allow an individual to compare his or her performance (e.g., any measured metric) to that of a professional athlete or another player (e.g., on a game-per-game level, on a metric level, etc.). Training advice or practice drills also could be downloaded to or provided to the player by systems and methods in accordance with this invention, optionally, based on the measured performance metrics stored in the system. Additionally, if desired, systems and methods according to examples of this invention also may be used to recreate an animation of the game (and the player's performance) on the computer screen after the game has been completed (or even while it is going on).

Aspects of this invention also may be useful for other purposes within the context of a team sport, such as a referee assistant (e.g., did a player have possession, was a player out of bounds, was the ball out of bounds, was the shot made before time expired, etc.). Coaches also could use features of the invention during practices, drills, or even during the overall game to determine which players should play, which players should play together, which players should not play together, as a motivational tool, when to substitute, etc.

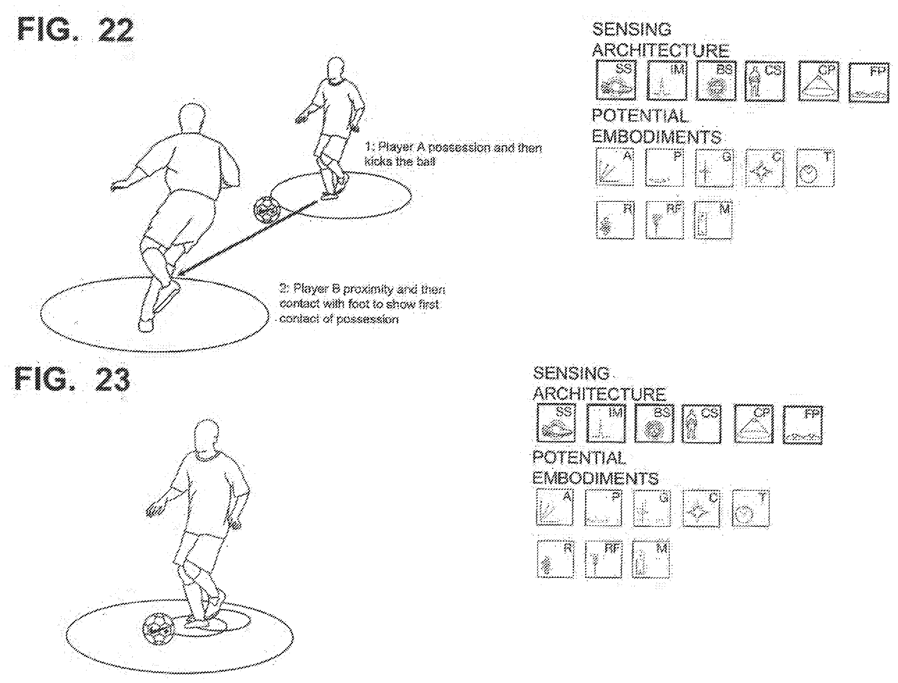

The following description, in conjunction with Figs. 22 through 94, provides some detailed information relating to measurement of various metrics and various other features of systems and methods according to examples of this invention that may be useful in various environments, including for use in monitoring athletic performance in the context of soccer (e.g., for use in a soccer game, soccer training, soccer practice, etc.) or other team based sports. Figs. 22 through 94 illustrate various soccer (or other sport) scenarios (e.g., typical game or practice events, types of plays, types of ball control or ball possession transfer, etc.) as well example "sensing architecture" and example sensors and/or combinations of sensors (called "Potential Embodiments" in Figs. 22-94) that may be useful in collecting the data and making the measurements for determining features, aspects, and metrics based on that scenario. The following abbreviations are included in the various figures, and these abbreviations have the meanings provided below:

**Motion Sensing Definitions:**

- CS - Core mass sensor (sensor(s) on the athlete's body core capturing player motion data)
- SS - Shoe (or foot) based sensor (sensor(s) in one or more shoes to capture foot motion data)
- BS - Ball Sensor (sensor(s) in the ball to capture ball motion data)

**Proximity Sensing Definitions:**

- CP - Core mass proximity sensor (sensor(s) on the athlete's body creating a proximity sensing field around the player, e.g., as described above)
- FP - Foot based proximity sensor (sensor(s) mounted on the shoes or near the foot creating a tight proximity sensing region between the ball and a foot (which may be the same as or similar to the core mass sensors described above))
- IM - Impact sensor (a time stamped impact on a foot sensor and a ball sensor indicating foot/ball contact)

**Sensor Types:**

- R - Radar based sensor system
- RF - Radio (or radio frequency) based sensor system
- GPS - Global positioning satellite based sensor system
- M - Magnet based sensor system (e.g., Hall Effect sensors, etc.)
- MC - Magnetic coil based sensor system
- P - Pressure sensor system (e.g., piezoelectrics, etc.)
- A - Accelerometer sensor system
- G - Gyroscope based sensor system
- T - Time sensor or clock
- C - Compass (e.g., electronic compass)

Figs. 22 through 35 illustrate various potential features for detecting interactions of soccer players with respect to the ball, e.g., during a game, practice session, etc. The features of these "player on ball" determination systems, methods, and metrics will be described in more detail below.

**Fig. 22** - **Receive Possession:**

To create useful metrics for the game of soccer, systems and methods according to at least some examples of this invention will have at least some manner of determining when possession of the ball starts (e.g., to determine individual player possession time, team possession time, etc.). Therefore, systems and methods according to examples of this invention include some manner of choosing and determining events that start the possession clock and/or keep the possession clock running. In accordance with this example of the invention, sensors in the shoe and the ball may be used to determine and start a possession event. Proximity sensing alone (e.g., player proximity to the ball, as described above) may not be sufficient to determine accurately when a possession actually starts for all uses, so additional sensing methods may be provided to more accurately determine when a possession time clock can be initiated in accordance with at least some examples of this invention.

As shown in Fig. 22, in this example system and method according to the invention, a sensing system in the ball (pressure sensor, accelerometer, gyro, magnetometer, etc.) detects an impact to the ball, and coincidently a sensor (accelerometer, piezo element, or other inertial sensing system) in the boot of a player matches the impact time exactly. This precise moment may be used in at least some systems and methods according to this invention to determine the start of possession. In other words, as illustrated in Fig. 22, when Player A kicks the ball toward Player B, Player B's proximity to the ball and then contact between Player B's shoe and the ball, optionally along with departure of the ball from the proximity of Player A, will be used to establish possession and start a possession time clock for Player B and/or continue a team possession time clock for one team (if Player A and Player B are on the same team) and/or start a new team possession time clock (if Player A and Player B are on different teams). Various examples of the sensing architecture and sensor systems that may be used for determining this metric are illustrated in Fig. 22.

**Fig. 23** - **Player Possession:**

In addition to determining when an individual player's possession starts, systems and methods according to at least some examples of this invention further may wish to track how long an individual player maintains possession of the ball. Fig. 23 illustrates various example features of potential systems and methods for determining individual player possession. This example system and method according to the invention uses sensors in the shoe and the ball to start this event (as described above in conjunction with Fig. 22), and then uses proximity detection features to confirm that the player has kept possession after that initial contact and the length of time associated with this possession. For example, when the player kicks the ball out of their proximity (at least under certain conditions as will be described in more detail below) or if the player is tackled and loses possession (as will be described in more detail below), these events may be determined as possession ending events (which can be used to at least temporarily stop that player's possession time clock). Various examples of determining player possession and/or proximity may be used, as described above.

As some more specific examples, as noted above, a sensing system in the ball (e.g., pressure sensor, accelerometer, gyro, magnetometer, etc.) detects an impact to the ball, and coincidently a sensor in the boot of a player (e.g., accelerometer, piezo element, or other inertial sensing system) matches the impact time exactly. This precise moment determines the start of possession. Then, an on body proximity sensor can be used (e.g., as described above, such as a RADAR, radio frequency, or magnet system) to confirm that the ball remains in the field of proximity and (via the time counting sensor) the amount of time that the ball remains within this field of proximity (optionally, without another player having contact with the ball, which would constitute a change in individual possession (but not necessarily team possession)).

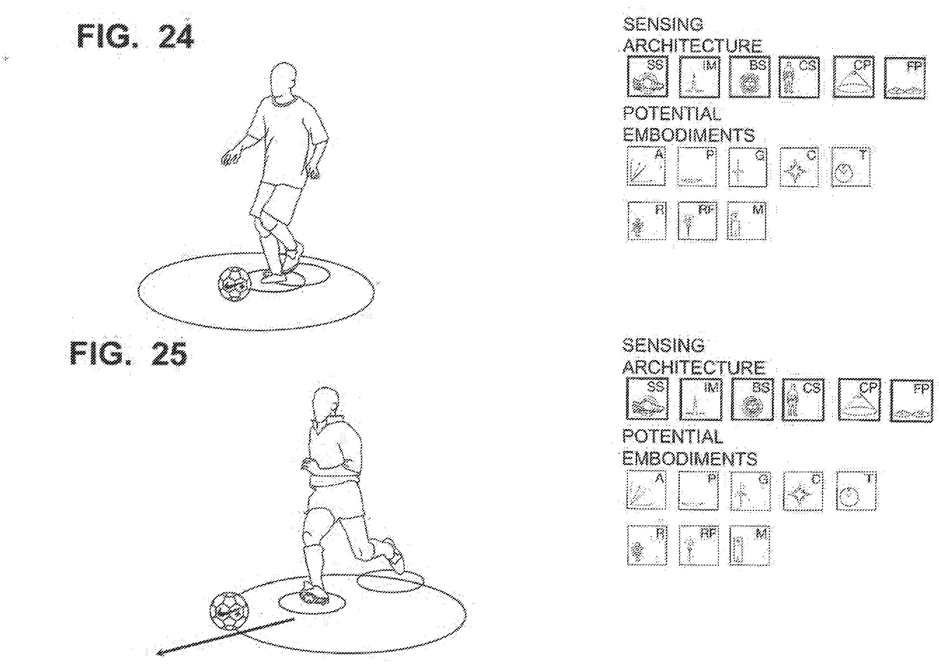

**Fig. 24** - **Speed on Ball:**

As described above, one metric that may be particularly useful for determination by systems and methods in accordance with examples of this invention constitutes a player's "speed on ball" metric (e.g., a measure of how fast a player moves while in possession of the ball). Fig. 24 illustrates an example system and method. In this example system and method, a proximity sensing system (such as RADAR, radio frequency, magnetic sensors, etc.) is used to determine when the ball is in proximity to the player. Impact sensing systems in the boot (accelerometer, piezo element, etc.) are matched to impact sensing systems in the ball (pressure sensor, accelerometer, gyro, etc.) to determine when the foot impacts the ball. Speed on ball is then determined as the speed at which the player moves while in continuous proximity to the ball, with repeated foot impacts to the ball, and/or as the speed at which the player moves while the ball is determined to continuously be in his/her possession.

As another alternative, systems and methods according to at least some examples of this invention may continue the "speed on ball" measurement metric (as well as a player possession metric as described above) even when the ball falls outside the core proximity sensing capabilities under certain circumstances. For example, the speed and ball and/or player possession metrics may continue running their clocks when the ball moves outside the core proximity sensing capabilities as long as: (a) the ball never is detected to be in the proximity of another player and/or (b) the amount of time the ball is outside the player's core proximity sensing range is below a specified time threshold. This would cover situations where a player is running fast and making long dribbles (which may extend outside the core proximity detection range) while still consistently maintaining control of the ball.

**Fig. 25** - **Short, Break, and Long Dribbles:**

As described above, output from an impact sensing system inside the ball (e.g., accelerometer, pressure sensor, etc.) may match timing with output from an impact sensing system inside the boot to time-match impacts so that systems and methods according to at least some examples of this invention will be able to determine when the ball is struck by a specific foot. A proximity sensing system also may be employed (e.g., magnetic sensing, RSSI, etc.) to determine when the ball is in proximity to the specific players on the field. A "dribble" action may be determined, e.g., by repeated foot/ball contacts by a single player. Combining a dribble action determination with other metrics, such as player speed/acceleration metrics, can provide other useful information for evaluating athletic performance. More specifically, systems and methods according to at least some examples of this invention can differentiate between different types of dribbles and allow determination of different metrics.

As some more specific examples, the following dribble types may be determined: (a) a "short dribble" can be defined as player dribbling the ball with a low player speed (e.g., below a threshold speed, optionally a threshold speed based on the individual player's top sprinting speed and/or average running speed), (b) a "break dribble" (or "break away" dribbling) can be defined as a player with an accelerating player speed, and (c) a "long dribble" can be defined as a player dribbling beginning with a break dribble followed by a steady player velocity and/or then repeated foot contact by the same player. Systems and methods according to aspects of this invention may further break up player possession time into the various times that the player spent in these various different dribbling activities.

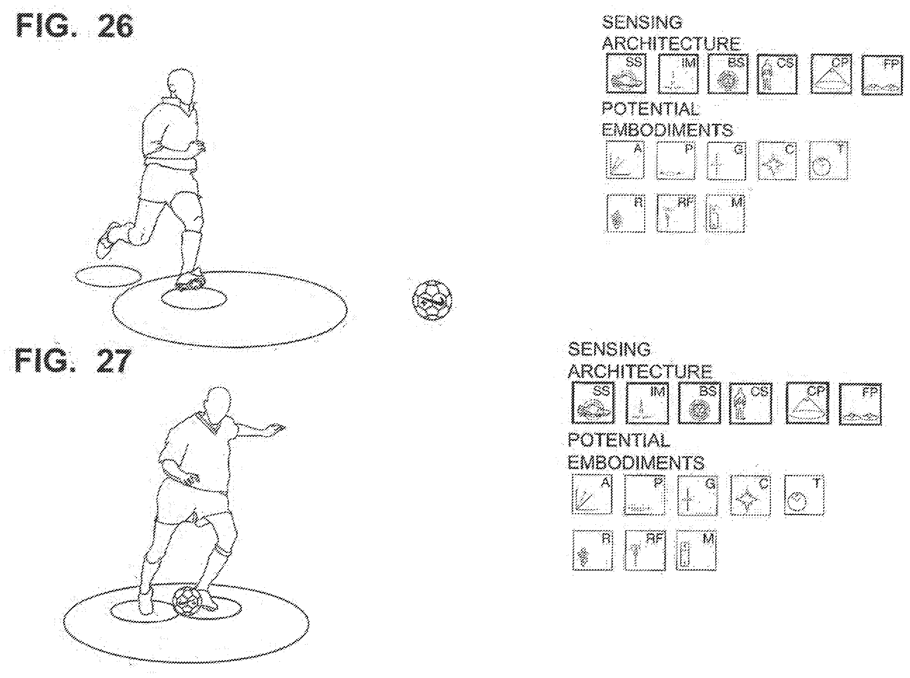

**Fig. 26** - **Knock On and Sprint:**

This common play in the game of soccer may be detected by systems and methods according to at least some examples of this invention using a multitude of sensing systems and combining their outputs. An impact sensing system inside the ball (e.g., accelerometer, pressure sensor, etc.) is matched to an impact sensing system inside the boot to time-match impacts to know when the ball is struck by a specific foot, as described above. Additionally, a player speed sensing system (e.g., foot based, core-mounted inertial sensing based, etc.) may be used to determine player speed. Using a determination of the start of possession as described above, one example sequence of events that could lead to a determination of a "knock on and sprint" event may include the following sequential steps:

a. Ball impact is detected along with a foot impact, determining start of possession;
b. The proximity sensing system determines when an opposing player comes within the possession radius;
c. The ball and shoe sensors then determine a kick by the player having possession;
d. The speed sensing system detects a sprint while the ball is located outside the proximity detection radius from the player;
e. The same player then runs onto the ball, and the proximity sensing system determines player/ball proximity;
f. Then, the start of possession determination methods described above are then used to determine the resumption of the player's possession.

The number of "knock on and sprint" events detected for an individual player during the course of a game (or other time period) may be determined as a metric, e.g., as a measure of the player's effectiveness at avoiding defensemen, as a player's ball control capability, etc.

**Fig. 27** - **Close Control:**

One important skill in the game of soccer is the ability of a player to keep the ball within very close proximity to himself or herself while still reaching very high running speeds. Systems and methods according to at least some examples of this invention may be used to determine a player's top speed (or average speed, etc.) when keeping the ball in close proximity. As some more specific examples, an inertial sensing system may be employed to determine player speed and movement distance (e.g., accelerometers, piezo elements, etc.), and an impact sensing system inside the ball (e.g., accelerometer, pressure sensor, etc.) may be matched to an inertial sensing system inside the boot to time-match impacts to enable determination of when the ball is struck by a specific foot. Optionally, sensor systems may be provided to enable determination of the path that the foot has traveled over the course of its movement (e.g., accelerometers, gyros, etc.). A proximity sensing system also is employed (e.g., magnetic sensing, radio frequency, RADAR, etc.) to enable determinations of when the ball is in proximity to the players on the field. Using such hardware, determination of "close control" may be performed as follows:

a. The proximity detection systems determine when the ball is close in to the player.
b. Speed is determined using an on-body or on shoe speed and/or distance system, such as an accelerometer, piezo element, or similar.
c. At no time during the run can the ball leave a defined proximity from the player.

Such a system may enable determination of the player's top speed, average speed, and/or other speed characteristics while at all times maintaining the ball within a defined proximity or distance from his/her body (i.e., movement speed while maintaining close control over the ball). Such a metric may be useful in identifying players with breakaway speed that will still have a good ability to maintain control and possession of the ball even at high speeds.

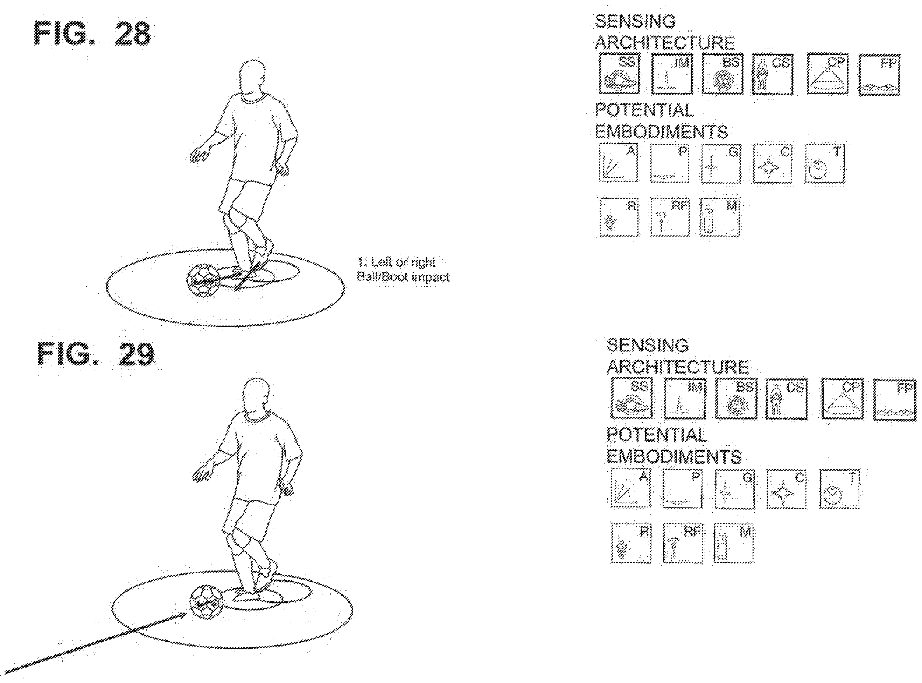

**Fig. 28** - **Dribble Foot Distribution:**

This example aspect of the invention combines an impact sensing system in each of the user's shoes and an impact detection system in the ball, as has been described above. Time correlated impact events between the ball and each individual shoe may be ascertained to enable determination of which foot struck the ball. This data can be logged over the course of a game (or any desired time period), and the system can store this information and/or wirelessly communicate the data to a remote location. The data can be presented to the player (or coach, etc.), e.g., as a chart, graph, histogram, etc., to inform the player how often they use each foot during dribbling. This metric also can be used at least in part to formulate a report for the athlete that includes suggestions on how to improve. This metric allows determination of the dominant foot used by the athlete, which can lead to further metrics (such as development of weak foot to provide better shots on goal, etc).

**Fig. 29 - Control of Incoming Ball:**

This example aspect of the invention uses a combination of various sensing systems described above to create a skill metric describing how well a player deals with an incoming ball (e.g., from a pass, during a steal, etc.). A formula can be created by the combination of two or more of the following metrics, some of which are described above and some of which are described in more detail below): (a) Kick Style, (b) Speed of the Ball, (c) Proximity, (d) Deceleration of the Ball (as determined by inertial/pressure sensing systems in the ball), and/or (e) Player Speed. As a more specific example, if desired, a ratio of (Speed of the Incoming Ball + Player Speed)Ball Proximity after first touch may provide a useful metric. If the ball is maintained in close proximity to the player during an incoming kick, this indicates good player control over the ball. Maintaining close proximity to the incoming ball after the player's first touch, particularly when the ball is moving at high speed and/or the player is moving at high speed, is even more difficult. Therefore, a high ratio as described above would provide one potential incoming ball control metric. Other control metrics may be determined, e.g., using the other metrics described above without departing from this invention.

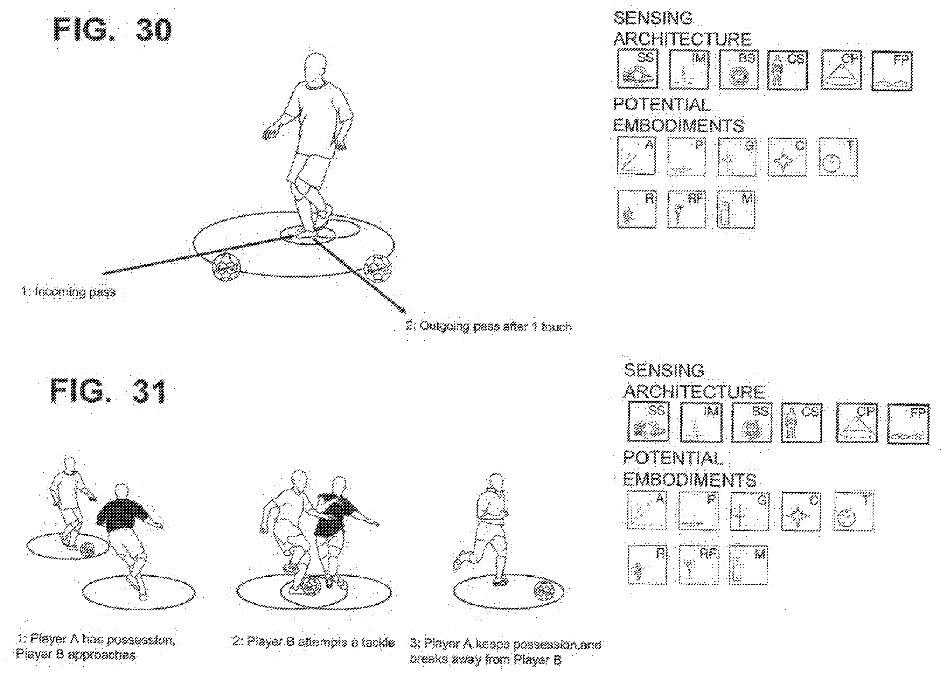

**Fig. 30 - One Touch Pass:**

A "one touch pass" is a frequently used play in soccer that can be very influential in the game, allowing for fast movement of the ball and creation of space between the ball and defensemen. A "one touch pass" determination may be accomplished in a manner similar to a combination of a "pass" determination and a "possession" determination as described above. In the "one touch pass" scenario, the ball comes into the player's proximity rapidly, strikes one of the player's feet one time (e.g., determined using time matched ball and boot impact sensors), travels out of proximity, and (optionally), into possession or proximity of a teammate. While the player making the one touch pass may not (and/or need not) get possession time credit

(because his/her possession time is too short), counting the player's involvement in the play and/or counting the player's pass can be very valuable information and a very valuable metric (e.g., for determining various other data or metrics, such as assists, passing efficiency, etc.) in understanding the effectiveness of a particular player.

**Fig. 31 - Tackle Avoided:**

For determination of this event and/or metric, output from an impact sensing system inside the ball is time matched with output from an impact sensing system inside the boot to enable a determination of when the ball is struck by a specific foot. A proximity sensing system also may be employed, as described above, to enable a determination of when the ball is in proximity to the various players on the field. A determination of a "tackle avoided" metric according to this example of the invention uses the above defined dribble metric and a contested time determination (e.g., defined as a time period when the ball is located within close proximity to players on both teams). The following sensor outputs may be utilized to determine whether a tackle has been avoided:

a. A dribble or possession is recorded by or awarded to a particular player.
b. The ball proximity sensing system detects a "contested time" event when two or more players, with at least one from each of the teams on the pitch, located within a predetermined proximity to the ball.
c. A short time later, another dribble or possession determination is recorded by or awarded to the same player as in step a above, but with no other players in proximity to the ball (as detected by the proximity sensing system).

This sequence of events may be used to award a "tackle avoided" event to the player maintaining possession. Tabulation of such events may provide useful ball control metrics for the various players.

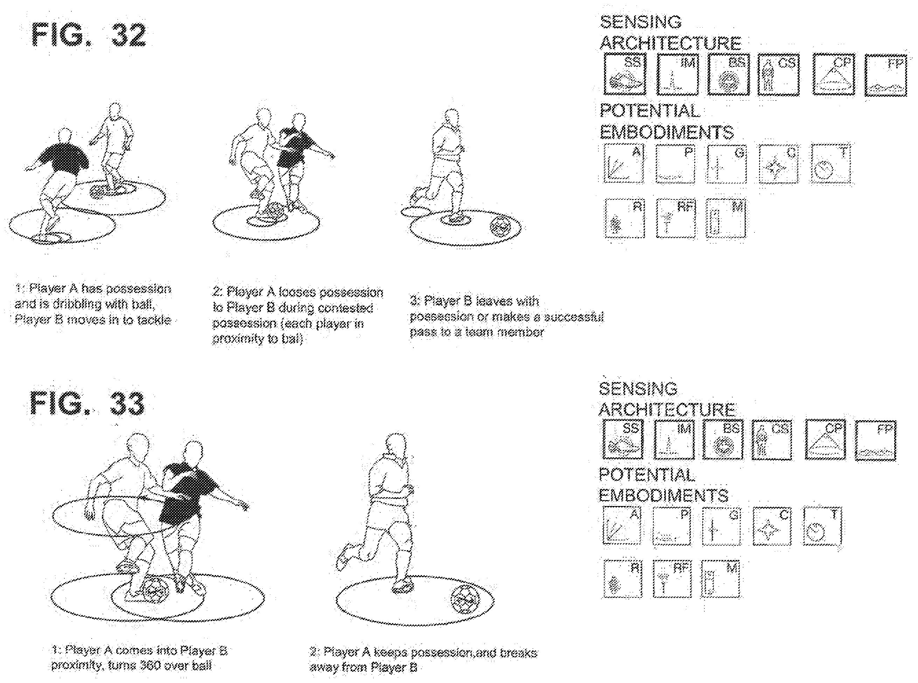

**Fig. 32** - **Tackle Successful:**

Determination of successful tackles also is a useful metric that may be tracked by systems and methods according to at least some examples of this invention. Determination of this metric is substantially the same as determination of the "Tackle Avoided" metric described above, except to have a successful tackle determination, an opposition player who was in proximity to the ball, a player that went in for the tackle, leaves in possession of the ball or successfully passes the ball to a teammate (a player on the opposite team from the player initially awarded possession). More specifically, as shown in Fig. 32, while Player A has possession of the ball (e.g., is dribbling), Player B from the opposing team moves in to attempt a tackle; Player A loses possession to Player B during the contested possession time; and Player B leaves with sole possession of the ball or passes the ball to a team member. Tabulation of successful tackle events may provide useful ball control metrics for the various players, e.g., for determining poor ball handlers, superior defensive players, etc.

**Fig. 33** - **A "Skin" Event:**

Determination of a "skin" event may utilize an impact sensing system inside the ball and impact sensing systems inside the boots to enable time-matching of ball and boot impacts and to enable determination of when the ball is struck by a specific foot. This determination also may utilize a proximity sensing system to enable a determination of when the ball is in proximity to the various players on the field, and, in at least some examples, a core-mounted player rotational sensor (e.g., a compass sensor, a gyro sensor, an accelerometer, etc.) to enable a determination of which direction the player is facing and/or player relative rotational information. Using such a system, a "skin" may be defined by the following sequence of events:

a. A first player receives a pass by registering proximity of the ball to the player as well as a simultaneous impact event on both the ball and boot.
b. A second player is detected by the ball proximity sensing system (e.g., beginning a contested time period determination).
c. The core mounted rotational sensor registers a 360 degree rotation of the first player (or some other significant rotational or other directional change move).
d. The proximity sensing system from the ball senses only the first player in proximity of the ball (e.g., a break away from the second player plus possession of or proximity to the ball).
e. A dribble or pass event is then recorded by the first player.

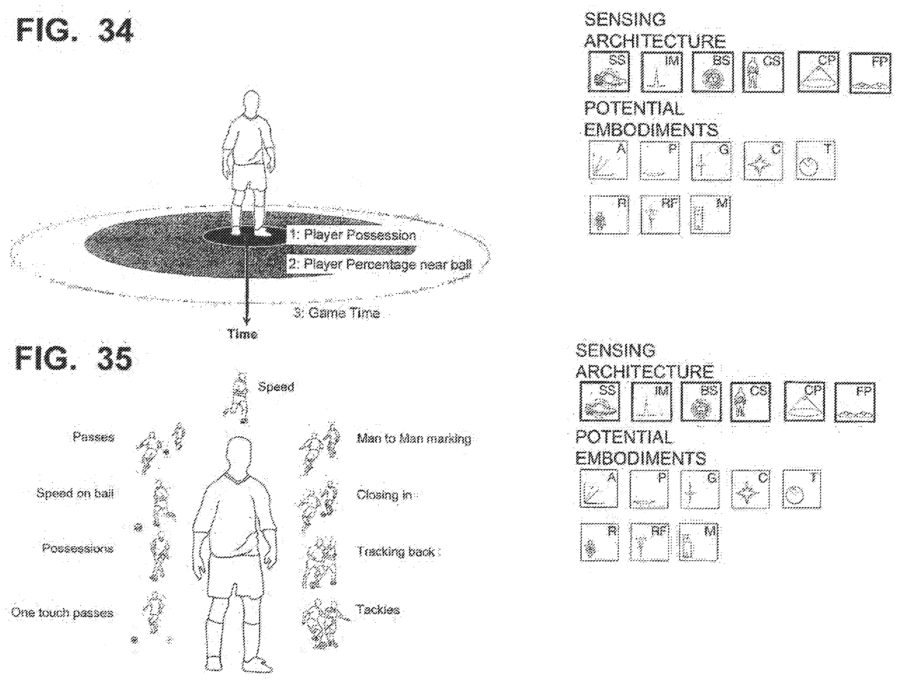

**Fig. 34** - **Possession "Heat Map":**

Using the possession and/or player proximity to the ball determination technology described above also can provide useful information for presentation of the data for player or coach review. For example, computer display screens and interfaces in accordance with at least some example of this invention can provide a graphic visualization of the amount of time each player was near the ball and involved in the game. For example, as illustrated in Fig. 34, a first region in immediate vicinity of a visual depiction of the player (e.g., a photo, an avatar, etc.), optionally having a first color or a first color intensity, may indicate the amount of time the player had possession of the ball; a second region surrounding the first region (optionally having a second color or a lighter color intensity from that described above) may indicate the amount of time that the player was in proximity to the ball whether or not in possession (e.g., contested time, defending time, etc.); and, optionally, a third region surrounding the first and second regions that indicates the entire game time or the entire time that the specifically identified player was on the pitch and in the game. Such data presentation can provide a quick visual indicator (optionally coupled with other data on the display, such as total play time, percentages, etc.) for the player or coach as to a specific player's involvement in the game.

**Fig. 35** - **Intensity:**

An intensity metric can be created, for example, using one or more of the sensing systems described above (e.g., player to ball proximity sensing, player to player proximity sensing, player speed, passes, tackles, etc.). As some more specific examples, an intensity metric may include information such as involvement in a play (e.g., ball proximity information (number of times close to the ball, number of times in possession of the ball, etc.), number of passes (including one touch passes), etc.), player proximity information (number of times close to another player, number of successful tackles, etc.), speed of the player on ball, speed of the player off ball, time spent near opposition players that are on ball, man-to-man marking, closing in on the ball, tracking back, etc. This information also can be displayed on a computer display device and/or a user interface therefore, in any desired manner, e.g., as shown in Fig. 35.

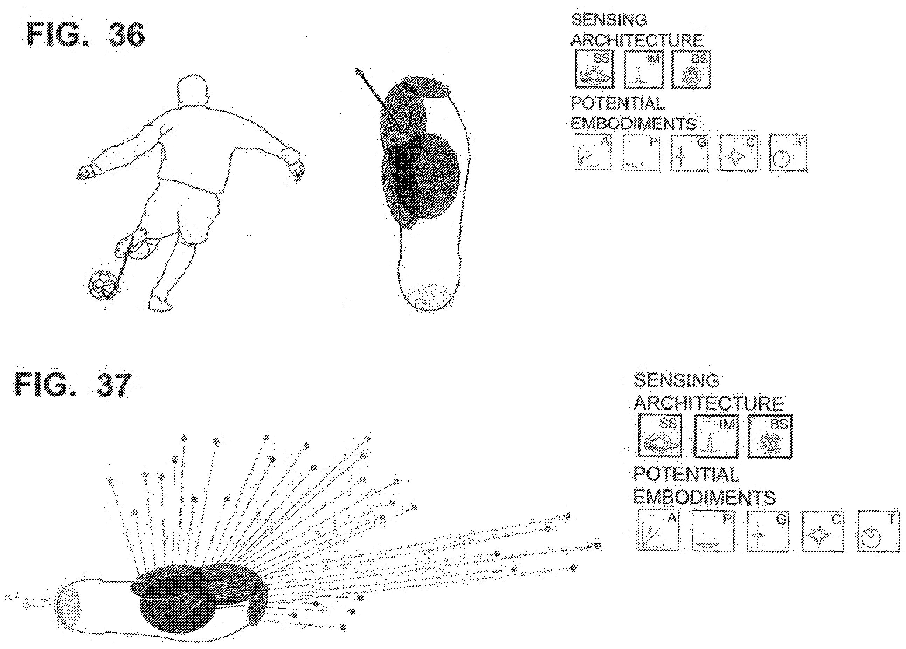

Figs. 36 through 45 illustrate various potential features for detecting and/or measuring various metrics relating to soccer players' kicking actions, e.g., during a game, practice session, etc. The features of these "kick" feature determination systems, methods, and metrics will be described in more detail below.

**Fig. 36** - **Kick Zone Determinations:**

At least some systems and methods according to examples of this invention will be able to determine the area of the boot and/or foot that impacts the ball during a kick. Such systems and methods may use, for example, an impact vector reporting sensor system (such as a 3-axis accelerometer) in the boot, combined with sensing mechanisms in the ball that can communicate the exact times of impacts. The acceleration vector produced by the impact of the boot with the ball is matched up to the exact time in which the ball is impacted. Because the soccer ball is approximately spherical, the impact vector as reported by the boot will be normal to the surface of the boot that impacted the ball. Therefore a distribution of kick zones on the surface of the boot can be output to the user to help inform skill level and areas of development.

This kick zone distribution information may be displayed on computer displays and/or user interfaces in accordance with at least some examples of this invention, for example, as shown in Fig. 36, where the color intensity or color area corresponds to the number of kicks produced in that area of the shoe (e.g., 1-5 kicks in a zone makes the zone appear red, 6-10 kicks in a zone makes the zone appear blue, etc.). Any number of zones may be provided in the display or a point for each individual kick may be provided in the display without departing from this invention (optionally with the ability for the user to "drill down" to get more data about the individual kick, such as ball speed, travel distance, kick results (e.g., successful pass, goal, turnover, out of bounds, etc.), and the like).

As an alternative, a rotational sensing system may be provided at or near the center of the shoe, and this sensing system can be used to determine the immediate rotation of the foot that occurs when the ball is impacted. This information will allow systems and methods according to this example of the invention to determine if the ball impact occurs ahead or behind the center of rotation axis of the sensor, as well as the side of the foot that impacts the ball.

**Fig. 37** - **Ball Flight Path Distribution:**

As another potential feature, systems and methods according to at least some examples of this invention will allow for determination of a ball flight path distribution. In this example system and method, the output of a three-dimensional accelerometer in the ball is used in combination with the kick zone determination features described above. As a more specific example, if the acceleration vector from the ball is known (and therefore, the flight direction can be determined), this information combined with the impact location on the boot, allows the flight path of the ball to be determined. This information can then be fed into a system that aggregates the distribution of these flight paths, and the information can be displayed on computer displays and/or user interfaces in accordance with at least some examples of this invention, for example, as shown in Fig. 37, wherein the flight direction off the boot from one or more kicks over the course of a game or other time period can be displayed. The length of the lines shown in the display of Fig. 37 may correlate to the length of the flight path of the ball (optionally with more data available for each individual kick, if desired, e.g., as described above). This information can be used by players and/or their coaches to determine appropriate drills or training sessions to help the player develop specific skills or improve his play and/or versatility. As shown in Fig. 37, the ball flight path information may be combined with the kick zone information in the display.

As some alternatives, a compass, gyro, or other rotational sensor can be added to the system to more accurately determine flight path. Faster rotations of the ball may be considered as producing a more curved flight path due to the aerodynamics of the ball. In such systems and methods, the ball flight path on the display of Fig. 37 may be displayed as a curved path with the degree of the curve displayed correlating to the amount of spin and direction of spin applied to the ball during the kick.

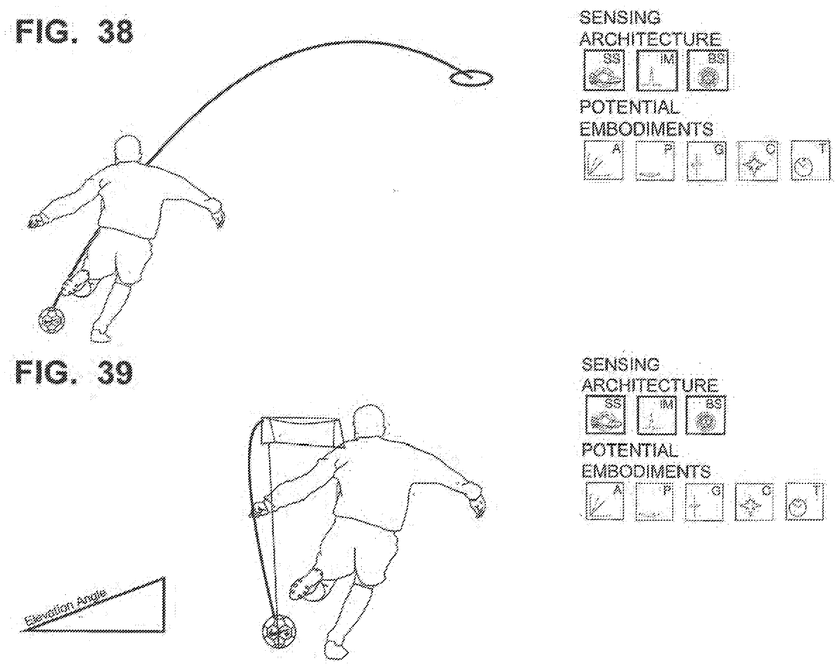

**Fig. 38** - **Longest In-Game Kick:**

As another metric, systems and methods in accordance with at least some examples of this invention may determine the longest ball kick by an individual player over the course of a game. As a more specific example, systems and methods according to at least some examples of this invention may use ball speed information (e.g., using known and commercially available technology, such as systems and methods available from CAIROS). Furthermore, this example system and method will collect data using in-ball sensing capabilities (e.g., including, but not limited to: pressure sensors, accelerometers, or gyros) to determine the first impact that occurs after the ball is kicked. Data relating to the kick speed combined with flight time data is then multiplied to get a "longest kick" metric. Additionally, if desired, ball travel directional vector information (e.g., from in-ball sensing systems), such as kick elevational angle as discussed below, can be used to provide an initial ball flight direction vector to provide further directional and distance information. Those skilled in the art can add modifiers to the product of kick speed and flight time (e.g., rotational information) that take into account aerodynamic or other flight effects which may reduce the total flight distance.

**Fig. 39** - **Kick Elevation Angle:**

Kick elevation angle may be an important metric in the game of soccer, particularly when it comes to game events, such as free kicks and penalty kicks. For example, on a penalty kick, a ball flight having too high of an elevational angle combined with high speed will never be capable of scoring a goal (e.g., if the ball sails over the level of the net). Systems and methods according to at least some examples of this invention may determine the kick elevation angle by using one of multiple methods of determining the gravity vector (e.g., such as an accelerometer), and then combining it with kick vector data as reported by an inertial sensing system within the soccer ball. The elevation angle of the kick with respect to gravity then may be determined and reported by the ball to a remote system (or stored for later download or use).

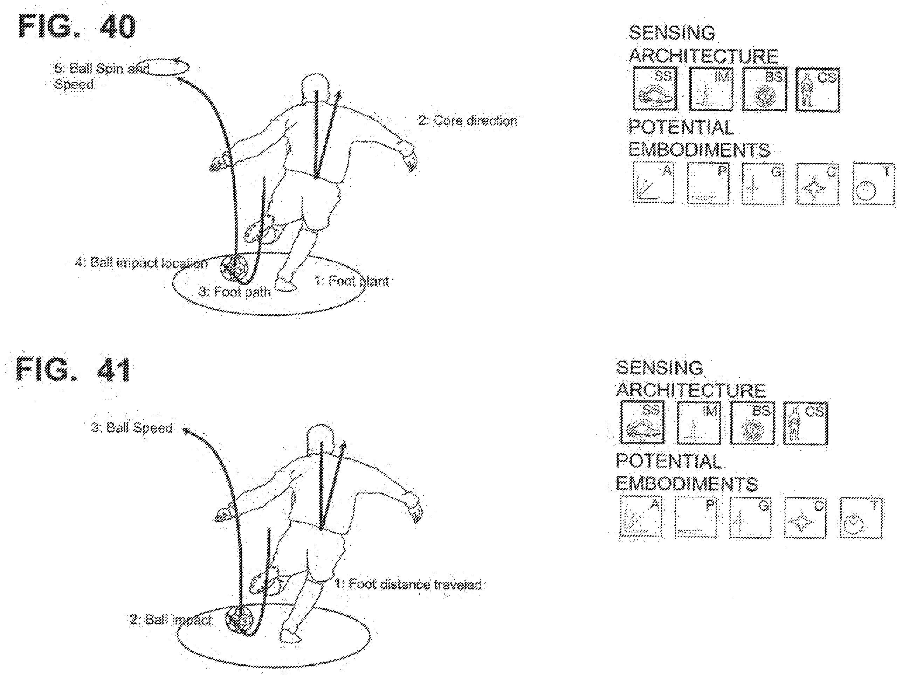

**Fig. 40** - **Kick-Type Distribution:**

Systems and methods according to at least some examples of this invention further may determine the various types of kicks and a kick type distribution for individual players (and/or for a team, a specific lineup or combination of players, etc.). Such systems and methods may include use of an impact sensing system inside the ball (e.g., accelerometer, pressure sensor, etc.) which may be matched to an inertial sensing system inside the boot to time-match impacts, which allows determination of when the ball is struck by a specific foot. The boot further may include sensors that allow determination of the path that the foot has traveled over the course of the kick (e.g., gyro, accelerometer, etc.). A proximity sensing system also may be employed (e.g., magnetic sensing, RSSI, etc.) to allow determination of when the ball is in proximity to the players on the field. A core-mounted player rotational sensor also may be employed (e.g., compass sensor, gyro, etc.) to understand which direction the player is facing as well as relative rotational information, and an inertial sensing system on the player can be used to provide additional data. Detection or determination of kick-type distribution information may be accomplished, for example, in the following way:

a. Inertial sensors in the shoe detect the plant foot's impact to the ground and static nature.
b. Core mounted rotational sensor wirelessly communicates the core facing direction (e.g., to a remote location), or this data is stored.
c. Inertial sensors in the kicking foot detect the path/arc that the foot goes through during the kick.
d. The boot impact location is detected, e.g., using the systems and/or methods described above.
e. The ball spin rate and velocity are then recorded and/or broadcast by the ball via wireless communication (or this data is stored).
f. All reported information is compiled to understand the total kick type, and all kicks are then aggregated to create a histogram (or similar graphical or tabular data or information) showing the number of specific kick types (e.g., a left-to-right curving kick, a straight kick, a right-to-left curving kick, the degree of curvature, high trajectory kicks, low trajectory kicks, kick speed, kick distance, etc.).

This data may be used to produce a graphical display illustrating the projected ball trajectory and/or distribution of kick types on a computer display.

As another alternative, if desired, this kick type distribution information may be combined with player-to-ball proximity sensing systems and methods described above to determine when a kicked ball reaches a teammate. This data can be used to produce various pass metrics, such as a pass distribution metric (e.g., number of passes to various teammates, types of passes to teammates, etc.).

**Fig. 41 - Leg Power:**

Systems and methods according to this example aspect of the invention use sensing systems to correlate ball speed and/or other ball flight characteristics to the path traveled by the foot before striking the ball. By determining the amount of "backswing" of the foot, it can be determined how much power the athlete is able to put into the ball given a specific backswing.

As some more specific examples of making this leg power determination, an impact sensing system inside the ball (e.g., accelerometer, pressure sensor, etc.) is matched to an inertial sensing system inside the boot to time-match impacts to enable determination of when the ball is struck by a specific foot, as well as to sense the path that the foot has traveled. A "leg power" metric may be determined in the following way:

a. An inertial sensing system inside the boot detects the distance/amount of travel the foot moves in the backward direction. Optionally, the inertial sensing system can detect when the moment the foot stops during the backswing and begins to move forward and then detects the amount of forward movement the foot travels before striking the ball.
b. At the time of impact, the ball and shoe sensors simultaneously record an impact, and that information is shared via wireless communication (or stored).
c. Pressure and accelerometers inside the ball report the speed of the ball immediately after the kick. Optionally an inertial sensor inside the ball could record speed.
d. Ball speed and foot travel path can then be correlated to determine how far the boot traveled before striking the ball.
e. Leg power is inversely proportional to the amount of distance the foot covered before the ball was struck, and is directly proportional to the speed of the ball immediately after impact. As another option, the peak pressure inside the ball can be used instead of the true ball speed, as the peak pressure will correlate to ball speed. As another option, the magnitude of acceleration of the ball immediately after kick may be used as opposed to the ball speed because these values will correlate to one another as well.

The leg power metric can provide useful data for a player or coach, e.g., to identify stronger players, to identify areas of individuals needing work or training, to compare one leg's capabilities and use against the other leg, etc.

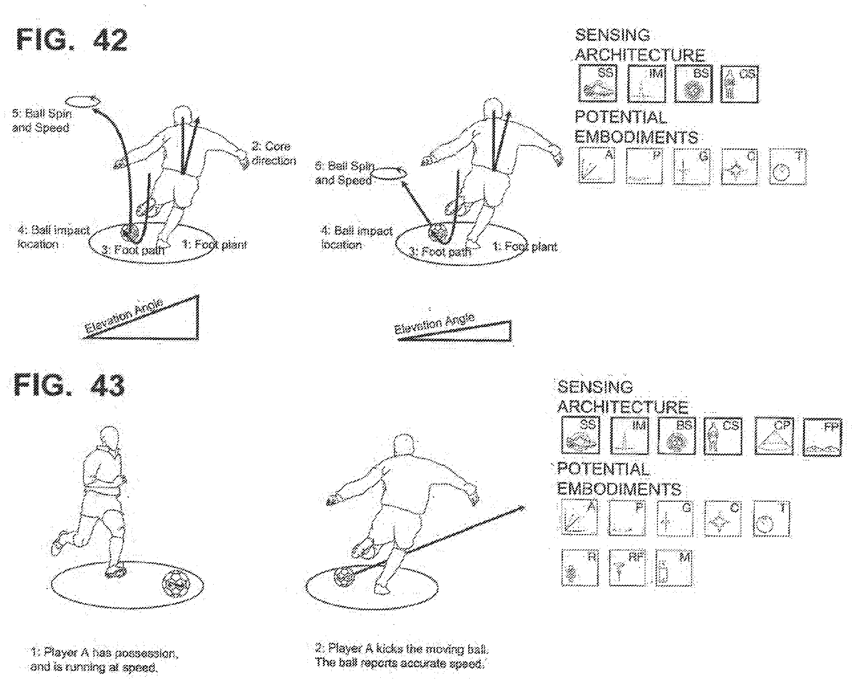

**Fig. 42** - **Kick/Pass Style:**

This example aspect of the invention provides a sensing system that can determine the type of kick that was made on a soccer ball. As one more specific example, this example aspect of the invention allows the system to differentiate between a lofted ball flight v. a ball flight that is closer to or along the ground.

Output from an impact sensing system inside the ball (e.g., accelerometer, pressure sensor, etc.) is matched to a rotational sensing system also provided with the ball (e.g., a compass sensor, gyro, etc.), and a lofted kick may be differentiated from an on-the-ground (or closer to the ground) kick, for example, by the following steps:

1. The impact sensing system in the ball senses an impact simultaneously to sensing of an impact by the inertial sensing system in the boot, thereby identifying that the ball has been kicked.
2. Inertial and rotational sensors in the ball then sense whether the ball is in free flight, e.g., defined by the rate at which the ball is slowing down and/or losing altitude. Additionally, rotational sensors sense a consistent rate of rotation (or a relatively consistent rate of rotation) indicating the ball is in the air.
3. If inertial and/or rotational sensors sense a dramatic reduction in speed due to friction or interaction with the ground, or a rapidly changing rate of rotation the ball, these features can indicate that the ball is rolling on the ground.

Different kick types may be advantageous at different times and/or under different circumstances in the game. This metric can allow determination of these different kick types, which also allows determination of the player's effectiveness at using these different kick types (e.g., by determining which kick types or the percentage of specific kick types that resulted in a successful pass to a teammate or that scored a successful goal, etc.).

**Fig. 43** - **Kick Power at Speed:**

Determination of this metric may use various data and metrics described above in this application. For example, using an on-body or in-shoe sensing system (such as a three-dimensional accelerometer or a piezoelectric sensor element) to determine player speed, as well as proximity/possession technology described above, systems and methods according to at least some examples of this invention further may determine the ability of a player to put significant impact force into kicking the ball while running at speed (a "kick power at speed" metric). The ball sensor(s) and the body-worn sensor(s) communicate their respective status, and this data then may be recorded on either of the two devices (or transmitted to an external device) for future visualization. This metric can be used as a skill metric to determine how much ball control a player has while at their top speed. As some more specific examples, any kick made while travelling at 75% of the player's top recorded running speed or higher (e.g., that particular game's top running speed, or an overall top running speed in all of the player's collected data), optionally traveling at 75% of the player's top recorded "on-ball" running speed or higher, may be a candidate for determining the kick power "at speed" metric so that high kick powers generated at relatively low speeds are not considered for inclusion in this metric.

If desired, this information may be displayed or visualized on a web page or hand-held device (such as a mobile phone) and compared with other metrics gathered by the system in previous and future games.

As an alternative, some ball speed sensing technology only has the ability to determine a relative change in velocity. For example, if the ball is already moving at 10 m/s and it is kicked such that the ball accelerates to 50 m/s, limitations of this technology force it to report only a 40m/s data value. In such a situation, the "kick power at speed metric" may be determined using an on-body (or on-shoe) speed measuring system to wirelessly communicate with the ball sensor system, which can then modify the reported ball speed value based on the speed of the player, thereby turning the measured value from a relative metric into an absolute ball speed metric, which may have been determined to be "on-ball speed" using technology described above.

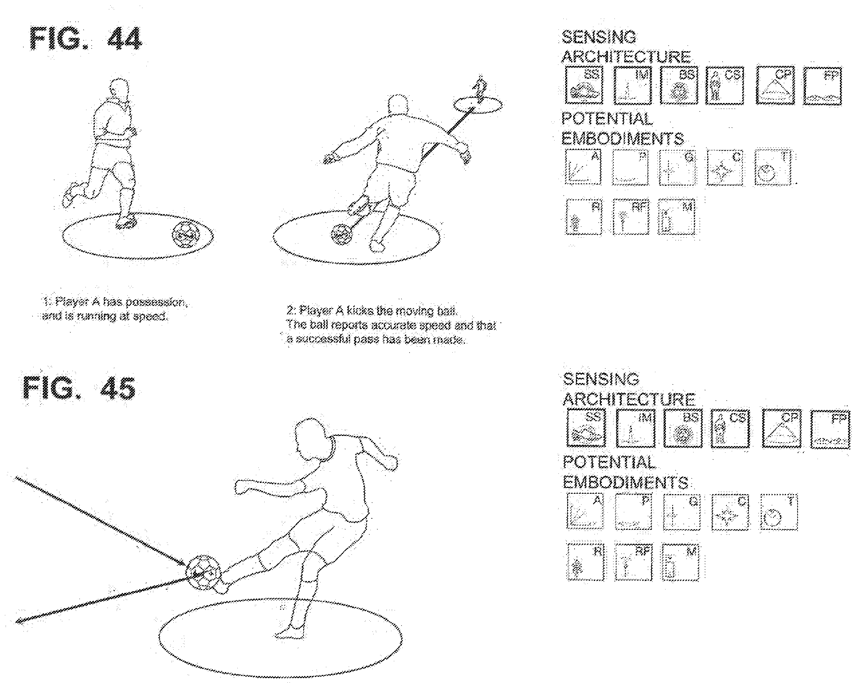

**Fig. 44** - **Pass Accuracy at Speed:**

This example aspect of systems and methods according to the invention measures the metric of pass accuracy (e.g., successful passes to teammates) with the additional passing player's speed associated with it. Using an on-body or in-shoe sensing system (such as a three-dimensional accelerometer or a piezoelectric element) to determine player speed, as well as player-to-ball proximity/possession technology described above, systems and methods according to at least some examples of this invention can measure the ability of a player to accurately pass to a teammate while moving at higher running speeds speed. More specific examples of measuring this metric follow.

Via wireless communication methods, the ball sensor and body-worn sensors communicate their respective status (e.g., player making the kick, the player receiving possession after the kick, the speed of the player making the kick, etc.) which is then recorded on either of the two devices (or transmitted to an external device) for future visualization and review. This metric can be used as a skill metric to determine how much ball control a player has while running at or near their top speed (e.g., while travelling at 75% of the player's top recorded running speed or higher (e.g., that particular game's top running speed, or an overall top running speed in all of the player's collected data), optionally while traveling at 75% of the player's top recorded "on-ball" running speed or higher, etc.).

If desired, this information may be displayed or visualized on a web page or hand-held device (such as a mobile phone) and compared with other metrics gathered by the system in previous and future games.

**Fig. 45** - **Volley:**

This example aspect of the invention measures information regarding volleys. For determining this information, systems and methods according to at least some examples of this invention use inertial and/or pressure sensing systems within the ball to determine ball speed. Wireless communication capabilities also may be provided within the ball to broadcast the ball speed information, as well as an exact time of impact (alternatively, this data may be simply stored). Additionally, inertial sensing systems may be provided as part of boot of the players, such as an accelerometer, a piezoelectric element, or other device. In such systems and methods, a volley can be determined by detecting coincident impacts to the boot and ball of one player, with then an "in-air" signature signal from the in-ball accelerometer. If the next impact registered by the ball is coincident with an impact to another player's boot, this then signifies a volley where the ball never touched the ground in-between the initial kicker's boot and the receiver's boot. In such a situation, the receiver may be credited with a "volley". Volleys are an important metric because they indicate an ability to keep the ball moving in a rapid manner (which may help avoid defenses, particularly when the volley is coupled with a successful pass to a teammate, a scored goal, or other favorable event, which also can be detected by systems and methods in accordance with at least some examples of this invention).

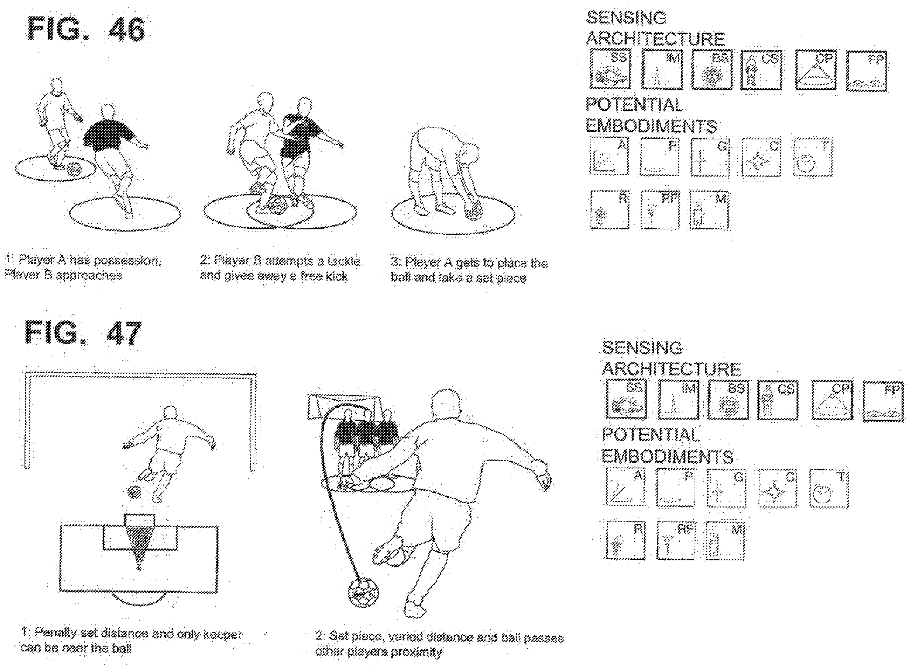

Figs. 46 through 50 illustrate various potential features for detecting and/or measuring various metrics relating to actions involved in sending the ball into play after a stoppage of play, such as an out of bounds event, etc. The features of these "set piece" feature determination systems, methods, and metrics will be described in more detail below.

**Fig. 46** - **Free Kick Awarded:**

Systems and methods according to at least some examples of this invention may determine when a free kick has been awarded. The free kick can be determined based on the combined technologies explained above for possession and tackle determination, as well as the technology described in more detail below for determining whether a set piece exists. More particularly, a free kick can be determined by the following steps:

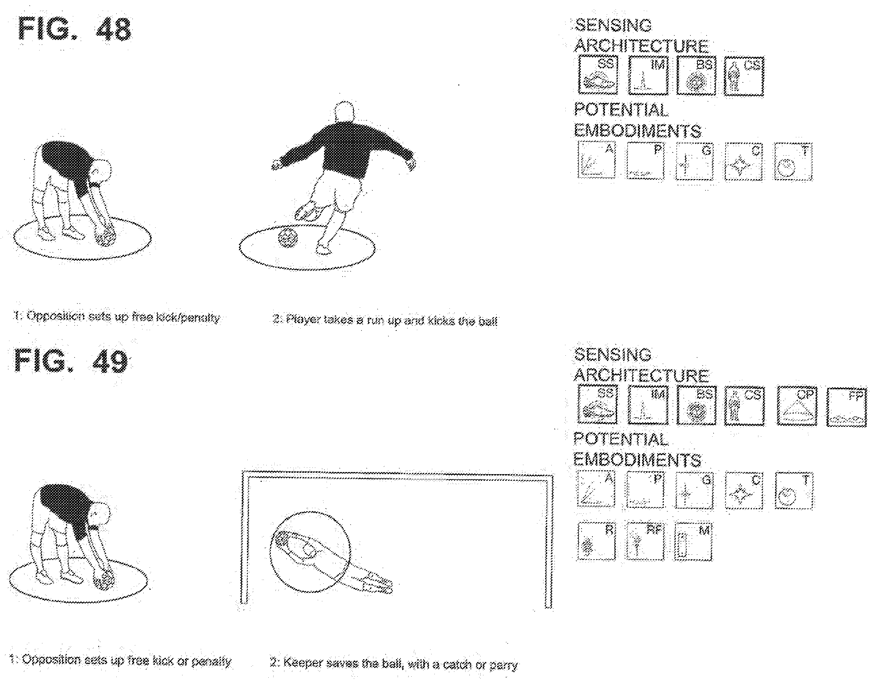

a. Possession of the ball is determined and awarded to a first player.
b. A second player comes into the area of the first player in possession of the ball (e.g., as determined by an attempted tackle, contested time, player-to-ball proximity, player-to-player proximity, etc.). This feature also may be determined, for example, based on person-to-person proximity and touching of the two people (e.g., as indicated by impact sensors provided on the players' bodies).
c. The ball detects a "set piece" play, as will be described in more detail below in conjunction with Fig. 48.

The "free kick" awarded metric may be a useful measure of the effectiveness of a defensive player or other information.

**Fig. 47** - **Free Kick v. Penalty Kick:**

Systems and methods for determining approximate flight distance of the ball are described above. Additionally, systems and methods for determining when the ball has been caught by the goalkeeper are described in more detail below. These features will be useful in automatically distinguishing a free kick from a penalty kick by systems and methods in accordance with examples of this invention.

A penalty kick is always kicked from the same spot on the field, where a free kick is not. Using an accelerometer and/or a combination of pressure sensor and an accelerometer, ball speed can be calculated. This example aspect of the invention uses time information from the kick to first impact within proximity of the keeper, combined with set piece knowledge (as described in more detail below) to determine if the kick was a penalty kick using ball distance. For example, if after a set piece determination the ball is kicked and comes into proximity of the goal keeper (or in contact with the goal keeper) within a certain time frame (e.g., depending on the ball speed), then it may be determined that the kick was a penalty kick. If no goal keeper proximity is detected after a set piece determination, or if no goal keeper proximity is detected within a predetermined time (e.g., depending on the ball speed), then it may be determined that a free kick occurred.

As an additional feature or an alternative feature, using a possession or proximity sensing system as described above, the two types of kicks may be differentiated. For example, a penalty kick, by definition, will not have other players (either offensive or defensive) within a very specific distance from the ball (as determine by the penalty box size). During the flight of the ball, a proximity sensing system (as described above) can determine whether the ball passed near any other players on its way to the goal. A free kick will always have defending players between the ball and the goal, and therefore, a shot on goal typically will register at least a brief proximity to a defensive player (at minimum) before reaching the keeper. As yet another example, player-to-player proximity detection may indicate two or more players on a team in tight proximity to each other (e.g., when in a wall position, as shown in Fig. 47), which also may be used as an indication that a free kick has occurred.

**Fig. 48** - **Set Piece Shot:**

"Set piece," as used in this context in this specification, refers to the soccer ball being placed on the ground for an ensuing penalty kick or free kick. It is an important metric for the player to know and distinguish "set piece kicks," as these tend to be the more difficult shots on goal during the game of soccer.

Using an accelerometer or other ball mounted inertial sensing system, it can be determined when a ball is not in motion (or when its motion is slow or minimal). Some more specific examples include, but are not limited to: a three-dimensional accelerometer in the ball, a three-dimension accelerometer combined with a gyroscope, an accelerometer in the ball combined with a compass sensor, ball movement speed and/or lack of rotation matching a player in proximity's speed, etc. One or more of these sensor outputs may be utilized to show the ball has been carried and placed, followed by the ball not moving, and then followed immediately by a kick (matching of boot impact to ball movement/pressure spike). While this kick could be a corner kick, a penalty kick, or a free kick, the type of kick may be determined, at least in some instances, by what happens next, e.g., by who's proximity it passes, by the next contact person, the timing between the kick and the next proximity, etc., e.g., as described above.

**Fig. 49 - Set Piece Save:**

This example aspect of systems and methods according to this invention determine when a kick after a set piece event (e.g., determined as described above) has resulted in a goal keeper save. As noted above, the term "set piece" refers to the ball being placed on the ground for an ensuing penalty kick or free kick, and it may be determined as described above.

As a more specific example, a set piece event may be determined by systems and methods according to this example aspect of the invention in the manner described above in conjunction with Fig. 48. Once a set piece event has been determined, and when the set piece event has included proximity to the goal keeper, a throw, pass, or drop kick initiated by the goal keeper may be detected (e.g., as described above and/or in more detail below) and used as an indication that the goal keeper successfully saved the kick resulting from the set piece event (e.g., by a goalkeeper catch or parry event). Various features of goal keeper save determinations will be described in more detail below.

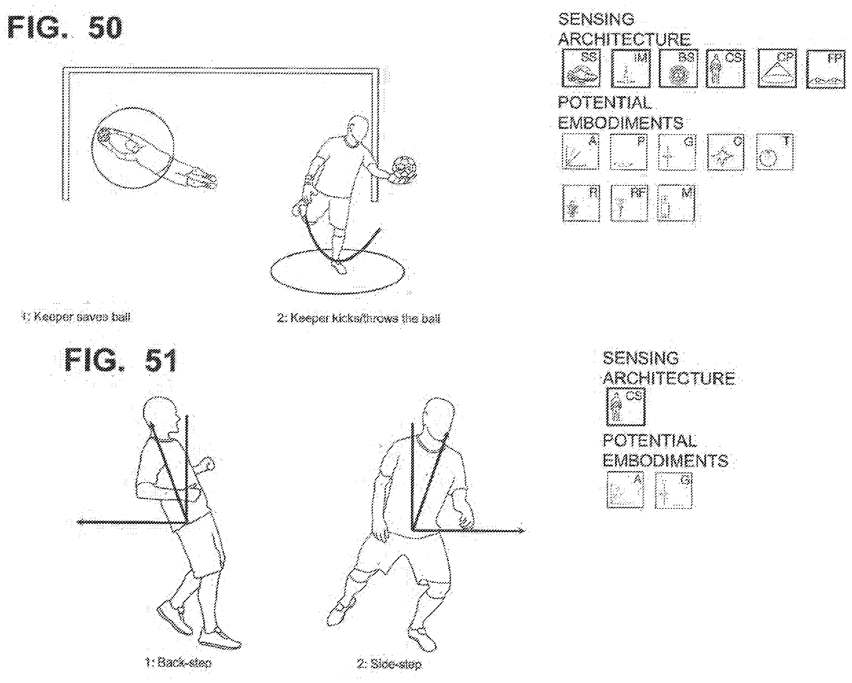

**Fig. 50 - Set Piece Kick - On Goal or Not:**

Example systems and methods for determining a set piece event are described above. This example aspect of systems and methods according to the invention uses the previously defined set piece sensing method and adds proximity/possession sensing systems and methods (such as magnetic sensing, RADAR, etc.), e.g., like those described above, to determine whether a set piece kick was "on-goal" or not. As a more specific example, when a set piece event has been determined, immediately followed by a kick, which is then followed by ball to keeper proximity, if the next event is a kick or a drop kick by the goal keeper, then a set piece save event may be determined.

Figs. 51 through 55 illustrate various potential features for detecting and/or measuring various metrics relating to player motion, e.g., during a game, practice session, training session, etc. The features of these systems, methods, and metrics will be described in more detail below.

**Fig. 51 - Direction of Movement Based on Body Angle:**

Systems and methods according to at least some examples of this invention will provide information regarding the direction of player movement, which may be based, at least in part, on the player's body angle during the motion. This determination may be made, in at least some example systems and methods according to this invention, using an "on body" accelerometer to sense the upper body's angle and translate this information into a direction metric. For example, when accelerating or moving in any direction (e.g., forward, backward, to the side, etc.), the upper body tends to lean in the direction of acceleration. For example, when accelerating in the forward direction, the body leans forward. This angle and lean helps move the body forward, and the legs follow. Generally, the greater the acceleration, the greater the lean angle. This same feature also works for back steps and side steps.

Accordingly, by measuring the lean of the body, information regarding the player's movement direction (and optionally the intensity of this motion) can be determined. This metric may be useful for determining a player's ability (e.g., if an offensive player spends too much time backpedalling or sidestepping, etc.) and/or ascertaining areas for training and game improvement.

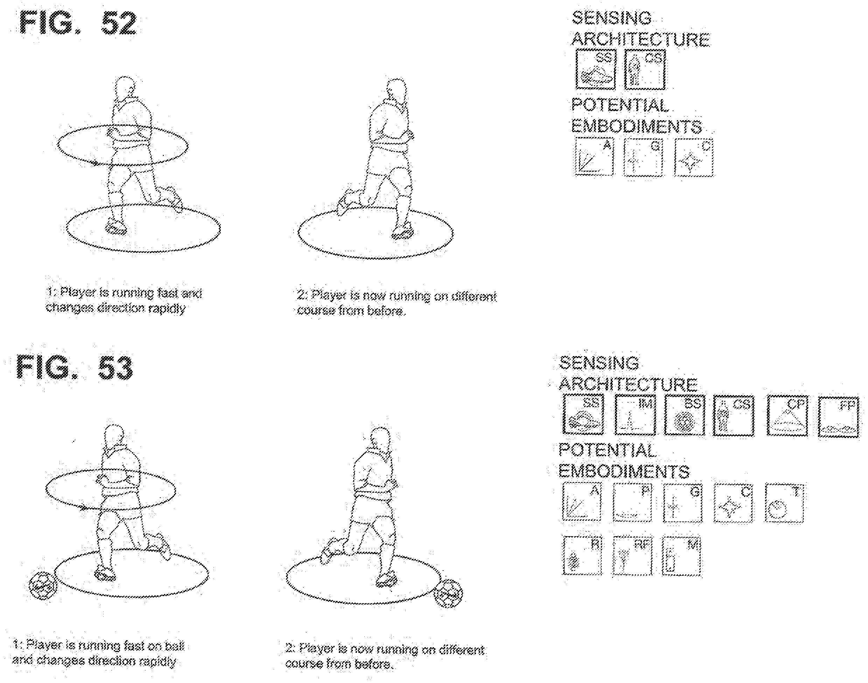

**Fig. 52 - Player "Turn In":**

This example aspect of the invention uses a sensing system on the player that determines player speed, such as an inertial sensing system, contact-time based pedometer system, etc., and a player mounted rotational sensor, such as a gyroscope, compass sensor, etc., to determine the amount of body rotation. Player "turn-in" can be defined as the amount of speed lost by the player during quick direction changes. This metric may be valuable in the game of soccer as a measure of a player's "quickness" or "agility." The acquisition of the "turn-in" metric may simply require the measurement of the speed sensing system before and after a measured rotation from the rotational sensing system. As one more specific example, the performance metric may be calculated by subtracting the player speed before the change in direction from the speed post rotation. Information relating to this metric can then be displayed or visualized on a web page or hand-held device (such as a mobile phone) and compared with other metrics gathered by the system in previous and future games. Moreover, information relating to this metric may be used to develop training programs to improve player quickness/agility.

**Fig. 53 - Player "Turn In" On Ball:**

This example aspect of the invention is similar to the "turn-in" determination as described above, but additionally includes the metric that the player is in possession of and/or in proximity to (and optionally maintains possession of and/or in proximity to) the ball. In other words, for any measured turn-in events, as described above, another metric can be developed for turn-in events that occur for the player while the player is in possession of or in proximity to the ball. This metric may be valuable with respect to the game of soccer as a measure of a player's "quickness" or "agility" while handling the ball or while closely defending the ball. Information relating to this metric can then be displayed or visualized on a web page or hand-held device (such as a mobile phone) and compared with other metrics gathered by the system in previous and future games. Moreover, information relating to this metric may be used to develop training programs to improve player quickness/agility while handling the ball.

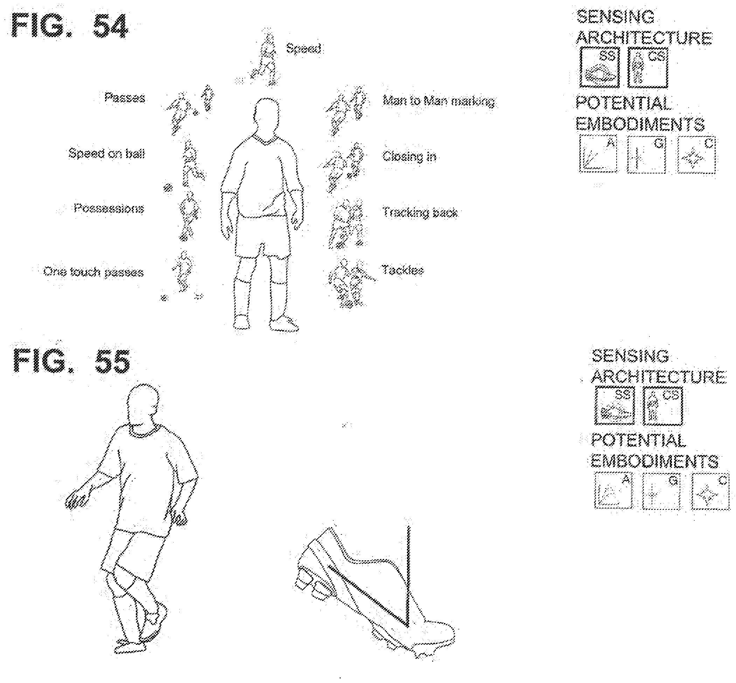

**Fig. 54** - **In Shoe Sensor Based Contextual Reporting:**

Athletic performance monitoring systems and methods according to at least some examples of this invention include an in-shoe sensing system for measuring speed and/or distance information (e.g., a pedometer type speed and/or distance sensor). This sensor also may provide contextual information about the specific part of sport the athlete is in, e.g., what types of activities he or she is performing, and this contextual information may be used by other portions of the athletic performance monitoring systems and methods (e.g., on body sensors, etc.) to change the kinematic models and/or algorithms used to determine the player's running speed and/or travel distance.

Output from the shoe based sensors (e.g., accelerometer, force sensors, etc.) may include a "signature" appearance that correlates to the type of activity being performed by the athlete. For example, the in-shoe based accelerometer output (e.g., the signal shape) may differ depending on whether the athlete is moving forward, moving rearward, side stepping, tackling, passing the ball, walking, dribbling, sprinting, slow running, skipping, jumping, sliding, sliding laterally, etc. By automatically determining the type of action with which the athlete is involved (using the shoe based sensor output), more specialized algorithms for determining player performance may be called up to enable a more accurate determination of the parameters involved in the player's performance. Different algorithms also may apply under other differing circumstances, for example, different speed and/or distance determining algorithms may apply depending on whether the player is on ball or off ball.

As one more specific example, because different in-shoe sensor waveforms may be involved in running forward or backward (e.g., different loft times, different pressure profiles, etc.), systems and methods according to examples of this invention may automatically determine whether an athlete is moving forward or rearward based on the characteristics of the sensor output. Because step size also may differ when moving forward as compared to moving backward, different algorithms for ascertaining speed and distance information may be called upon for providing speed and distance data, depending on whether the motion is forward or backward. Accordingly, this aspect of the invention allows for a more accurate determination of speed and/or distance based the determined manner in which the athlete is moving.

Moreover, metrics involving the type of movement or other actions performed by the athlete may be useful for the player or coach, e.g., to indicate whether an offensive player spends too much time backpedalling or sidestepping, to measure player's efforts and intensity, etc.

**Fig. 55** - **Time Spent on Toes:**

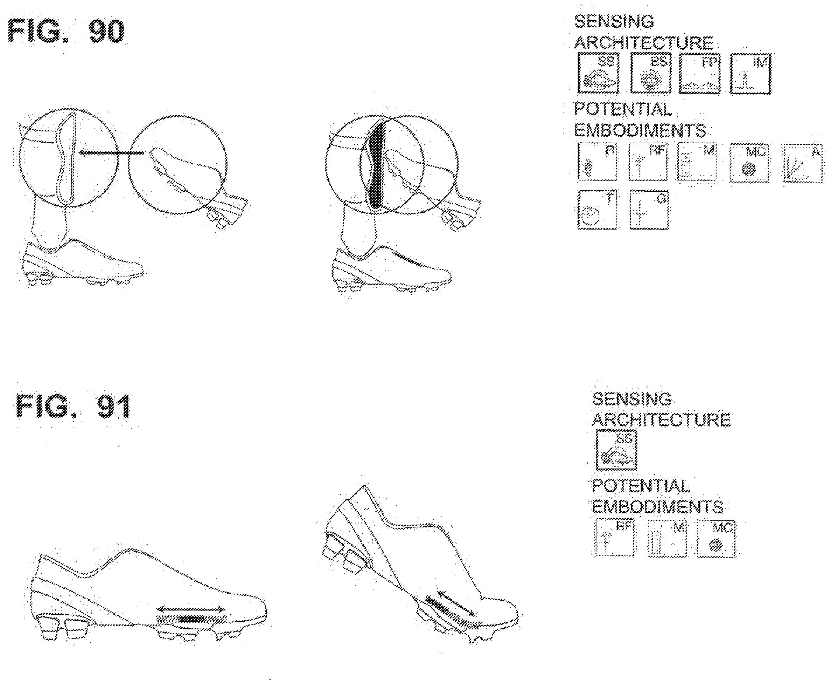

In sports and athletic performances, it is often important for the athlete to stay on his/her toes. Being on one's toes generally enables quicker reactions and/or indicates that the athlete is performing with more intensity (e.g., while sprinting, an athlete spends more time on his/her toes than when jogging or walking). Systems and methods in accordance with at least some examples of this invention may include an in-shoe sensing system that determines the foot angle so as to enable a determination of the amount of time the athlete spends on his or her toes. One more specific example of hardware for making this measurement may include an accelerometer that compares the gravity vector to the orientation of the sensor within the shoe. As another example, the shoe may include a rotational sensing system, such as a gyroscope. The shoe also may contain a measuring system like that described in more detail below in conjunction with Fig. 91. The determined information may be transmitted wirelessly to another system for processing and/or stored. The finally determined metric may include, for example, the total amount time on one's toes, the percentage of time spent on the toes, the percentage of actual movement (or running) time spent on the toes, etc.

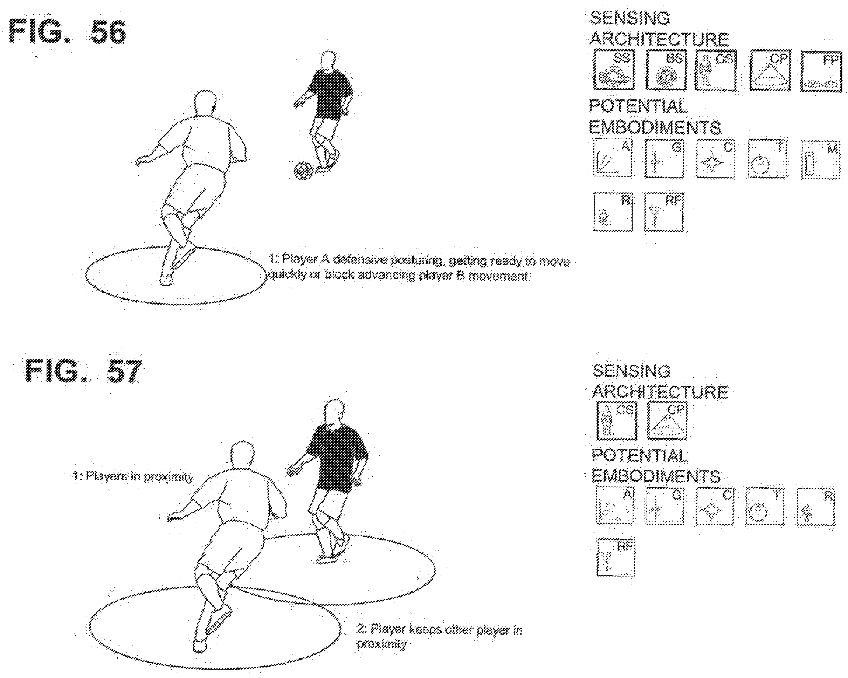

Figs. 56 through 65 illustrate various potential features for detecting and/or measuring various metrics relating to playing the game of soccer, which may be used and evaluated during a game, practice session, training session, etc. The features of these systems, methods, and metrics will be described in more detail below.

**Fig. 56** - **Player Posturing:**

"Player posturing" is the determination of the ball movement direction as it relates to the player's core facing direction. Using this information, one can determine if a player is in a defensive posture, in an aggressive or attacking posture, etc. The hardware used for determining this metric, in at least some example systems and methods according to this invention, include: a directional sensing system inside the ball (such as a compass sensor, accelerometer/gyro combination, etc.) to give ball movement direction; and a body-mounted sensor of similar architecture (compass sensor, accelerometer/gyro, etc.) to give player facing direction. The following example steps may be used to determine a "player posturing" metric:

1. Using inertial sensors in the ball, the direction the ball is moving (rolling or in flight) is determined.
2. Using a core-mounted sensor (such as gyro, compass, etc.), the direction the body core is facing is determined.
3. Combine these two pieces of information allows a determination of the relative ball motion to core facing direction, to help understand contextually what is happening between the player and the ball.

Additionally or alternatively, core worn sensors between opposing players can be used separately (or added to the above) to determine the player to player relationships, and therefore enrich the data-set to build more confidence on the posturing. For example, the direction of motion (and/or the facing direction) of the player in possession of the ball can be compared to the direction of motion (and/or the facing direction) of the defensive player to provide additional information relating to this "player posturing" metric.

**Fig. 57 - Man to Man - Opposing Position:**

The determination of what opposing player a particular player had been marking can be a useful piece of information when determining a player's performance metrics. Systems and methods according to at least some examples of this invention will use proximity determination methods as described above, but this technology will be used on each individual player to provide player-to-player proximity data and information.

As one alternate, if desired, peer-to-peer networking technology may be used to determine and track proximity between players (as well as between other elements within systems and methods according to at least some examples of this invention). When two players are close enough to establish a peer-to-peer communication channel (e.g., between devices that they are carrying, such as shoe mounted sensors, body core mounted sensors, etc.), this could be established as a proximity event. By tracking and timing such proximity events, systems and methods in accordance with these examples of the invention will know which nodes of the network (e.g., which other players) a given player was in communication range with during the majority of the game. As players get further away from each other, they may get out of range (and thereby break the peer-to-peer communication channel). Other ways of determining player-to-player distance may be used without departing from this invention. If desired, a "heat map" or other graphic display may be provided to indicate the opposing team players with which any given player most stayed near during the course of the game, and this will allow a determination of the player being defended or marked during the game.

As another alternative, some RF modules have RSSI ("radio signal strength indicators"). RSSI technology can be used on each player to determine which player was closest to another player for the majority of the game.

The Opposing Player metric may be useful, for example, to determine a defensive player's relative performance with respect to the player or players that he was defending (e.g., goal scoring effectiveness, successful passing, successful interceptions, etc.).

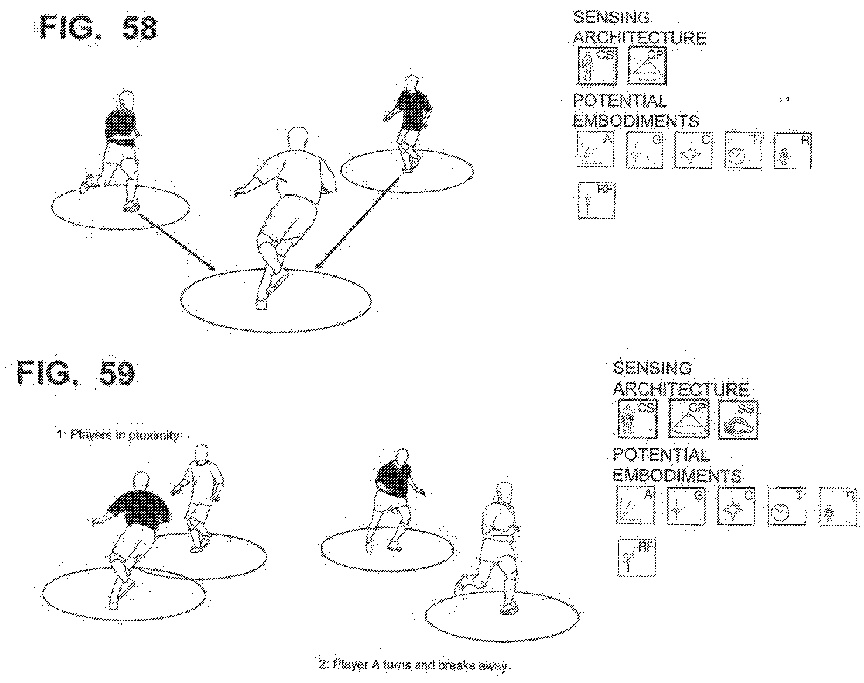

**Fig. 58 - Drawing Opposition:**

The Man to Man Opposing Position detection capability described above can be combined with other metrics to provide additional interesting data and information relating to soccer (or other sports). For example, combining the Man to Man Opposing Position detecting capability with player-to-player proximity detection and player speed determination (e.g., in boot inertial sensors, as described above) may be combined to provide a metric relating to the ability of a player to draw the opposition. Using an inertial based sensing system, sprints or bursts of speed can be measured and combined with the player-to-player proximity to determine if a player is drawing opposition. Example systems and methods according to this aspect of the invention follow.

First, proximity sensing systems and methods as described above can determine when two players are near each other. If one player sprints away and the proximity detection system shows no players near him and shortly thereafter an opposition player is detected by a proximity sensor again, this suggests that the initial player (the one that initially sprinted away) has pulled the opposition players with him. Ball possession determinations also may be used in such systems and methods (e.g., to determine the player's ability to pull opposition even without the ball).

Additionally, if desired, skill metrics can be created based on the amount of time a player spends within proximity of the opposing player. If a player is meant to be in an offensive position (striker), the more time spent away from an opposing player the better. On the other hand, a defensive player could be considered better the more time he/she spends in proximity to the opposition.

**Fig. 59** - **Breakaway Speed:**

The Man to Man Opposing Position detection capabilities as described above open the door to yet determination of additional information and metrics. As another more specific example, an inertial sensing system can be placed on the cores or boots of the athletes and a comparison can be made between the relative accelerations of each player at the same time. Such a system may be used to determine a "breakaway speed" metric.

An example system and method according to this invention for determining breakaway speed comprises a speed detection system and combines this information with a wireless communication system to determine coincident accelerations of two players. The relative speeds of the two players can be determined (optionally coupled with directional information), and this information then can be used to produce a performance metric, e.g., determining whether the player was faster than the player defending him/her (e.g., were you faster than the player that was marking you, etc.).

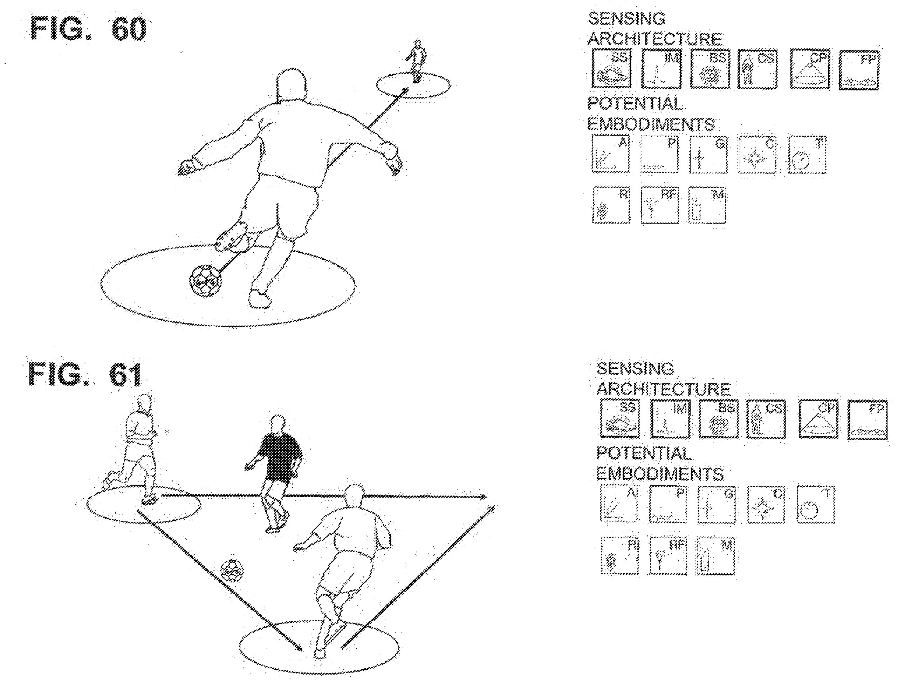

**Fig. 60** - **Successful Pass:**

Completion of a successful pass is incredibly important in the game of soccer (and other sports). The following describes an example system and method for determining when a successful pass event has occurred (e.g., a "successful pass" means a pass from one teammate to another).

In this example system and method, output from an impact sensing system inside the ball (accelerometer, pressure sensor, etc.) is time matched to output from an impact sensing system inside the boot to enable determination of when the ball is struck by a specific foot. A ball proximity sensing system is also employed (magnetic sensing, RSSI, etc.) to enable determination of when the ball is in proximity to a player. A successful pass is determined by systems and methods according to this example of the invention in the following steps:

a. Ball possession by a specific player is determined, e.g., as described above.
b. Kick impacts are registered both on the in-shoe sensor and the in-ball sensor.
c. The ball leaves the proximity of the player that kicked it.
d. The ball enters the proximity of a teammate, as determined by the proximity sensing system.
e. Impacts are measured simultaneously by the teammate's boot and the ball, and a successful pass is recorded.

Determination of the number of successful passes and the number of unsuccessful passes are useful metrics for evaluating the performance of the player.

**Fig. 61** - **Give and Go:**

The "give-and-go" is another common play in the game of soccer. The following describes one example sensing system, method, and logic that may be used to interpret the various sensor signals for determining when a "give-and-go" event has occurred.

Output from an impact sensing system inside the ball (accelerometer, pressure sensor, etc.) is time matched to output from an impact sensing system inside the foot to enable determination of when the ball is struck by a specific foot. A ball proximity sensing system is also employed (magnetic sensing, RSSI, etc.) to enable determination of when the ball is in proximity to the player. A give-and-go event may be determined in the following manner:

a. First, ball possession by Player A is determined, e.g., as described above.
b. A kick by Player A is registered on Player A's in-shoe sensor and the in-ball sensor.
c. The ball leaves the proximity of Player A.
d. The ball enters the proximity of a teammate, Player B, as determined by the ball proximity sensing system.
e. Impacts are measured simultaneously by Player B's boot and the ball (i.e., a successful pass is recorded).
f. The ball leaves the proximity of Player B (e.g., by a kick by Player B).
g. The ball enters the proximity of Player A and contacts Player A's boot (another successful pass).

Optionally, a successful give-and-go event may require successful passes from Teammate A to Teammate B and back to Teammate A within a predetermined time frame (e.g., in less than 5 seconds). The determination of this event also may require the ball to pass in proximity to, but not into the possession of, a player on the opposing team (e.g., a "Through Ball/Pass" event, as described below). Successful "give-and-go" events help provide a measure of how well groups of players work together and move the ball on the pitch.

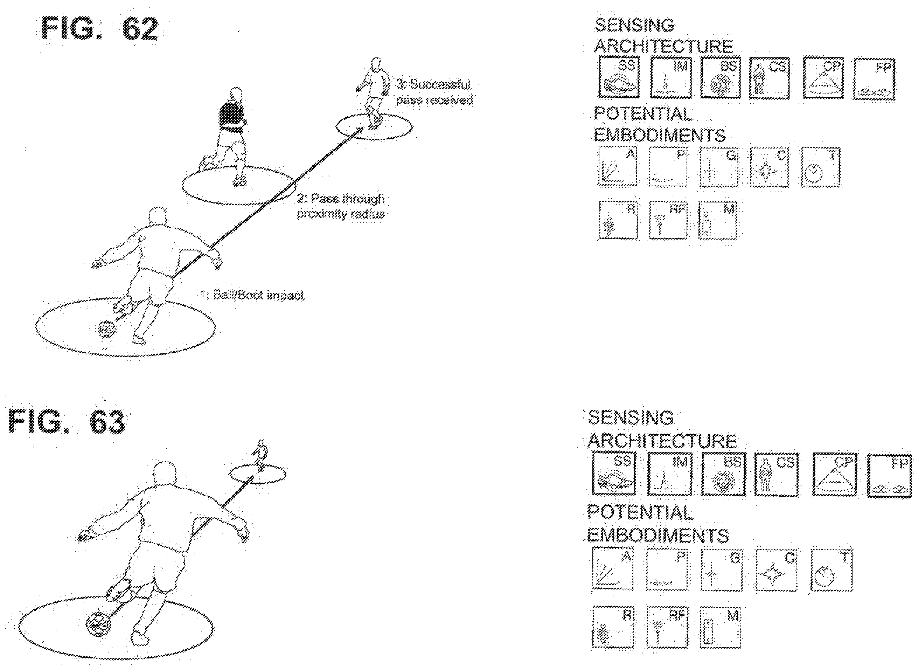

**Fig. 62 - Through Ball/Pass:**

Another interesting metric that may be measured by systems and methods in accordance with at least some examples of this invention relates to determination of a "through ball" or "through pass" event. A "through ball" or "through pass" as used herein in this context means that the ball is successfully passed from one teammate to another and, during the course of the pass, the ball passes in proximity to an opposition player. In some examples of such systems and methods, output from an impact sensing system inside the ball (accelerometer, pressure sensor, etc.) is time-matched to output from an impact sensing system inside the boot to enable determination of when the ball is struck by a specific foot. A proximity sensing system is also employed (magnetic sensing, RSSI, etc.) to enable determination of when the ball is in proximity to the players on the field. Then, a "through ball" or "through pass" event is determined by the following steps:

a. A player on team "A" is determined to have possession of the ball.
b. Impacts are registered on both the shoe sensor and the ball sensor simultaneously, registering a kick by a player on team A.
c. The ball leaves the proximity of the player that kicked it.
d. The ball is determined as having passed through the proximity of one or more players on the opposing team.
e. The ball enters the proximity of a teammate to the original kicking player (team "A"), optionally, a player that has been running forward onto the ball.
f. The ball sensor and the kick receiving teammate's shoe sensor simultaneously register an impact and optionally continued proximity to the teammate (beginning a ball possession event by the receiving player).

Optionally, if desired, the ball must pass in proximity to one or more players on the opposing team without the opposing team contacting and/or possessing the ball. This metric may be useful for evaluating the performance of players and their passing skills in a more closely defended environment.

**Fig. 63 - Pass Distribution:**

Pass distribution information also may be an interesting and/or important metric for soccer players to consider and evaluate. As some more specific examples, a determination of a direction of a pass (e.g., advancing the ball, retreating, etc.) may be useful in evaluating player performance.

Output from an impact sensing system inside the ball (accelerometer, pressure sensor, etc.) may be time matched to output from an impact sensing system inside the boot to enable determination of when the ball is struck by a specific foot. Additionally, a rotational sensing mechanism (such as a magnetic sensor, gyro, etc.) inside the ball may be used to enable determination of an absolute direction of movement of the ball. A pass distribution metric may be determined through the following steps:

a. Direction of play is determined, e.g., as described herein.
b. Possession is determined, e.g., using techniques like those described above.
c. Simultaneous impacts to the boot and ball are recorded and communicated wirelessly (or stored) to indicate the ball has been kicked by a specific player.
d. Inertial sensors inside the ball are then used to determine the relative direction of flight of the ball.
e. Rotational sensors then record the absolute orientation of the ball as a result of the kick.
f. The two pieces of information from steps d and e can be used to determine the relative direction of ball flight to the direction of play determined in step a. This information can be then compared and evaluated to determine if the kick was advancing on the opponent or retreating, sent to the player's right or left, etc.
g. The final step is a possession determination awarded to a teammate, in order to call it a complete and successful pass.

The steps above constitute a determination of a successful pass between teammates. If, in step number g, the ball is detected to be in possession of the opposition team, this is also useful information. The direction of all passes made by a player throughout a game can be aggregated to determine pass success/failure rate when trying to advance/retreat the ball, as well as the amount of time the player moves the ball forward or retreats over the course of a game.

Finally, if desired, a core mounted directional sensor (e.g., compass, etc.) can be used to determine what movement/facing direction changes occur as a result of a player receiving the pass. Therefore, it is possible to use this technology to help give performance metrics, such as how often the teammate had to come to the ball, wait for the ball, or if the pass was laid out perfectly in front of the player.

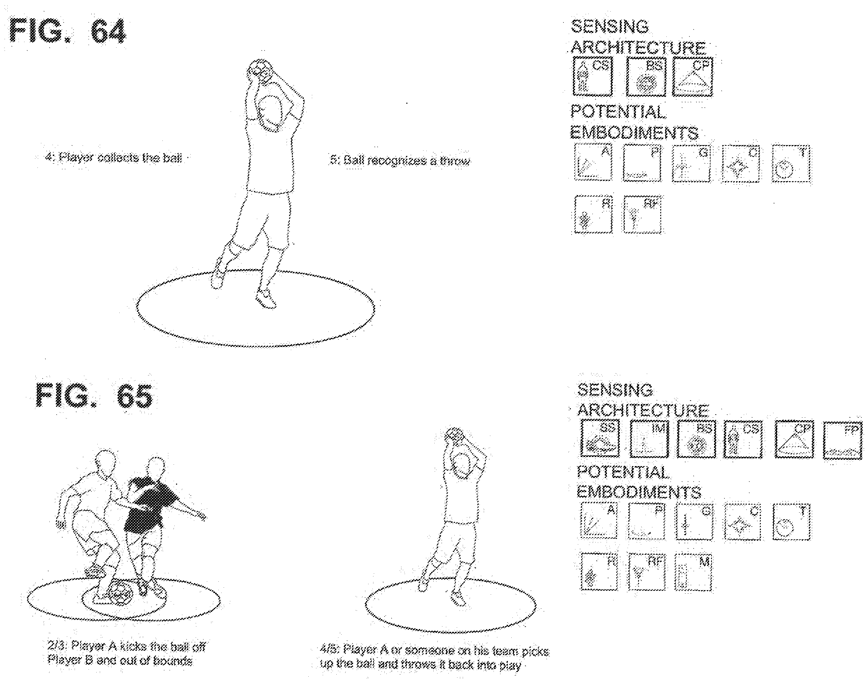

**Fig. 64** - **Out of Bounds:**

In order for an athletic performance monitoring system and method to understand the play of a soccer game, the system and method should not take into account possession, kicks, and other activities that occur when the ball is out of play. The following is an example of a system and method that may be used to determine when a ball has gone out of bounds.

Output produced by an impact sensing system inside the ball (e.g., accelerometer, pressure sensor, etc.) is time-matched to output produced by an inertial sensing system inside the boot to enable a determination of when the ball is struck by a specific foot, and optionally, to enable determination of the path that the foot has traveled. A proximity sensing system also may be employed (e.g., magnetic sensing, RSSI, etc.) to enable a determination of when the ball is in proximity to particular players on the field. One example process that may be used to determine when the ball has gone out of bounds is as follows:

1. An individual player possession is determined using technology/procedures as described above.
2. Optional: the ball detects a kick by the simultaneous impulse on the inertial sensing systems within the boot and the pressure/acceleration sensing systems in the ball.
3. Optional: the ball is detected to be within the radius of proximity of an opposing player.
4. Inertial sensors in the ball detect when the ball has been picked up (e.g., identifying the low frequency signals as compared to foot/ground impacts; identifying no motion, slow motion, or low spin motions for extended play; identifying speed of motion consistent with player's speed in proximity to the ball (i.e., the player holding the ball); etc.).
5. The ball either detects a throw-in or a set piece play using previously described methods.

Once this type of "out of bounds" situation is detected, systems and methods according to at least some examples of this invention can adjust the various determined metrics, such as possession time (e.g., by deducting from the determined possession time for an individual player or team the length of time between the throw-in or set point event and the previous kick (which induced the out of bounds event), etc.). Other metrics also may be adjusted based on "out of bounds" determinations without departing from this invention.

**Fig. 65** - **Intentional Out of Bounds:**

In a specific subset of normal "out of bounds" situations, as described above, sensing systems and methods in accordance with at least some examples of this invention may differentiate situations when a ball has been intentionally kicked against another player to send the ball out of bounds, resulting in maintaining possession. The same equipment may be used as described above in conjunction with Fig. 64, but additionally, ball proximity to another player and/or ball impact with another player also may be detected and relevant to the "intentional out of bounds" situation. The following example process may be used for detecting an intentional out of bounds situation:

1. An individual player possession is determined using technology/procedures as described above.
2. The ball detects a kick by the simultaneous impulse on the inertial sensing systems within the boot and the pressure/acceleration sensing systems in the ball.
3. The ball then detects another impact that does not coincide with a boot impact for any other player on the pitch (optionally, the ball also may be detected to be within the radius of proximity of an opposing player).
4. Inertial sensors in the ball detect when the ball has been picked up (e.g., as described above).
5. The ball either detects a throw-in or a set piece play using previously described methods.

Information relating to the ability of a player to induce an intentional out of bounds situation on the opposing team can be useful in ascertaining the skill of the player causing the intentional out of bounds situation (e.g., ball handling skills, defense avoidance skills, etc.), as well as the skill level of the defensive player against whom the ball was kicked to produce this situation.

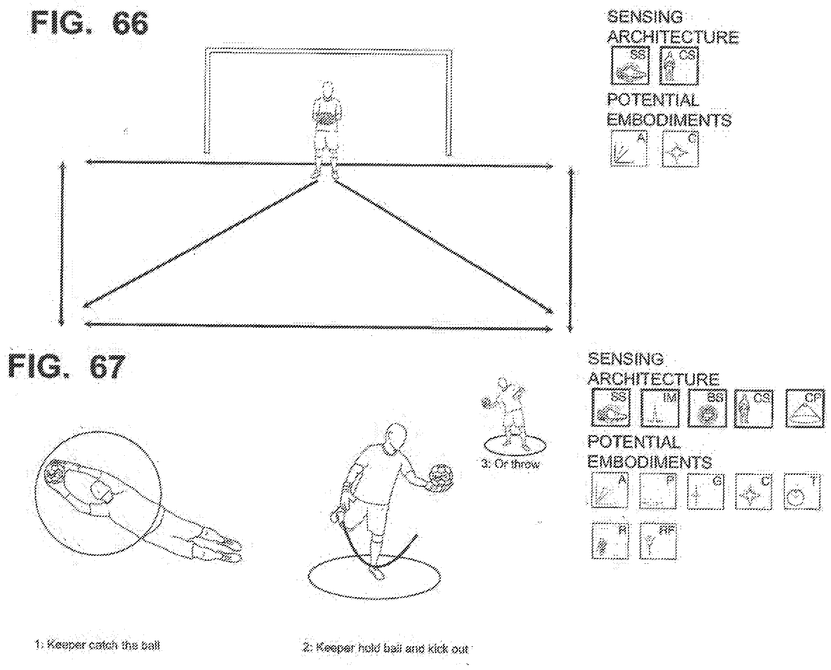

Figs. 66 through 75 illustrate various potential features for detecting and/or measuring various metrics relating to goals and/or activities of the goalkeeper in the game of soccer, which may be used and evaluated during a game, practice session, training session, etc. The features of these example systems, methods, and metrics will be described in more detail below.

**Fig. 66** - **Keeper Recognition:**

While systems and methods according to at least some examples of this invention may request input or special equipment for the goal keeper, if desired, at least some systems and methods according to examples of this invention may be capable of automatically identifying which player is the goal keeper based on detected activities that occur over the course of a game.

Example hardware for use in recognizing the goal keeper may include: (a) an inertial sensing system on the player (e.g., either on the core or in the boot) to provide player speed and distance information; and (b) a wireless communication system to allow the sensing systems on the individual players to broadcast their signals/processed data (or storage capabilities for this data). Then, as one example, the automatic determination of the keeper may be accomplished in the following way:

a. Speed and distance information is collected and considered for each player on the pitch.
b. The keeper, due to his/her position, will do the majority of his/her movement within an 18 yard box located near the goal.
c. After (or during) the game, the data from the sensing system can be evaluated to understand which player on the pitch moved the least, and stayed predominantly within an 18 yard box.

Different performance metrics (e.g., the performance metrics described in more detail below) may be determined for the player identified as the goal keeper.

As another alternative, if desired, the goalkeeper may be equipped with gloves that have the capability of determining contact with and/or proximity to the ball (e.g., impact sensors, accelerometers, ball-to-glove proximity sensing systems, etc.). Data collected by such gloves also may be used in various ways for determining various metrics, such as the metrics described in more detail below. As yet another example, systems and methods according to examples of this invention may allow the various players to enter data identifying their positions.

**Fig. 67** - **Save/Goal Protection:**

Systems and methods according to at least some examples of this invention may include features to enable determination of goal keeper saves and protection of the goal. This aspect of the invention may be accomplished using various sensors to determine when a keeper saves a shot on goal. For example, systems and methods according to at least some examples of this invention may utilize an inertial sensor on the body core of the keeper, a ball proximity sensing system, and an inertial sensing system within the ball, e.g., of the various types described above. A determination of an impact to the ball with significant magnitude (e.g., above a threshold level, such as would be present in a typical shot on goal, or a header off of a corner kick, for example), immediately followed by (or simultaneous with) ball proximity to the keeper, followed by a picked up ball, and then a drop kick or throw, may be used an indication that the goal keeper has saved a shot on goal (and successfully protected the goal). Additionally or alternatively, if desired, the goalkeeper may be equipped with gloves that have the capability of determining contact with and/or proximity to the ball (e.g., impact sensors, accelerometers, ball-to-glove proximity sensing systems, etc.), and such contact may be an indication of goal keeper interaction with the ball. As another alternative, sensor data taken from the goal keeper’s body-worn accelerometer could be compared to sensor data from the accelerometer data in the ball. As the keeper runs or moves with the ball, the two sensors will indicate a very similar net path taken. This data can be used to determine possession of the ball by the goal keeper.

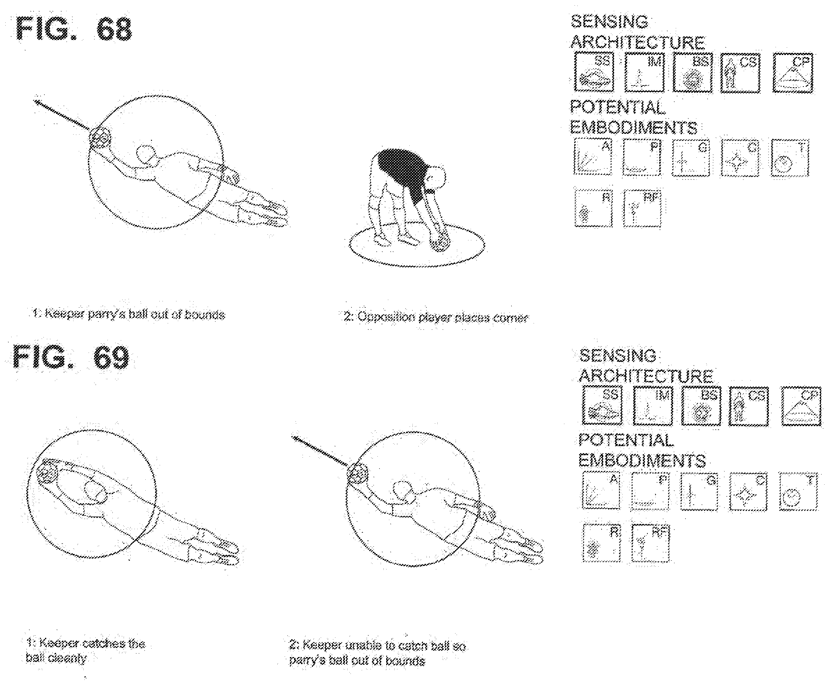

**Fig. 68** - **Keeper Parry:**

This example aspect of the invention relates to systems and methods capable of determining a "keeper parry" scenario, i.e., a situation where the keeper gets his hands (or other body part) on a shot on goal, which deflects the ball out of bounds (e.g., outside the goal, over the goal, etc.). As a more specific example, using inertial and pressure sensing systems inside a soccer ball, the ball will generally show a softer impact signature on the accelerometer and/or the pressure sensors when it contacts a player’s hands, as compared to a goal-post impact, kick, or ground impact. This unique sensor signature and determination of a non-shoe/ground/goalpost impact, combined with detection of proximity to the keeper, followed by a set piece event (as described above, e.g., a corner kick), is a unique sequence of events that only happens when a keeper parry event occurs. Additionally or alternatively, if desired, the goalkeeper may be equipped with gloves that have the capability of determining contact with and/or proximity to the ball (e.g., impact sensors, accelerometers, ball-to-glove proximity sensing systems, etc.), and fleeting contact or proximity of the glove to the ball may be considered an indication of a keeper parry situation (optionally, combined with some of the other features of this scenario described above).

**Fig. 69** - **Hard Shot Keeper Parry or Catch:**

This example aspect of the invention involves determination of a keeper parry event or keeper catch of the ball that has been kicked hard. Defending against a hard shot will typically require improved goaltending skills, and the ability to differentiate saves in this situation may provide an additional interesting metric for coaches or players to consider. Systems and methods according to at least some examples of this aspect of the invention may use inertial and/or pressure sensing systems within the ball to determine ball speed as well as wireless communication capabilities included with the ball that are capable of broadcasting ball speed information and impact time information. Furthermore, systems and methods according to at least some examples of this invention further may include proximity and/or possession determination technology (such as magnetic, RF, or other) that allows a determination of when the ball is within proximity to (or in the possession of) specific players, and in this scenario, in proximity to or in the possession of the keeper.

The combination of the keeper's ability to catch or parry the ball (e.g., using sensing technology described above) vs. the ball speed can then be mapped into a player skill metric (e.g., percentage saves of shots on goals over a predetermined kick speed, etc.). For example, for faster ball speeds, the keeper's ability to parry or catch the ball can be considered more skillful.

As another alternative, keeper reaction time can be determined, for example, by comparing the time of kick with the time of impact by the keeper's hands. The time difference between the two events can inform how much time the keeper had to react to the shot on goal.

Information relating to this metric can then be displayed or visualized on a web page or hand-held device (such as a mobile phone) and compared with other metrics gathered by the system in previous and future games. Moreover, information relating to this metric may be used to develop training programs to improve player quickness, agility, and/or reaction time (if necessary).

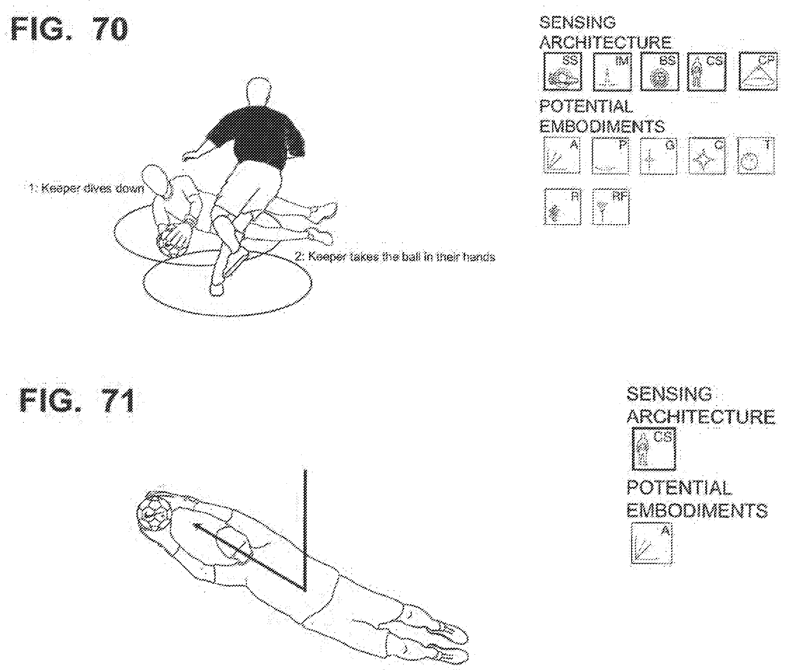

**Fig. 70** - **Keeper Advance (Tackle):**

This example aspect according to the invention uses a set of sensor systems on the keeper and in the ball to determine when the keeper performs a successful tackle, taking the ball away from the opposition. As some more specific examples, systems and methods according to this aspect of the invention may determine when an opponent has possession of the ball, followed by a contested time period between the keeper and the opposing player (e.g., both the keeper and the opposing player in close proximity to the ball), followed by a dive event performed by the keeper (e.g., determined by an on-body inertial sensing system carried by the keeper), followed by a picked up ball (e.g., which may be determined based on sensors in the keeper's gloves, accelerometer and/or gyro sensors in the ball, etc.). These events, happening in this sequence, are unique to a keeper tackle event. Tracking keeper tackle events provides an interesting and useful metric for evaluating keeper performance.

**Fig. 71 - Keeper Dive/Player Dive/Player Jump:**

An inertial sensing system, such as a three-axis accelerometer, when mounted on the body of a player during a soccer match or other activity (especially at the body core), will spend the majority of the time in a fairly flat plane of motion (e.g., a certain height off the ground determined by sensor mounting location). When the keeper (or other player) dives to the ground, the sensor will make a sharp deviation downward to the ground, followed by the player standing up and resuming motion within the original plane of motion. These two events can be used to determine when the player has made a diving action and/or when he/she is standing up. This same technology may be used, for example, to determine when a player has jumped a significant height in the air.

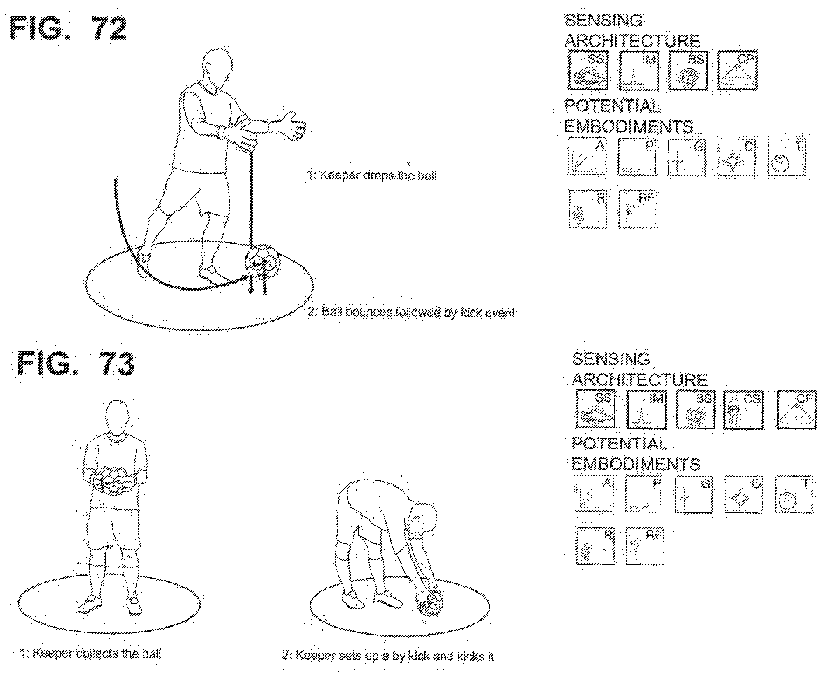

**Fig. 72 - Drop Kick:**

A "drop kick" event (a common event performed by a goal keeper in the game of soccer) also may be detected by systems and methods in accordance with at least some examples of this invention. Commercially available accelerometer technologies today can determine when the accelerometer (and hence the device with which it is engaged) is in a free-fall condition. Systems and methods according to this example of the invention use an accelerometer placed in a ball, in combination with an impact-sensing system in a shoe. These sensors can be used to determine the following event sequences, which correspond to and may be identified as drop kick events:

a. For a direct drop kick (in which the ball does not touch the ground first): the ball is picked-up, dropped (i.e., detected as being in free-fall), followed by a kick-impact (ball and shoe impacts at the same time).
b. For a bounced drop kick (in which the ball touches the ground briefly before being kicked): the ball is picked-up, dropped (i.e., detected as being in free-fall), makes a small impact due to contact with the ground, followed by a kick-impact (ball and shoe impacts at the same time) when the ball is traveling away from the ground. Alternatively, the ball may experience the kick-impact at the same time the ball contacts ground.

If desired, a maximum threshold time period may be initiated once the ball contacts the ground during which the kick event must be recorded in order for a successful bounced drop kick event to be counted.

**Fig. 73** - **Shot on Goal that Goes Out of Bounds:**

Systems and methods according to at least some examples of this invention may utilize a system of sensing elements in the ball (and optionally sensors in the boot) to determine when a ball goes out of bounds beyond the goal line (resulting in a goal kick), e.g., due to a wide kick or a high kick. The detectable events that enable determination of a "Shot on Goal that Goes Out of Bounds" are as follows:

a. Coincident impacts to the ball and boot are recorded to determine that a kick event has occurred.
b. The ball is then picked-up (which may be determined, for example, by detection of a very slow rotational pace and/or low frequency accelerations using inertial sensing/rotational sensing methods - the sensor output from a carry event will appear different from the sensor output from a kick event, e.g., in ball rotation, acceleration, etc.).
c. A set piece event then occurs (and optionally, a kick from the set-piece event may be detected).

This metric may be useful, for example, to determine offensive player skill and effectiveness, identifying missed opportunities during a game, defense effectiveness, etc.

As another example, if desired, the goal posts could include electronic modules thereon that allow proximity detection between the goal posts and the ball.

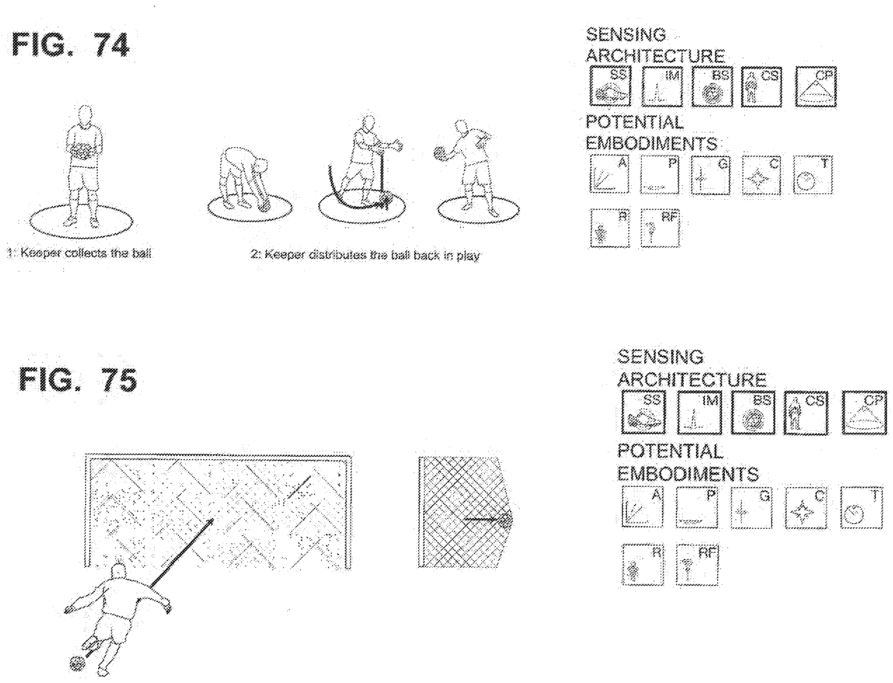

**Fig. 74** - **Shot on Goal:**

An important part of the game of soccer is the shot on goal. Systems and methods according to at least some examples of this invention include ball mounted sensors and/or player mounted sensors that will allow for detection of when a shot on goal has occurred. In one example system and method, output from an impact sensing system inside the ball (e.g., accelerometer, pressure sensor, etc.) is time matched to output from an impact sensing system inside the boot to enable determination of when the ball is struck by a specific foot. A proximity sensing system also may be employed (e.g., magnetic sensing, RSSI, etc.) to enable determination of when the ball is in proximity to specific players on the field. A core-mounted player rotational sensor also may be employed (e.g., compass sensor, gyro, etc.) to enable determination of which direction the player is facing as well as relative rotational information. Additionally, an inertial sensing system on the player can be used to provide additional signals and information. The events that occur to determine a shot on goal according to this example of the invention are as follows:

a. Possession by a member of the attacking team is determined, e.g., using techniques described above.
b. Signals from the pressure sensor or inertial sensor within the ball occur simultaneously to signals from the impact sensing technology within the boot.
c. Wireless communication between the boot and ball match the time exactly, recording the event as a kicked ball.
d. Proximity sensing systems record the ball entering the proximity radius of the defending team's goal keeper.
e. Inertial and rotational sensors within the ball record low-frequency signals that are characteristic of the ball being held by a person. Alternatively, inertial sensors on the player correlate closely to the path of travel recorded by the inertial sensors within the ball, suggesting the ball is being carried.
f. The ball is thrown, the ball is drop kicked, or a set-piece play is executed.

The "shot on goal" determination may be useful for a variety of metrics that may help determine the effectiveness of a goal keeper, the effectiveness of one or more offensive players, the effectiveness of one or more defensive players, team or line up effectiveness, etc.

**Fig. 75** - **Goal Scored:**

Systems and methods according to at least some examples of this invention also may be able to automatically determine when a goal has been scored. This may be accomplished, for example, by considering, at least in part, the behavior of the ball when it strikes the net and comes to a rest during a goal. As a more specific example, the following events may be used to determine that a goal has been scored:

a. Ball possession by a member of the attacking team is determined, e.g., using one or more of the techniques described above.
b. Signals from the pressure sensor or inertial sensor within the ball occur simultaneously to the signals from the impact sensing technology within the boot.

c. Wireless communication between the boot and ball match the time exactly, recording the event as a kicked ball.

d. Optionally, proximity sensing systems record the ball entering the proximity radius of the defending team's keeper.

e. An internal accelerometer in the ball recognizes that the ball has hit the net by producing signals indicative of a slow stop due to the ball being caught in the net (and optionally a gravity drop to the ground). This signal or series of signals will appear different from a more abrupt stop or direction change resulting from a catch or kick and/or the slow stop produced as the ball rolls to a stop.

f. Inertial and rotational sensors within the ball record low-frequency signals that are characteristic of the ball being held or carried. As an alternative, inertial sensors on a player correlate closely to the path of travel recorded by the inertial sensors within the ball, suggesting the ball is being carried.

g. The ball is carried back to the center circle and is place like a set piece for a restart to the game by the team that did not score. (Optionally, other events, like those described below, may be used as an indicator of play resumption).

The "goal scored" metric may be combined with other metrics, like possession information prior to the goal (e.g., to determine which player made the goal, assist information, etc.), goal keeper effectiveness, individual player effectiveness (both offense and defense), line-up effectiveness (both offense and defense), etc.

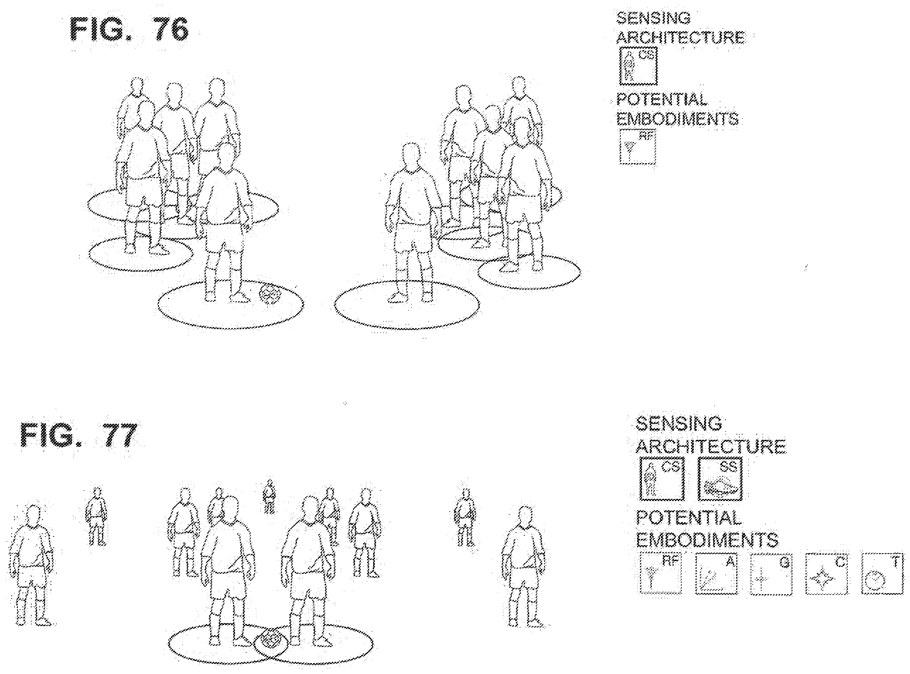

Figs. 76 through 83 illustrate various potential features and/or functionality of systems and methods according to some example aspects of this invention relating to the various teams, team metrics, game features, and the like. The features of these example systems, methods, metrics, and functionality will be described in more detail below.

**Fig. 76** - **Automatic Pick of Team Captains:**

On "pick up" soccer matches, there is often the need to choose a captain of each team who will then each choose their players one at a time. If desired, systems and methods according to at least some examples of this invention may be programmed and adapted to automatically pick captains from an assembled group of players, e.g., based on one or more metrics relating to the player of the assembled group of players.

As some more specific examples, systems and methods according to at least some examples of this invention may utilize the data and contextual information amassed by the assembled players over multiple games played. This example system involves nodes on each player that comprise the sensing systems described above, as well as a means of communicating wirelessly. One or more metrics for the assembled players can then be communicated to a common location (e.g., a cellular telephone, a palmtop computer, a laptop computer, a sideline computer, one of the player's body mounted devices, etc.) where the data can be collected and compared. Once the devices have communicated relative skill levels of the assembled players (e.g., by transmitting any of the various metric information as described above), the two best players (or any other metric such as the two worst players, the two best passers, the two best (or worst) goalkeepers, etc.) can be chosen to be the captains. If desired, systems and methods according to at least some examples of this invention may determine the best two overall players and the best two goal keepers and then divide these four players between the teams so that the best goal keeper is on the team of the second best player and so that the second best goal keeper is on the team of the best player. Any desired way of dividing up the players and/or choosing the captains may be used without departing from this invention.

As another alternative, rather than simply picking captains or goal keepers, systems and methods according to at least some examples of this invention can assemble, compile, and review the data to determine the fairest distribution of the assembled players among the teams using the metrics that have been amassed over multiple games played using the sensing systems and methods according to this invention.

As yet some additional options, if desired, systems and methods according to at least some examples of this invention that automatically choose the entire teams based on the assembled players may perform additional functions as well. For example, any way of advising the players of the team on which they should play on may be provided without departing from this invention. As some more specific examples, systems and methods according to the invention could send a team indicator message to the cell phone or other electronic device of each player (e.g., "You are on Team 1" or "You are on Team 2"). As another option, if desired, the computing system that automatically chooses the teams can wirelessly communicate with an electronic module provided on a garment or jersey, which can then change color, present textual information, or produce other features to show the team assignment decisions that were made.

**Fig. 77** - **Determination of Game Start:**

Systems and methods according to this invention may determine when a game actually starts (which can be the signal to start accepting data from the various sensors, e.g., mounted on the ball, players, goalposts, other equipment, etc.). Any desired way of ascertaining the start of the game may be used without departing from this invention. As one example, one player or other individual (such as a referee, a coach, etc.) may be tasked with manually providing an indication to a computing system as to when the game has started. As another example, the "game start" event can be determined by detection of a set piece event (as described above) within a short time frame after all players (or a majority of the players) in the game activate their on-body sensing systems using a peer-to-peer network, followed by a very short pass within team members.

Other ways of automatically determining the start of the game may be provided without departing from this invention. For example, in some example systems and methods according to the invention, all players on the field that are using the sensing systems and have on-body inertial sensing systems in accordance with this invention will be in communication with one another over a peer-to-peer network. The beginning of the game is one of the very few situations where the players are all standing reasonably still and two players on the same team are in close proximity to the ball. Detection of this type of activity or situation, followed by sudden and simultaneous movement by almost all of the players, may be used as an indication that the game has started.

As another example, in some systems and methods according to examples of this invention, all (or many) players may have an on-body sensing system that determines the orientation of the core of the body. Each sensing system may be connected via a wireless communication method that defines a peer-to-peer network. In such a system, all the modules can broadcast the direction on the field in which each person is facing. Combining this facing direction information (all team members facing the same direction, which is opposite to the direction that the opposing team faces) with detection of a set piece event, and optionally adding the proximity information described above where two players of the same team are standing within close proximity to the ball, can be used as an indication that the start of the game is about to occur (or has occurred once the initial kick is sensed).

As yet another alternative, the start of the game may be determined by substantially simultaneous movement by each player from a generally standing still position, due to the kickoff (optionally, correlated to a set piece event and/or an initial kick detection event, as described above).

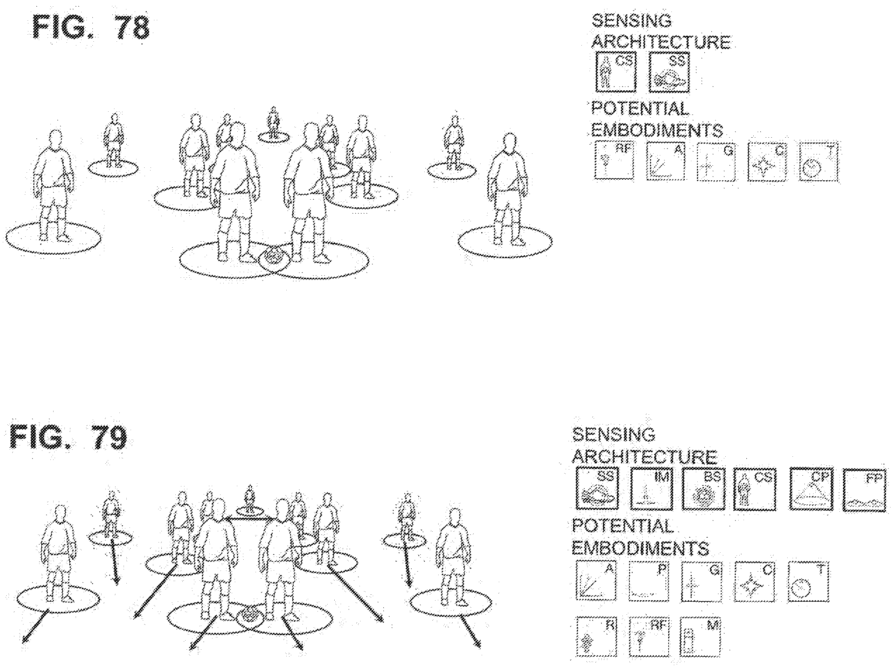

**Fig. 78** - **Direction of Play:**

For various metrics relating to the play of soccer (e.g., to determine the course of play, to determine whether a team tended to be attacking or defending, to determine various skill metrics, etc.), the direction of play for each player and/or each team may be a useful piece of information for sensing systems and methods according to at least some examples of this invention (e.g., so the systems and methods know which goal each team and player is defending and which goal each team and player will approach to score). Systems and methods according to at least some examples of this invention may determine the direction of play automatically, e.g., based on the movements of the various players over time. Determination of the direction of play according to at least some examples of this invention may utilize a body-mounted sensor with direction sensing capabilities (e.g., a compass sensor, accelerometer/gyro, etc.) to determine the direction that a player is facing at any given time. For such systems, direction of play may be determined by the following steps:

a. Multiple players on the pitch have sensing systems that include wireless communication means for sharing directional information.

b. Sensor signals are read on each individual player and are broadcast wirelessly to all sensor nodes (e.g., on each player).

c. The nodes are all integrated over the course of play to determine which players spend most of their time facing a particular direction.

d. Teammates will all share a similar bias toward facing the opposition goal.

This technology may be used to automatically determine which players are teammates. Additionally, as noted above, it may be useful for determining various metrics relating to the game, both on a team level and on an individual level. For example, offensive players that spend too much time facing their own goal may not be as effective as offensive players that spend less time facing their own goal. This data may also be used to determine which team seemed to play a more "attacking" game v. which team seemed to play be more defensive.

**Fig. 79 - Direction of Play Alternates:**

Fig 79 helps illustrate various alternative features for automatically determining direction of play (or information that may be used in automatically determining direction of play and/or automatically ascertaining teammates) that may be used in systems and methods according to at least some examples of this invention. For example, knowledge of the "start of game" metric, as described above, can be used to instantaneously look at the output of the core sensors to understand direction of play of individuals and/or teams and/or automatically determine the teammates. More specifically, in general, at the start of the game, members of each team will face the opponent’s goal. Therefore, the individual facing direction information at the beginning of the match for each individual may be stored, and this information can be used, at least in part, to determine the direction of play for each individual and/or the members of each team.

As another alternative, ball possession information (and the sensors that collect individual player possession information) may be used in combination with the direction facing sensors described above to enable determination of which direction the players are facing when on-ball, and the majority of dribbling performed by that player will be presumed to be driving toward the opposition goal.

As another alternative, pass sensing technology (e.g., as described above) can be used to determine a general pass direction bias, optionally combined with the length/direction of passes, to enable a determination which direction a particular team or individual is most often trying to move the ball. This directional information may be presumed to be oriented toward moving the ball toward the opposition goal.

Another potential alternative for automatically determining the individual and/or team direction of play (and optionally the identity of teammates) may take place during "set piece" plays. More specifically, during set piece plays, the majority of each team's player's will be facing toward the opposition goal. Directional sensors can combine with determination of a set piece condition (e.g., as reported by the ball via a wireless network, e.g., using technology described above), which can then be used to trigger a communication of all players' facing directions by the core-worn sensing systems.

As yet another potential option, during long dribbles, the body core worn sensor on the individual player will tend to report movement toward the opposition goal. This can be either an inertial sensor system (accelerometer, etc.) or a rotational sensor (gyro, compass, etc.), as both may be capable of reporting a movement/facing direction biased toward the opposition goal.

The various automatic direction and/or teammate recognition technology, as described above in conjunction with Figs. 78 and 79, may be used individually or in any desired combination to provide data relating to and useful in the final determination of an individual and/or player direction of play and/or recognition of teammates.

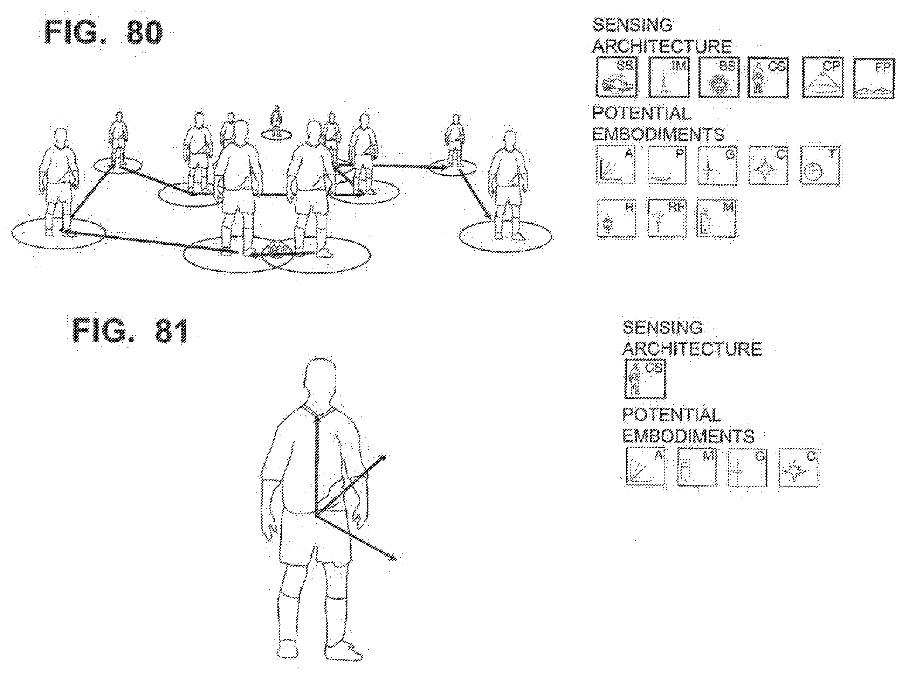

**Fig. 80 - Teammate Recognition Using Pass Distribution During a Game:**

This aspect of systems and methods according to at least some examples of this invention uses the pass distribution technology previously described (see Fig. 63). By aggregating the pass distribution data over time (e.g., via wireless communication between sensor modules), systems and methods according to at least some examples of this invention may determine the people that are most frequently passed to by a particular player, and thus the systems and methods may conclude that these frequent pass recipients are teammates of the passing player. During the course of a game there may be multiple pass interceptions, but presumably, the dominant number of passes that occur will be to a player's teammates. Over time, a pattern will emerge that will allow the system to dynamically figure out who is on the same team, and who is not. Player-to-player and player-to-ball proximity information also may be used in this aspect of the invention, e.g., this data may better allow a determination of whether the passing player tends to try to pass to an individual or whether the passing player tends to send passes so as to avoid an individual.

Alternative technology may be provided that allows players to manually enter the team on which they play (e.g., by input to their body worn sensors, by selection from a menu, etc.).

**Fig. 81 - Determination of Team Based on Object Orientation:**

Various examples of ways of determining which players are on which team (or at least data relating to this determination) are described above. Additionally or alternatively, systems and methods according to at least some examples of this invention may use the orientation of the receiver system (or any component of the system) to determine or as an indicator of which team a particular player is on. Because the game of soccer always involves only two teams, this determination or indicator system may be binary.

Various binary indicators may be provided without departing from this invention. As one example, using an accelerometer or other inertial sensing system, the gravity vector may be used to determine the orientation of the object. As another example, a pocket or clip that is intended to house at least some part of the sensing system may have a magnet embedded in it, and this magnet may be sensed by a Hall-effect sensor, reed switch, or similar to determine object orientation. As some example, the location of the magnet could be in a plastic housing, or even embedded into an apparel pocket. As another alternative, a passive element, such as a ball bearing or similar object, may be pulled downward by gravity, making an electrical contact with two electrodes inside the object. The side of the housing or other object toward which the ball bearing is pulled by gravity can be used as an indicator of the orientation (and therefore team) of the object. The players could wear the various sensors or the housings therefor in one orientation on one team and in the opposite orientation on the other team.

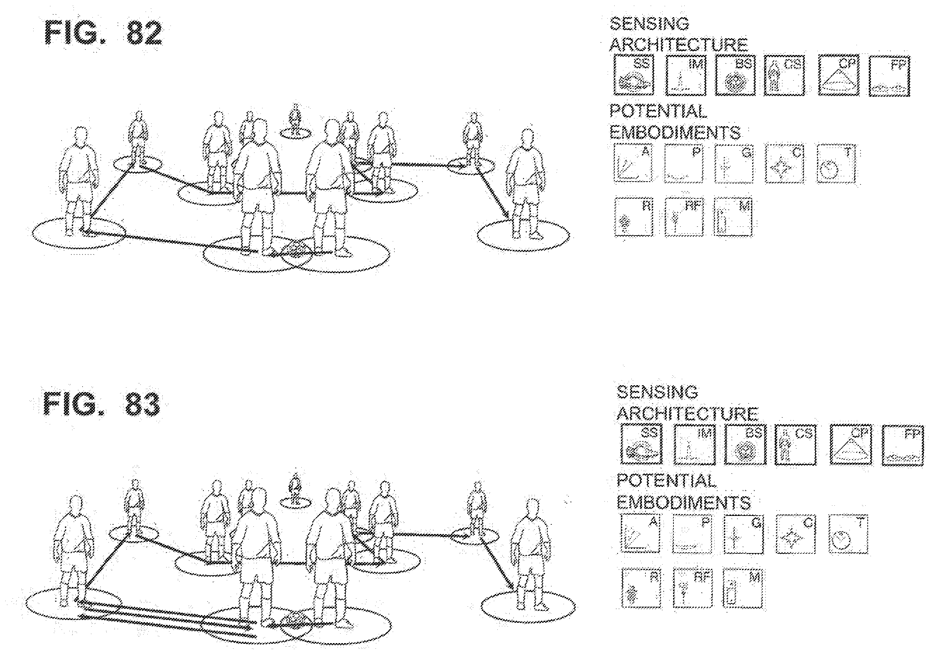

**Fig. 82 - Determination of Team Using Ball Proximity/Passing:**

Aspects of this invention, as described above, may include proximity sensing systems in the ball, as well as inertial/impact sensing systems in both the ball and the boot. As another feature, systems and methods according to at least some examples of this invention may use the ability of the ball to know when it is in tight proximity or know when a simultaneous impact event occurs between the boot and the ball, which may be communicated wirelessly, signaling the ball's presence at the feet of a particular player. This example feature according to the invention uses a simple algorithm that allows the system to learn the teams. For example, prior to the start of the game (or at some other desired time), the ball may be simply passed around to each member of a team, signaling their status as teammates. This example system and method can then use the "passed around" players as one team, and any other players the ball comes in proximity to can be assumed to be on the opposing team.

Alternatively, if desired, a controlled behavior (such as squeezing the ball, picking the ball up, throwing the ball, etc.) can be used to signal the "transition" from passing around between the players on team #1 to passing the ball around between the players of team #2, and in this manner the ball can positively identify the various members of each team, e.g., before the game begins.

**Fig. 83 - Use of Pass Frequency to Determine Teammates:**

This aspect of the invention uses the technology described above to determine when a successful pass has been made, but it but removes the knowledge of knowing teammates at the start of the game. If desired, systems and methods according to examples of this invention can automatically determine the teammates using pass frequency information. In this example system and method according to the invention, the system wirelessly communicates whenever the ball has traveled from one player to another, regardless of team. Throughout the game, patterns will emerge between certain players, and the pass frequency between players on the same team should be much higher. Statistical predictors can then figure out which 11 players (or other number of players) are most likely on the same team by evaluating the frequency of passing among them (many passes between two individuals most likely identifies them as teammates - two players cannot be that bad to always kick intercepted passes to the same person). Player-to-player and player-to-ball proximity information also may be useful in this determination.

Pass frequency features can be used in other manners in systems and methods according to examples of this invention, if desired. For example, an individual player's "preferences," such as which players are passed to more often by a particular player, can be identified and visualized online for improvement suggestions. As a more specific example, if a mid-fielder is always passing to the left side, he/she may become more predictable to the opponent. A coach noting or informed of this preference or tendency can develop drills for this player to help improve his/her skills and confidence in passing to the right side of the field.

Additionally or alternatively, if the speed of the player during the passing is added to the above pass frequency information, the system and method can be improved. Pass accuracy may change based on player speed. So added weight in the algorithm can be placed on passes that occur when players are moving at relatively low speeds (as compared to higher speeds). This may be most evident, for example, when the players pass the ball around the backfield, trying to create space within the field to open up a player for a pass near the opponent's goal.

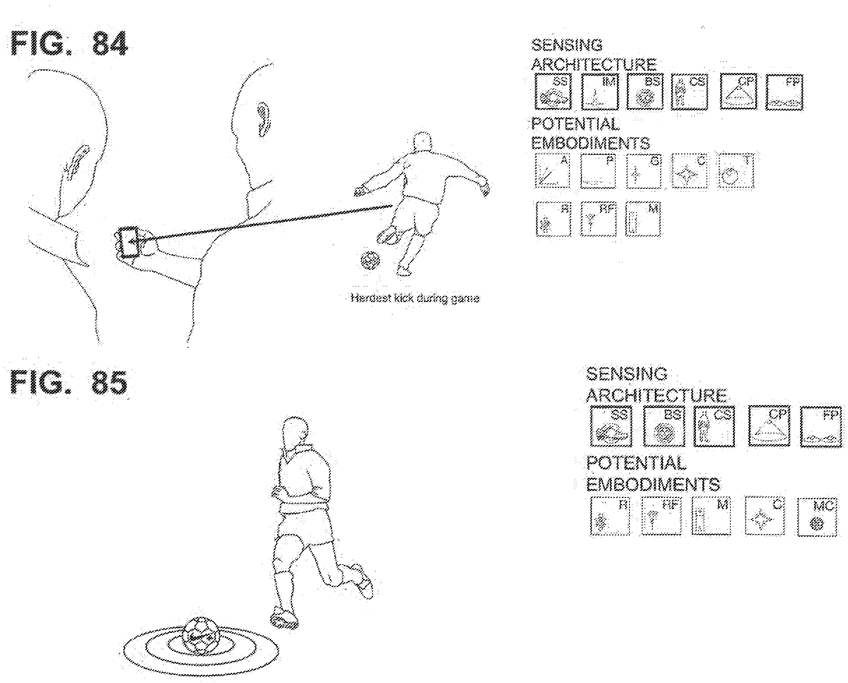

**Fig. 84** - **Post Game Concepts**

Various post game features may be made available by systems and methods according to examples of this invention, e.g., such as displaying data and various metrics regarding player performance as described above. If desired, systems and methods according to at least some examples of this invention may allow players to gather and play some "quick games" using display devices immediately after the game. For example, the various players could gather after the game (e.g., on one team, both teams, portions of either team, etc.) and the data collected for these players may be combined (e.g., via wireless communication technology, peer-to-peer connections, etc.) to enable the players to compare and contrast their performances over the course of a game, workout, or practice session. Examples of the data that may be determined and displayed after the game in a quick gathering of players (e.g., on one or more player's cell phones, handheld computing devices, etc.) include, but is not limited to identification of: Who had the longest successful pass? Who reached the fastest speed on/off ball? Who was the best passer (e.g., most passes, fewest interceptions, highest successful pass percentage, etc.)? Who was the workhorse (e.g., who ran farthest, who had most possession time, etc.)? Who had the fastest kick? Who had the most tackles? These metrics, quick games, and competitions can be displayed on an LCD or similar display immediately following the game (or at any other desired time), e.g., giving the system a richer experience with immediate feedback after the game has been played (or even during the game). The data displayed may include only data among the players gathered at the end of the game for this type of session, or it may include data collected from all of the players by systems and methods according to this invention.

Figs. 85 through 93 illustrate various potential features and/or functionality of systems and methods according to some example aspects of this invention relating to the various miscellaneous metrics, game features, and the like, including various uses of magnetics and magnetic properties. The features of these example systems, methods, metrics, and functionality will be described in more detail below.

**Fig. 85** - **Electromagnetic Coils in Ball:**

This example aspect of the invention uses coils with pulsed current loads placed inside the ball to create a magnetic field that can be detected by sensors outside the ball. Adding a pulsed magnetic field can allow sensing mechanisms in accordance with some examples of systems and methods of the invention to filter for a very distinct signature, giving greater range/proximity detection (e.g., it allows body mounted detector systems to look for specific signal patterns representative of the ball and/or provides better ability to filter out "noise"). As another potential option, if desired, coils with different pulsation rates can be placed throughout the ball to allow sensors (e.g., body mounted sensors, shoe mounted sensors, etc.) to detect specific places on the ball, as well as the direction of rotation, based on the sequence of the magnetic pulse rates detected. This data may be useful, for example, to determine features of kick length, pass length, and/or other performance metrics.

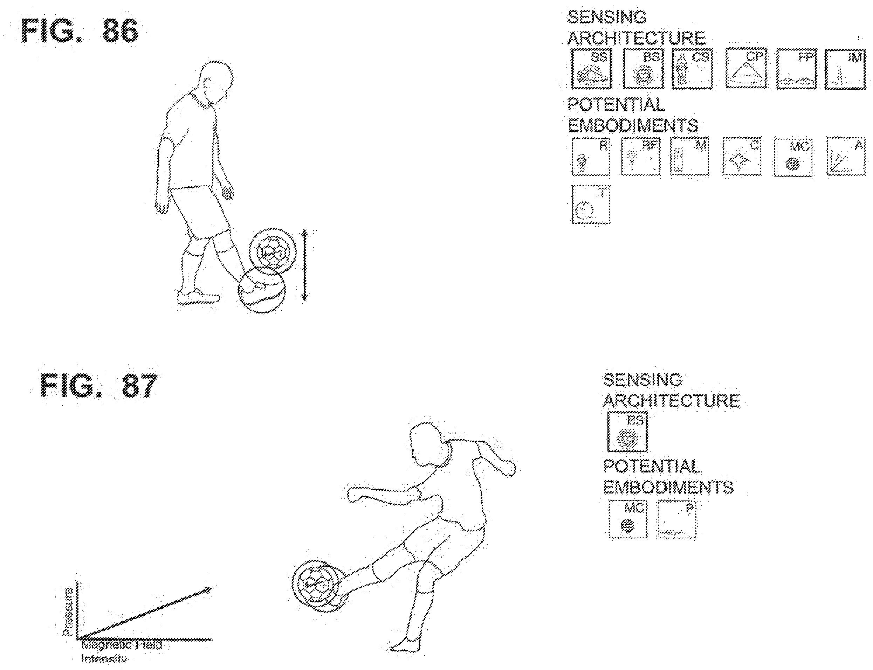

**Fig. 86** - **Juggling:**

This example aspect of the invention uses the previously described integration of magnetic coils and sensors in the ball combined with sensing elements in the boot to detect very close proximity to the ball. Additionally, inertial or pressure sensors may be provided within the ball to detect an impact. When an impact is detected by the ball, the magnetic sensors also can be polled to understand if there was a simultaneous impact or close proximity to the boot, and such a system can wirelessly communicate (or store) the number of times in a row the ball was "juggled" by a player.

Alternatively, impact sensing elements in the shoe (e.g., accelerometer, piezo element, etc.) may be combined with inertial or pressure sensing elements in the ball. The simultaneous impact to the ball and shoe denote a kick, and wireless communication between the two systems may be used to determine how many times in a sequence the ball was kept in the air without impacting another surface, giving the player the number of times the ball was juggled. Additionally or alternatively, if desired, time between impacts, impacts with player's knees, and/or other features may be factored in and considered in determining whether a juggling event has continued.

**Fig. 87** - **Ball Creates Magnetic Field Proportional to Pressure:**

Systems and methods according to this example of the invention include an electrical, electro-mechanical, or mechanical system inside a soccer ball that creates a magnetic field that is proportional to the pressure inside the ball. The magnetic field generated then can be sensed by external sensors, such as sensors on the boot and/or body core mounted sensors. Examples of implementation and use of this example aspect of the invention include, but are not limited to, ball proximity detection (when kicked), detection of internal pressure using external sensing, kick speed, kick force, kick distance, etc.

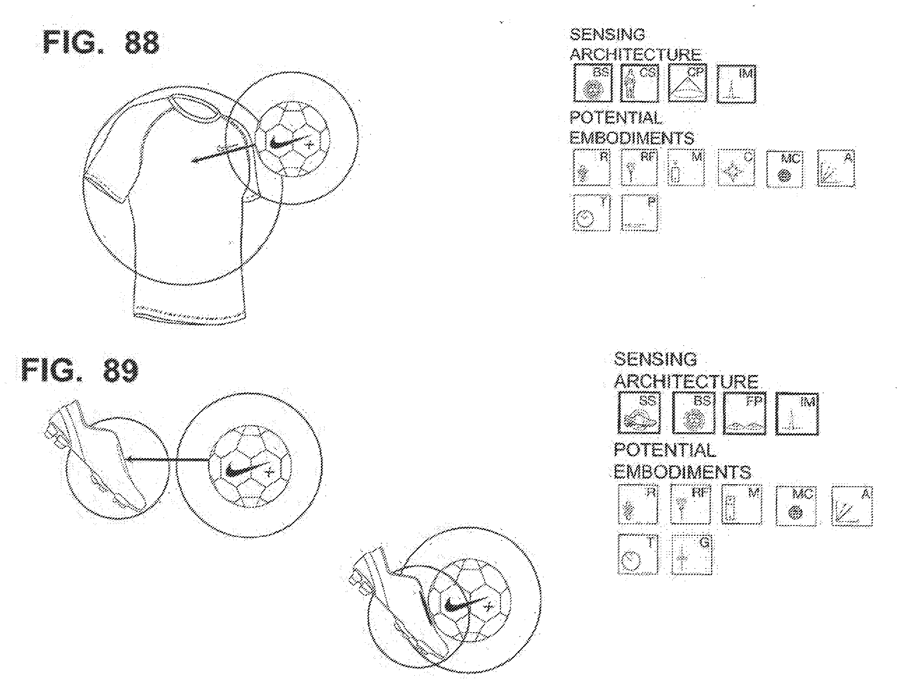

**Fig. 88** - **Integration of Magnets into Apparel for Ball Detection:**

Another potential feature of systems and methods according to at least some examples of this invention relates to the use of magnets (either permanent or electro-magnets) and their integration into apparel for a soccer player. The magnets are placed in locations which allow a magnetic sensor within the ball to detect their field, and as such detect what part of the body had just interacted with a ball. As a more specific example, the chest is used in the game of soccer to trap or stop a highly-lofted ball. Upon close proximity to the garment, the ball may detect the magnet in the clothing and knows which part of the body is closest (e.g., the magnet could be provided in shirt to demonstrate and detect chest/shoulder control, in the shorts to demonstrate thigh or knee control, in a headband or hat to demonstrate head control, etc.). Alternatively, if desired, the magnet could be included in the ball and the sensor mounted on various articles of clothing and the data could be transmitted or stored in the article of clothing.

As another alternative, if desired, inertial and/or pressure sensing systems provided inside the ball may activate/trigger the magnetic detection sensors when an impact is recorded, allowing the power system to save battery power and gain efficiency.

**Fig. 89** - **Shoe Power Plate:**

This aspect of the invention uses a fluidic material that hardens when exposed to a magnetic field. Fluid pockets are created within the shoe and/or protective gear (such as a shin guard, etc.), and the fluid included in the pockets remains viscous and soft until a magnetic coil residing underneath or on top of the pocket energizes. This action makes the material very hard, which can protect the foot, provide a harder kicking surface (to produce greater shot power), etc. Magnetic "smart" fluids, also called "magnetorheological fluids" are known and used in the vehicle suspension arts and as "liquid body armor" (e.g., for bulletproof vests).

Alternatively, if desired, the fluidic pockets need not have a magnetic coil underneath them, but rather the ball may be adapted to contain magnets that, when in close enough proximity to the fluid, change the state of the fluid, making the boot hard. As another alternative, if desired, a combination of the sensing systems, e.g., as described above, can offer contextual information to a processing system provided in the shoe, which in turn can activate magnetic field generators (e.g., also in the shoe), which can actively change the hardness and flexibility of the shoe based on real-time information about the game. Alternatively, the shoe can use skill-based metrics gained from previous contests to understand what kind of player the athlete is, and how a shoe may better serve the specific needs of the player.

**Fig. 90** - **Shin Protection Plate:**

Aspects of the "shoe power plate" technology described above in conjunction with Fig. 89 may be used in other environments as well. For example, this same type of magnetic "smart" fluid or magnetorheological fluid may be provided in a pocket of a sock or other clothing to function as protective gear (such as a shin guard, etc.). If desired, an opponent's shoes may be equipped with a magnet or magnetic force generating system which would trigger/activate the magnetorheological fluid when the shoe closely approached the protective gear. In this manner, the sock or other item may conform well to the wearer's body (so that it is comfortable and stays in place) during normal use and only becomes hardened when a magnet equipped boot (or the ball) approaches.

Fig. 91 - Magnetic Coil to Sense Shoe Properties During Running:

This aspect of the invention involves placing a coil of wire inside a shoe, as well as a permanent magnet that passes through the coil, generating a current flow through the coil. This current flow then may be used to sense the "contact time" of when the shoe is on the ground. More specifically, when running, the shoe will flex, which through a mechanical mechanism moves a magnet within the coils generating the field. When a runner is running, the shoe will flex until a "toe off' event, and then while in the air the shoe will return to steady state (e.g., a flat sole). Then, after a "heel strike" event occurs, the shoe will begin to flex again, moving the magnet within the coil. These two signals, from the heel strike and the toe off events, can be used to determine when the shoe is on the ground and when it is in the air. This information can be used, e.g., with conventional pedometer type speed and distance determination algorithms, as data useful in determining player speed metrics, which can be integrated to get a player distance moved metric.

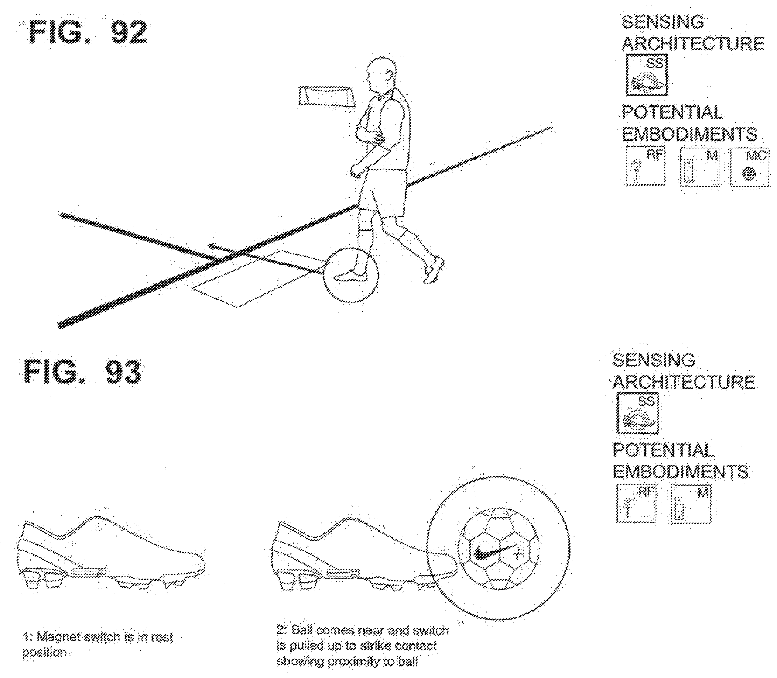

**Fig. 92 - Magnetic Sensors Coming on Pitch Turns on Body Sensor:**

This example aspect of the invention uses a magnetic sensor in the boot or on the player's body (e.g., sensors already provided for player-to-ball or player-to-player proximity detection or for any of the previously described purposes) to act as a switch to prepare the system for the start of the game. For example, magnetic mats (or cones or other structures) may be provided at the side of the pitch, and as the players approach and enter the field, they will pass over/through this the system. This action may be used to turn on the system and get it into a "ready" state for the start of the game. The system can then be started when a game start event is detected (e.g., as described above), or when a player manually activates the system at the start of the game. The magnetic field also could be directionally varied (e.g., change in strength over the course of its length) so that systems and methods according to this aspect of the invention can ascertain whether the player is entering or exiting the field.

**Fig. 93** - **Magnet in Ball Pulls Up Magnetic Sensor Switch in Shoe:**

This aspect of the invention may be used, for example, as an alternate system in determining player-to-ball proximity and/or player possession as described above. Systems and methods according to this example of the invention use a magnetic switch in shoe that moves to signal proximity when the magnets in the ball come close. As an example, as illustrated in Fig. 93, a reed type switch may be provided in the shoe that makes contact with an electrical contact provided in the shoe when a magnetic source provided in the ball induces the reed portion of the switch to move upward or downward. When the magnet in the ball is out of range of the switch, the reed returns to its neutral, un-contacting position. Thus, data collected resulting from contacts between the reed switch and the contact in the shoe can be used to determine and count interactions between the ball and shoe (and thereby provide information regarding proximity to the shoe and/or ball contact with the shoe (e.g., possession, passes, juggling, etc.)).

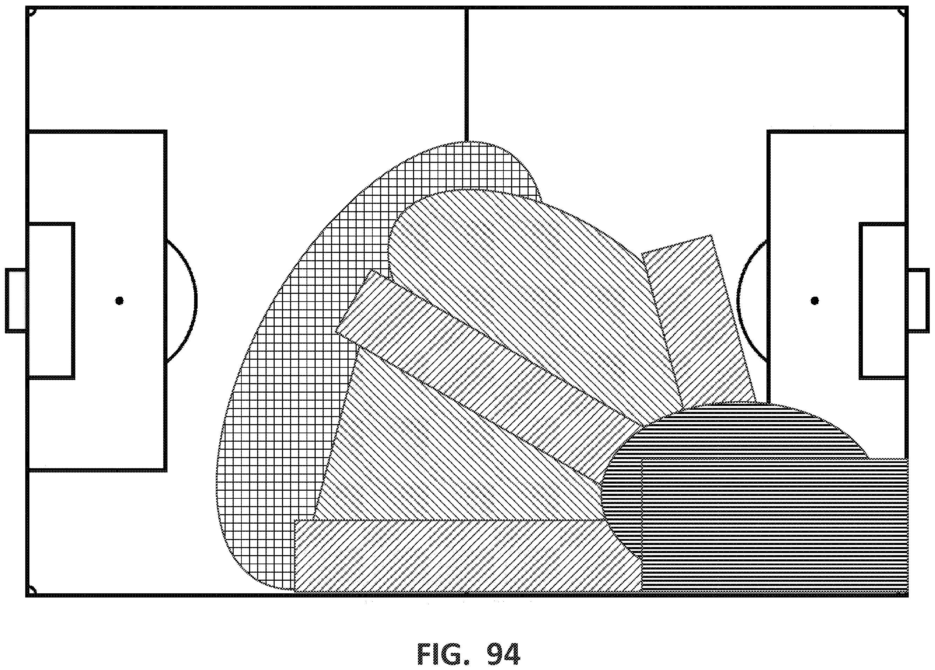

**Fig. 94: Field Location "Heat Map":**

If desired, systems and methods according to at least some examples of this invention may produce a field location "heat map" that indicates where on the field the player spent time and, optionally, an indicator of the amount of time spent on that portion of the field. Fig. 94 illustrates an example field "heat map" that may be generated using systems and methods according to at least some examples of this invention. As shown in Fig. 94, the representation of the soccer field (which may be provided on any desired type of display device, e.g., as described above) may include various zones or regions that indicate where the player spent his or her time during the course of the game. The colors of the various zones may provide an indicator of the amount of time spent within that zone. This type of information may be useful, for example, by a coach and the player, to determine how well the player stays in position and/or when/if the player spends time outside of the desired or optimal positions. This information also may be useful as an aid for determining whether a player or team is in more of an attacking or defending posture. If desired, the "heat map" may be capable of displaying player positioning during an entire game or practice session, during any desired portion of a game or practice session, and/or even comparing player performance from one game to the next (e.g., by overlaying one heat map on another).

Any desired type of player location determining systems and methods may be used without departing from this invention, such as GPS. As another alternative, the initial player location of the field may be entered, e.g., by the player starting at a fixed location for his/her position, and then systems and methods according to aspects of this invention may track the player's location from this initial starting location, e.g., using one or more of: an accelerometer, a gyroscope, a compass, etc. As yet another alternative, player location may be determined automatically over the course of a game, e.g., by noting the player's tendency to avoid going over end lines and side lines, the player's general positions and movement on the field may be determined based on approximate determined locations for the end lines and/or the side lines. As yet another example, the general heat map may be generated without reference to a location on the field, and after the fact the user could anchor the heat map location with respect to a representation on the field, e.g., based on knowing an approximate location where they started or ended the game, based on their position, etc.

Also, if desired, the heat map may include information regarding ball possession. As a more specific example, if desired, a special heat map may be developed and presented to identify locations on the field where the player had possession of the ball. This heat map may include different colors to indicate the number ball possessions at the indicated location, the time of possession at the indicated locations, etc.

**Other Information:**

As noted above, systems and methods according to at least some examples of this invention will be capable of determining when a ball is sent out of bounds. Data to assist in evaluating and determining this feature may include, for example, data indicating that the ball has decelerated, data indicating that the ball is not rotating (e.g., being carried), or data indicating that the ball is moving slowly (e.g., being carried), etc. Optionally, this deceleration, non-rotation, and/or slow motion activity may be required to last for a predetermined time period (e.g., at least 2 seconds, at least 3 seconds, etc.). Once it is determined that the ball is out of bounds, systems and methods according to examples of this invention may work backwards to subtract accumulated possession time (e.g., individual or team) from the time stamp of the previously ascertained and recorded kick (i.e., the last "in bounds" kick).

Also, as noted above, systems and methods according to at least some examples of this invention may know or be capable of determining when two or more players are located within close proximity to the ball. During this time, neither player may be considered as being in clear "possession" of the ball. This time also may be categorized by systems and methods according to examples of this invention as "contested time." A determination of "contested time" may trigger a stop in accumulation of team and/or individual possession time (optionally, depending on whether the opposing player contacts the ball during the contested time or whether the initial party determined to be in possession of the ball maintains the ball free from contact of or proximity to the other player during the contested time). A new "possession time" may begin (for either team or any present individual) after the "contested time" period ends. Optionally, if desired, an individual's and team's possession time could continue during a contested time period, e.g., at least until the opposing player contacts the ball, or until the opposing team clearly gains possession of the ball. Contested time also could accrue when two players reach a loose ball at or near the same time (i.e., when no one had clear prior possession, such as when the ball moves from one contested time situation to another).

While many example systems, methods, features, metrics, and aspects of this invention have been described in conjunction with the game of soccer, aspects of this invention also may be extended for use in a variety of other sports, such as football, basketball, lacrosse, tennis, baseball, rugby, hockey, field hockey, cricket, and golf.

**III. Exemplary Embodiments of Passive Tag for Use with Frequency Doubling Possession Detection System**

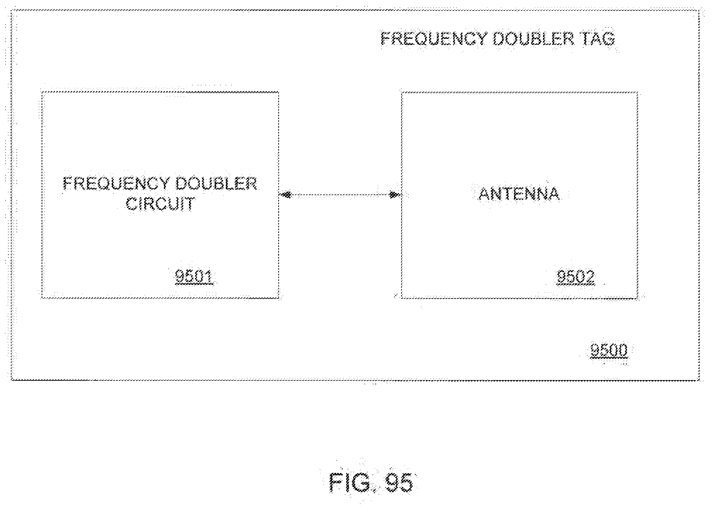

Figure 95 shows an exemplary embodiment of passive frequency doubler tag 9500 that may be embedded on a puck or ball, *e.g.,* soccer ball 1200 as shown in Figure 12. With some embodiments, tag 9500 operates at a basic (fundamental) frequency (*e.g.*, 2.4 GHz) and a second harmonic frequency that is double the first frequency (*e.g.*, 4.8 GHz). While tag 9500 may have a reduced range compared with an active tag because of doubler frequency conversion efficiency, tag 9500 typically does not require an external power source for frequency doubler circuit 9501.

While tag 9500, as depicted in Figure 95, doubles the transmitted frequency, a tag on the ball may transform the frequency of the transmitted signal in another predetermined fashion. For example, the tag may triple the transmitted frequency or may add a predetermined offset to the transmitted frequency.

The frequency doubler tag 9500 comprises an antenna 9502 and frequency doubler circuit 9501. Performance monitoring system (*e.g.*, system 100 as shown in Figure 1) may include a transceiver embedded into a soccer shoe (*e.g.*, shoe 104 or on the player's body) and frequency doubler tag 9500 that may be embedded in a soccer ball. Frequency doubler tag 9500 in the ball receives a signal from the shoe at a specific frequency and bandwidth and reradiates the signal's second harmonic. When detected by the on-body transceiver, the propagation delay of the received second harmonic signal may be converted into another signal with a frequency that is proportional to the distance between the shoe and the ball. The performance of the system is typically affected by the gain of antenna or antennas in the ball and the shoe. To achieve the maximum range at any relative shoe/ball orientation, antenna 9502 and the antenna in the shoe should have uniform omni-directional patterns.

Frequency doubler circuit 9501 follows a non-linear square law relationship between the amplitude of the signal and the generated second harmonic. With this relationship, as the signal decreases by 10 dB, the resulting second harmonic signal decreases by 20 dB. Consequently, antenna efficiency is often important. The antenna pattern should be uniform to avoid orientations with lower range. Low 'Q' broadband width element types should be selected to avoid gain reduction due to detuning in the typical soccer ball environment.

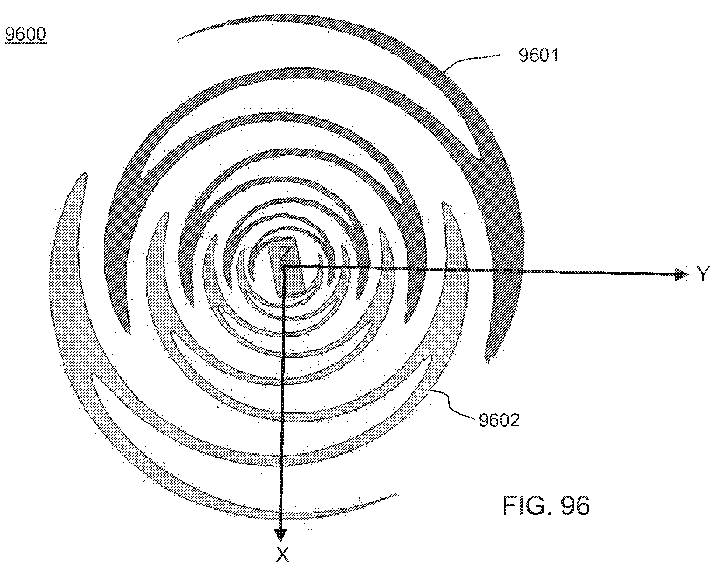

Fig 96 shows a two-element sinuous antenna 9600 that may be incorporated with frequency doubler tag 9500 in accordance with embodiments of the invention. With some embodiments sinuous antenna 9600 has a broad frequency range and planer structure needed to fit in the space constrained area on ball 1200. As shown in Fig. 96 antenna 9600 comprises elements 9601 and 9602 through feed 9603, although other embodiments may utilize a different number of elements, e.g., four elements. A matching circuit (not explicitly shown) may be inserted between sinuous antenna 9600 and frequency doubler tag 9500, where sinuous antenna 9600 typically has a balanced input. However, with some embodiments, frequency doubler tag 9500 may directly feed into sinuous antenna 9600 by adjusting antenna 9600 to have an impedance sufficiently matched to that of tag 9500.

With some embodiments, sinuous antenna 9600 has a diameter of approximately 3 inches; however, antenna may be redesigned to fit into the 2 inch diameter area. With some embodiments additional element types were designed and simulated to provide alternatives to the sinuous elements. One characteristic of the antenna that may significantly affect system performance is the interaction between the antenna impedance of antenna 9600 and frequency doubler circuit 9501. Alternate antennas may be designed with different terminating impedances at both the fundamental frequency (approximately 2.4 GHz) and the second harmonic frequency (approximately 4.8 GHz). As will be discussed different antenna element types (*e.g.*, crossed magnetic dipole 9900 and turnstile dipole 10200 may be selected to implement an alternative antenna for antenna 9600.

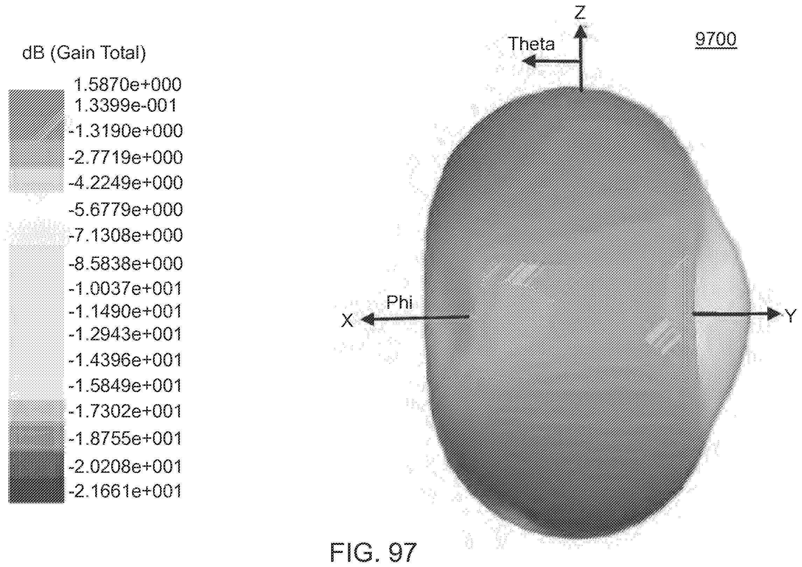

Fig. 97 shows an antenna plot for a two-element sinuous antenna at approximately 2.45 GHz in accordance with embodiments of the invention. Frequency doubler circuit 9501 receives a received signal from transmission element 110 (as shown in Figure 1) through antenna 9600 at the fundamental frequency.

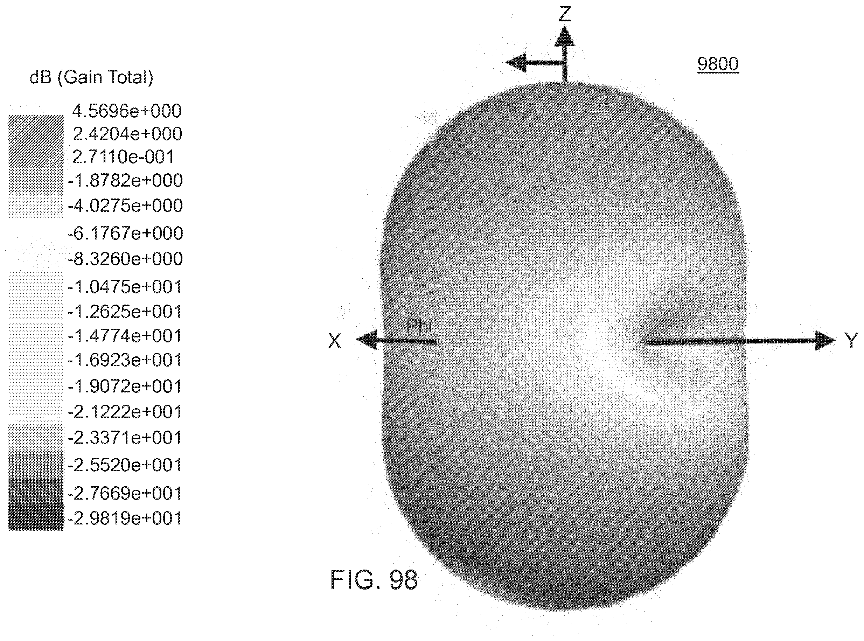

Fig. 98 shows an antenna plot for a two-element sinuous antenna at approximately 4.8 GHz in accordance with embodiments of the invention. Frequency doubler circuit 9501 transmits a transmitted signal to receiver 108 though antenna 9600 at the second harmonic frequency.

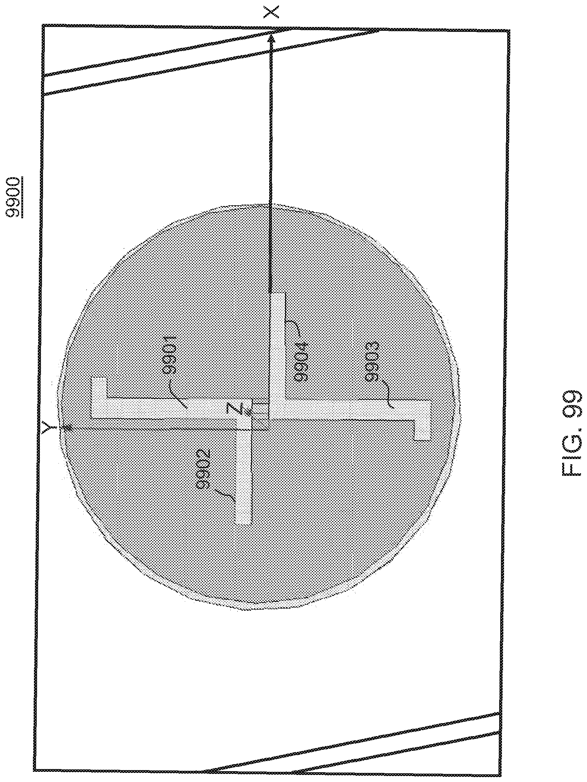

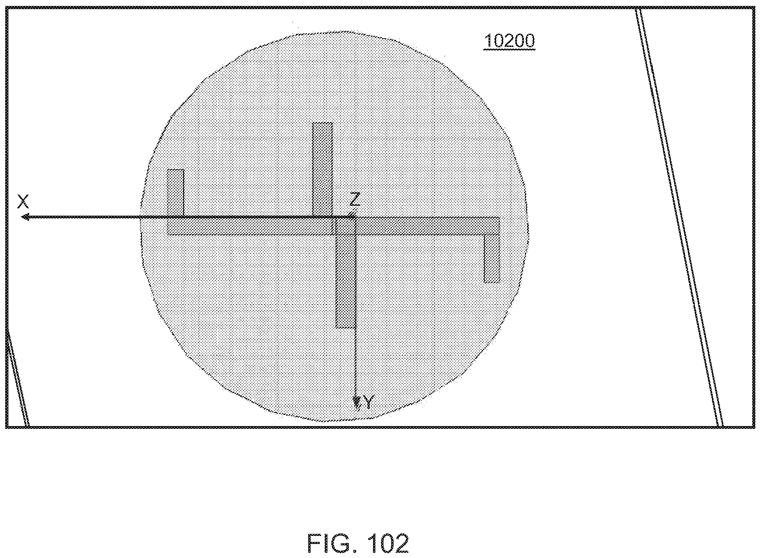

Fig. 99 shows crossed magnetic slotted dipole 9900 that may be incorporated with a frequency doubler tag 9500 in accordance with embodiments of the invention. Crossed magnetic slotted dipole 9990 incorporates slots 9901-9904 to appear as an inverted image of a dipole antenna. With an embodiment, dipole 9900 has a low profile with a thickness of approximately 0.002 inches, a high characteristic impedance, a broad bandwidth, and omni-directional characteristics. Crossed magnetic dipole 9900 typically presents higher impedances at both the fundamental and the second harmonic frequencies while turnstile dipole (as shown as 10200 in Figure 102) typically presents low impedance at both the fundamental and the second harmonic frequencies. Magnetic dipole element 9900 is typically less sensitive to impedance variation due to the presence of near field surfaces and objects when compared to turnstile element 10200. Sinuous element 9600 typically presents higher impedances at both the fundamental and the second harmonic frequencies. However, because of the compression to the 2-inch diameter from the 3, inch diameter sinuous antenna 9600 typically presents a low highly reactive impedance at the fundamental frequency and a high impedance at the second harmonic frequency. The impedance at the fundamental frequency may be compensated by adding matching circuit components.

The relative impedance of sinuous antenna 9600 may be less affected by near field surfaces and objects due to its broadband low Q characteristics. Sinuous antenna 9600 typically provides the most omni-directional pattern of total gain with respect to antennas 9900 and 10200.

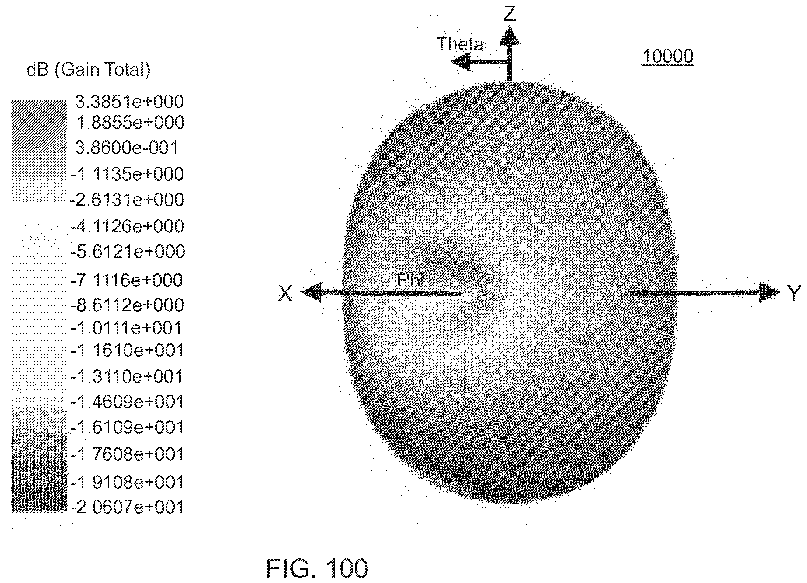

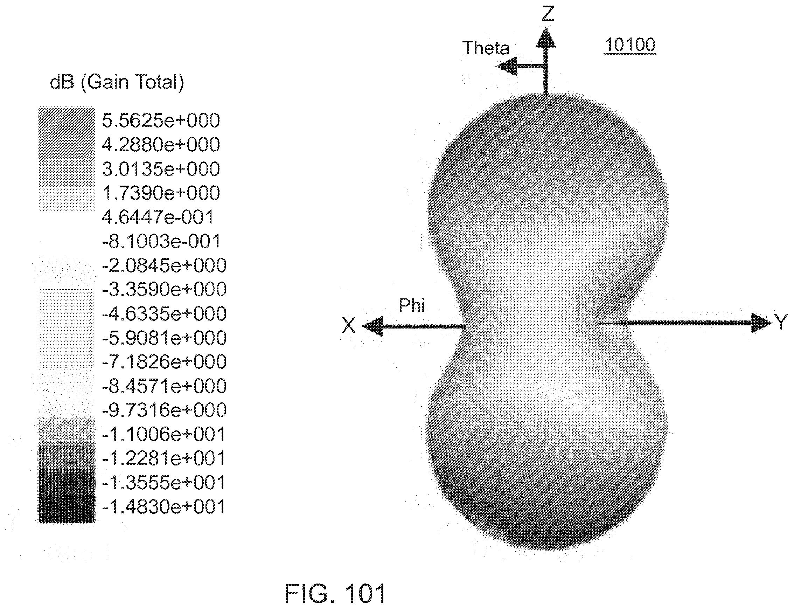

Fig. 100 shows an antenna plot for crossed magnetic slotted dipole 9900 at 2.45 GHz , while Fig. 101 shows an antenna plot for crossed magnetic slotted dipole 9900 at 4.9 GHz in accordance with embodiments of the invention.

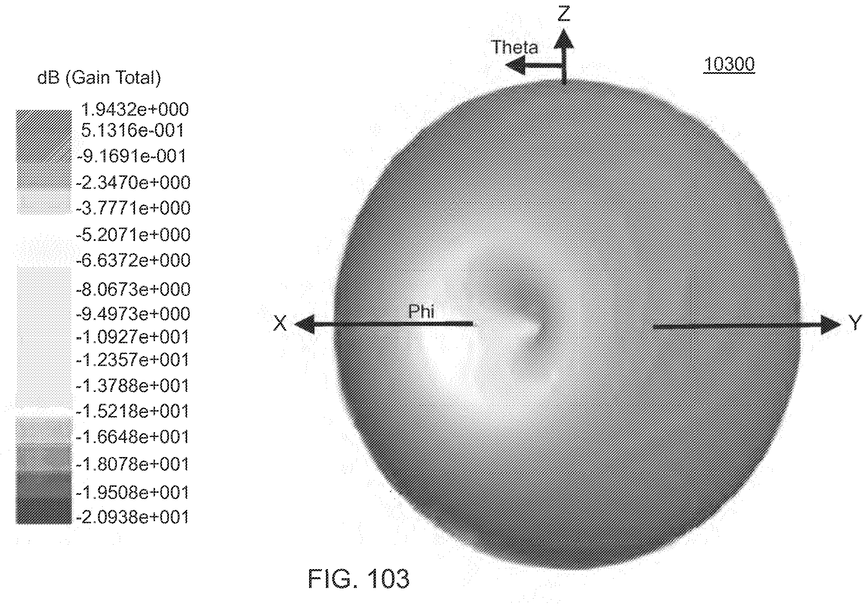

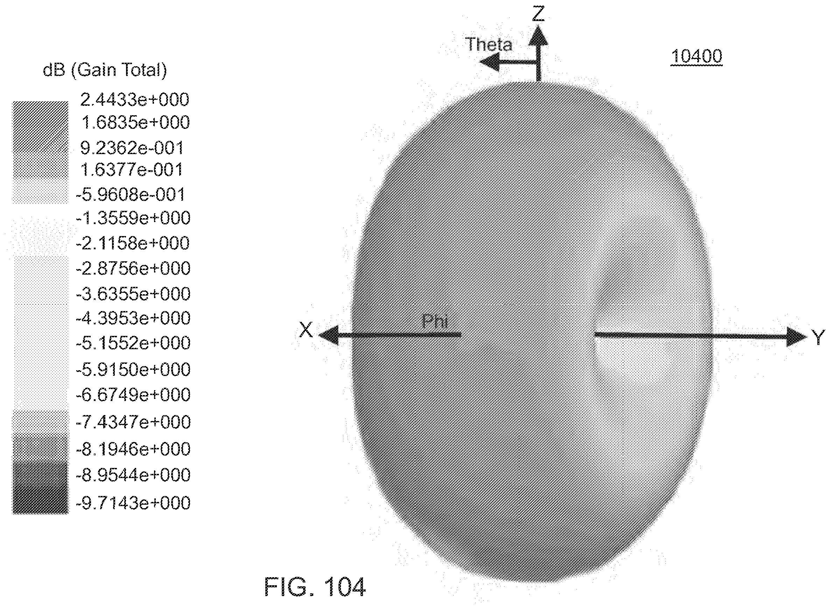

Fig. 102 shows turnstile dipole 10200 that may be incorporated with frequency doubler tag 9500 as was previously discussed. Fig. 103 shows an antenna plot at 2.45 GHz, and Fig. 104 shows an antenna plot for turnstile dipole 10200 at 4.9 GHz. With some embodiments, turnstile dipole 10200 has a thickness of approximately 0.002 inches, a width of 4.25 inches, and a length of 2.62 inches.

Frequency doubler circuit 9601 may be designed using Schottky diodes having a low barrier voltage. Several approaches may be considered that have different conversion efficiencies, circuit complexities, sizes and termination impedances. For example, a matching circuit may be inserted between doubler circuit 9501 and antenna 9502 to better match impedances between circuit 9601 and antenna 9602 at the fundamental frequency and the second harmonic frequency. The performance impact to conversion efficiency due to termination impedance and the interaction between antenna 9502 and frequency doubler circuit 9601 layouts may be determined by concurrent simulation. The selection the frequency doubler design may be based on reliability testing rather than optimization for conversion efficiency.

The mechanical construction of frequency doubler tag 9500 into the ball may be focused on reliability. The reliability of tag 9500 may be an area of concern during both installation into a ball and use. The selection of materials and construction typically impacts reliability. Due to the complexity of embedding a tag in soccer ball 1200, test machines may be used to induce failure and reveal weak points in the construction. In addition, soccer game play may be used as the final test of the best performing tags embedded in balls. To reduce the number of soccer balls needed for testing, multiple antenna tags may be installed in each ball.

The material selection and installation methods may be important areas in the construction analysis. The space allocated for tag 9500 in the soccer ball top level assembly is a slightly curved area on one of the carcass panels with a diameter less than 2.1 inches. The 2.1-inch diameter area may undergo extreme flexing and folding when the ball is in use. Given these constraints, printed circuit board (PCB) material and assembly processes should be reviewed for implementation. With some embodiments, planar antenna elements may be implemented using standard PCB construction methods.

With some embodiments, materials with acceptable properties, processes, lead time and cost include 4 mil thick FR4 PCB and 2 mil flex circuit PCB. The bottom surfaces of antennas 9502 have no components and are a smooth surface with a uniform solder mask. To prevent soldered joints from cracking during ball flexing, a stiff reinforcement coating such as ResinLab EP965 and/or a resin stiffener may be added to the top surface around the components. The reinforcement coating or stiffener may be as small as possible to minimize the stress induced during flexure and limit the potential for the reinforcement from failing. To mitigate potential reinforcement failure (delamination or cracking of the reinforcement coating on the circuit board material), a material with lower durometer may be used in some embodiments, e.g., Humiseal 1A33 or Dow Corning 1-1277.

Also, the adhesive used to hold tag 9500 to the carcass may induce strain during flexure of the tag material/conformal coating or may crack solder joints if the PCB material stretches. The adhesive may be applied over a small surface area to minimize potential strain on the tag material or copper traces. Possible adhesives include barge, 3M 300LSE, scotch weld epoxy adhesive 2216, Scotch weld rubber adhesive 1300, or Loctite 330. An alternative approach that avoids potential adhesive transferred strain comprises a fabric or latex pocket to hold antenna 9502. The pocket may be made with a known ball construction material and adhesive.

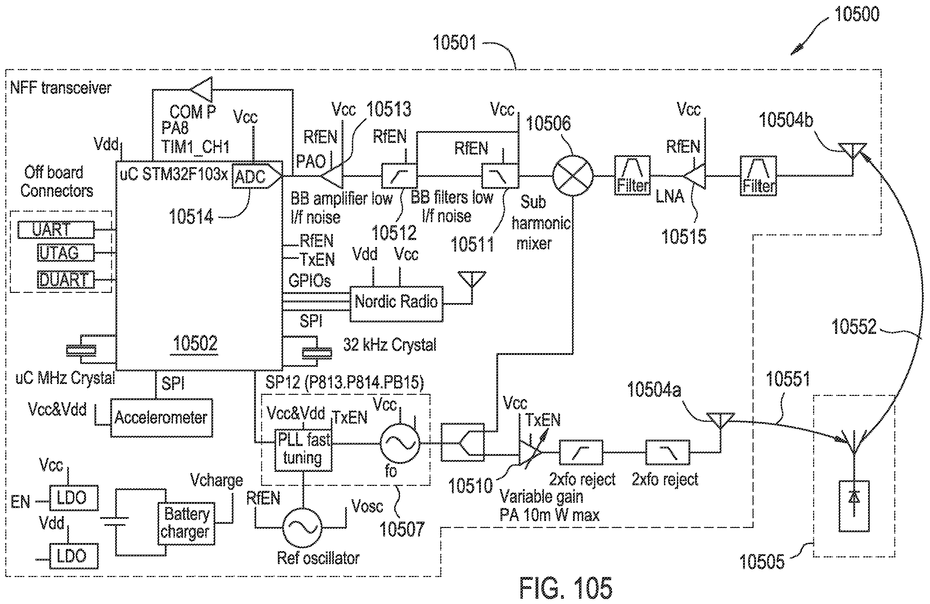

Fig. 105 shows system 10500 for performance monitoring system 100 (as shown in Figure 1) in accordance with embodiments of the invention. Component areas of the transceiver architecture in shoe 104 include radio section 10501, microcontroller 10502, power management (not explicitly shown), and antenna 10504. Tag 10505 is typically located at ball 130 as shown in Figure 1.

System 100 may estimate the distance between tag 10505 and transceiver antennas 10504 by transmitting a signal to a frequency doubling circuit in tag 10505 and processing the returned frequency doubled signal. System 100 may include radio section 10501 that transmits a wide bandwidth digitally modulated signal to a wide band antenna that reflects some portion back to the transceiver at double the frequency. The returned signal contains with both digital modulation and a frequency offset. The distance between antennas 10504a, 10504b and tag 10505 is typically proportional to the frequency offset. The frequency offset is converted to a difference frequency by mixer 10506 and the second harmonic of transmitted digitally modulated signal.

The antenna for tag 10505 may incorporate antenna 9600, antenna 9900, or antenna 10200 as discussed above. However, antennas 10504a, 10504b may comprise a planar inverted F antenna with some embodiments. Antennas 10504a, 10504b may address different considerations because antennas 10504a, 10504b are typically located in a player’s garment or shoe having a different antenna environment than that of a ball. For example, the proximity (typically varying) of a player’s shoe to the ground may have a substantial effect on the electrical characteristics of antennas 10504a, 10504b.

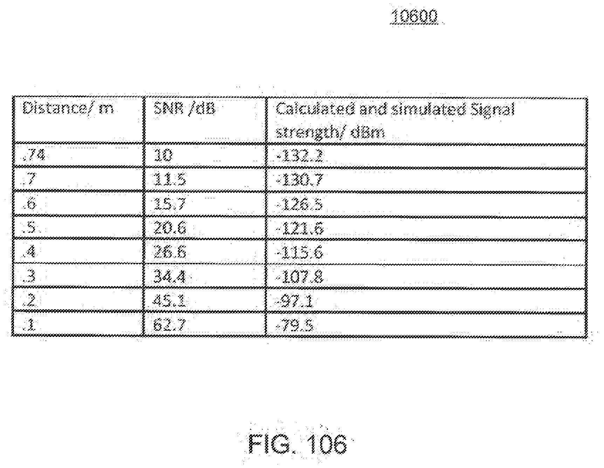

The link budget is typically a calculation of the range using parameters such as the frequency of operation, RF path loss, bandwidths, noise figures, transmitted power, antenna gains and propagation models. The link budget is used to determine the required radio specifications to meet desired range or to determine the range as a result of selected system performance parameters. Fig. 106 summarizes the link budget calculations showing the SNR with distance for exemplary simulation results.

Radio section 10501 may be controlled through firmware executing on a highly integrated system on chip based microcontroller component 10502. For example, microcontroller 10502 may comprise a microcontroller from the MSP430 family with 256kB of flash memory, 16 kB of RAM memory (which may be included as part of data storage not explicitly shown), and built in analog-digital converter 10514. Microcontroller 10502 controls radio section 10501 and stores the data that represents the distance between the tag and the transceiver antennas. Radio section 10501 may be controlled through a system packet interface (SPI) interface and general purpose input/outputs (GPIOs). Microcontroller 10502 may also control a lithium ion battery charging circuit. A rechargeable battery may be selected due to the current consumption and for the lightweight, compact form factor available through lithium polymer cells. Radio section 10501 includes transmitter and receiver sections. The transmitter includes a phase lock loop (PLL) synthesizer, transmit amplifier and several filters. The receiver includes a low noise amplifier 10515, harmonic mixer 10506, PLL synthesizer based local oscillator 10507, IF section 10513, and filters 10511 and 10512.

The processed signal from IF section 10513 may be digitized by analog-digital converter 10514, and the resulting frequency difference information (as will be further discussed) may be processed by controller 10502 to estimate the distance between the player and the ball.

While radio section 10501 combines both transmitting and receiving operations, some embodiments may separate transmitting operation from receiving operation. In such a case, separate antennas may be used for transmitting (antenna 10504a) and for receiving (antenna 10504b) as shown in Figure 105 rather than using a common antenna.

The distance may be estimated through receiving a signal relayed through the frequency doubling tag 10505. With some embodiments, a transmission is sent from the transceiver when the microcontroller programs A PLL synthesizer through a series of frequency tunes that represent a Gaussian frequency-shift keying (GFSK) modulation. The transmitted signal has wideband GFSK modulation, is centered at 2442 MHz, has an approximate bandwidth of 80 MHz, and an output power of approximately 10 mW. For example, variable control oscillator (VCO) 10507 sweeps the transmitted frequency from 2402 MHz to 2483 MHz during a time duration (*e.g.*, several hundred microseconds) to generate a sweep signal that may be referred as a chirp.

With a multi-player environment, it is typically desirable to avoid the overlapping of chirps generated by different transceivers, where each transceiver is associated with a different player. If chirps for different players overlap, the resulting return signals may interact with each other, causing an error in determining the correct difference frequency and consequently the estimated distance to the ball. Embodiments of the invention may use different approaches for collision avoidance, including randomizing generation of chirps to reduce the probability of collisions.

The radiated transmission is relayed through passive frequency doubling tag 10505. The second harmonic of the signal is received and centered at 4884 MHz. Consequently, tag 10505 doubles the transmitted frequency of the sweep signal from radio section 10501.

With embodiments of the invention, radio section 10501 transmits the sweep signal over transmission path 10551 to tag 10505 and simultaneously receives a signal from tag 10505 over receive path 10552 (*i.e.*, full duplex operation) in contrast to traditional systems in which a complete chirp must be transmitted before processing the returned signal. With the exemplary embodiment shown in Figure 105, the sweep signal is transmitted through amplifier 10510 while the returned signal is simultaneously received.

The received signal at radio section 10501 is amplified by low noise amplifier (LNA) 10515 and filtered by filter 10516 and filter 10517. The amplified signal is down converted to the baseband and IF circuit through the multiplication in the subharmonic mixer with the PLL synthesizer output. With some embodiments, the IF circuit has a bandwidth of 200-50000 Hz with a high-pass characteristic starting at 200 Hz. and comprises four very lower power OPAMPs in a 4th order low pass Butterworth Sallen Key configuration with an approximate gain of 57 dB.

The volumetric estimate of the layout based on block diagram 10500 may be calculated assuming microvias and compact component selections. The layout of block diagram 10500 may include some large connectors for loading firmware, extracting data, and debugging that may be needed in a final version and may not be included in the volumetric estimate. Exemplary dimensions for the PCB are approximately 1 by 1 by.3 inches. If connectors are included, the interior volume may be 2/3 higher. Items not included in the volumetric estimate are the antenna and the enclosure. The rechargeable battery may be approximately 1 by .5 by.3 inches.

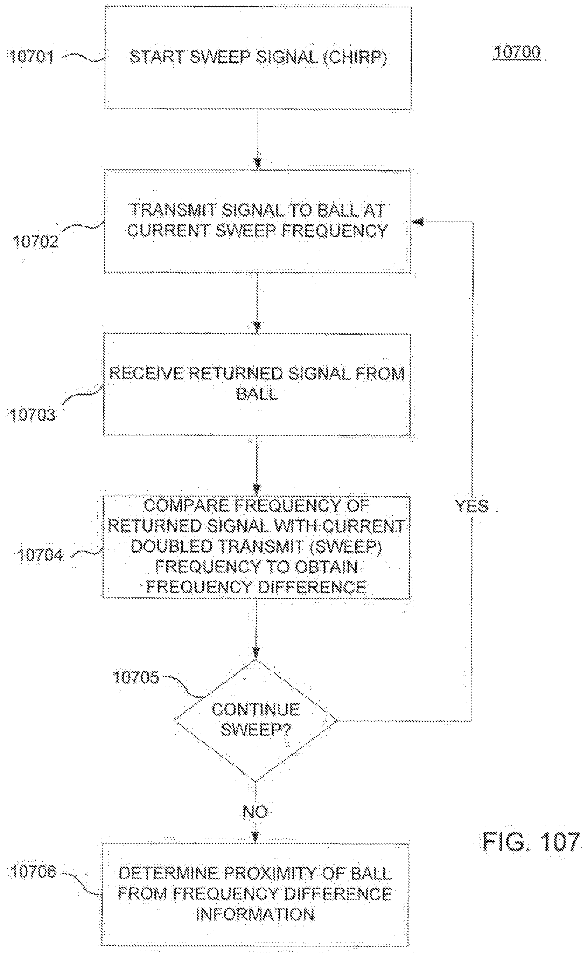

Figure 107 shows flow chart 10700 that may be performed by system 10500 as shown in Fig. 105 in accordance with embodiments of the invention. With some embodiments, VCO 10507 independently changes the frequency of the transmitted signal in a predetermined series of frequency changes once initiated by controller 10502. Embodiments of the invention may utilize different frequency tuning characteristics including a linear, exponential, or a Gaussian function. However, with some embodiments, controller 10502 may directly control VCO 10507 to generate the transmitted frequency.

Some or all blocks 10701-10706 may be performed sequentially or may be performed in parallel.

Controller 10502 may instruct VCO 10507 to start the chirp at block 10701. As previously discussed, the generation of the chirp may be randomized to reduce the probability that a chirp is being generated by a transceiver associated with a different player in a multi-player environment. Radio section 10501 consequently transmits the sweep signal to tag 10505 at block 10702 and simultaneously receives the returned signal from tag 10505 at block 10703. Because of the propagation time between radio section 10501 and tag 10505, the doubled transmitted frequency is different from the received frequency.

At block 10704 radio section 10501 compares the frequency of the returned signal from tag 10505 with the current doubled sweep frequency to obtain a frequency difference.

Execution of blocks 10702-10704 continues during the time duration that the chirp occurs as determined by block 10705. When the sweep has been completed, the frequency difference information is used to determine the distance (proximity) between the player and the ball at block 10706 based on EQs. 1-3 as will be discussed. For example, the average value of the frequency difference over the measurement time duration may be used to estimate the distance between the player (corresponding to radio section 10501) and the ball (corresponding to tag 10505). A plurality to measurements of the frequency difference may be obtained. When the variability of the frequency difference measurements is greater than a predetermined threshold, process 10700 may determine that the proximity estimate is not acceptable and may generate another chirp to repeat process 10700.

Once the chirp has been sent and the proximity estimated, process 10700 may be repeated to update the estimated proximity.

The frequency change may be performed in a continuous or a discrete basis. For example, the transmitted frequency may be linearly increased over a predetermined time duration using the following relationship:

$$f_{transmitted}(t) = f_0 + \alpha t \qquad \textbf{(EQ. 1)}$$

where $f_0$ is the initial frequency and $\alpha$ is the change of frequency during the predetermined time duration. Tag 10505 returns a received signal in which the received frequency of transmitted signal is doubled. Accounting for the one-way propagation time (T) between antennas 10504 and 10505:

$$f_{received}(t) = 2f_0 + \alpha(t-2T) \qquad \textbf{(EQ. 2)}$$

The frequency of the transmitted signal is doubled ($f_{2,t}(t)$ which represents the second harmonic of the currently transmitted signal) and is mixed with the received signal (corresponding to EQ. 2) by mixer 10506 to obtain a frequency difference:

$$f_{2,t}(t) - f_{received}(t) = 4\,\alpha T \qquad \textbf{(EQ. 3)}$$

While EQs. 1-3 model a continuous process, some embodiments may change the transmitted frequency as discrete process, where the sweep (transmitted) frequency is incremented $\Delta$ every time increment $t_{i+1} - t_i$. Rather than determining the frequency difference using EQ. 3, some embodiments may use a predetermined lookup table that is represented by Table 1, where data representing Table 1 is stored in memory. Consequently, controller 10502 may determine the frequency difference and use the value to lookup up the propagation time T. The distance between the player and the ball may then be determined from the determined propagation time.

| TABLE 1: PROPAGATION TIME AS A FUNCTION OF FREQUENCY DIFFERENCE | | | | |
|---|---|---|---|---|
| PROP. TIME | FREQUENCY DIFF = $\Delta_1$ | FREQUENCY DIFF = $\Delta_2$ | FREQUENCY DIFF = $\Delta_3$ | FREQUENCY DIFF = $\Delta_4$ |
| $t_1$ | $T_1$ | | | |
| $t_2$ | $T_1$ | $T_2$ | | |
| $t_3$ | $T_1$ | $T_2$ | $T_3$ | |
| $t_4$ | $T_1$ | $T_2$ | $T_3$ | $T_4$ |

With some embodiments, rather than obtaining the propagation time from the frequency difference, the distance between the tag (corresponding to the ball) and the transceiver (corresponding to the player) may be varied by experimentation. The corresponding frequency difference may be observed without specific knowledge of the multipath characteristics between the transceiver and the tag. The experimental information may then be later used when monitoring player performance during athletic activities.

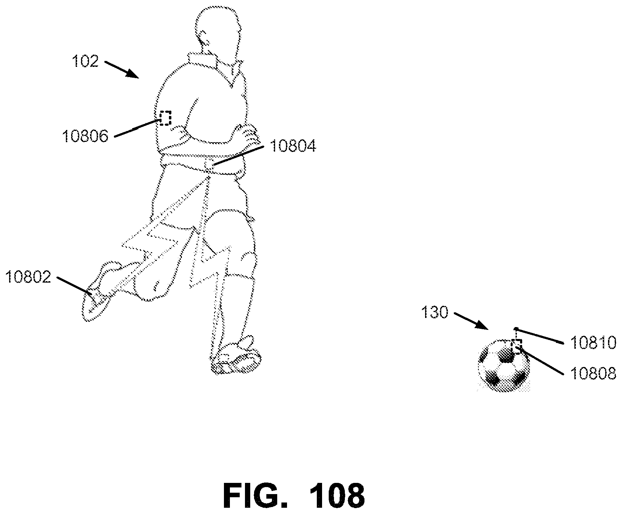

In accordance with additional examples, athlete 102 may wear at least one of a camera and transducer (e.g., microphone) to assist in determining whether the athlete is in possession of an object (e.g., soccer ball). Fig. 108 illustrates an example embodiment where a camera and a transducer are worn by an athlete while participating in an athletic activity. In the depicted example, athlete 102 is playing soccer; however, the examples discussed herein may be implemented in other sports where it is desirable to maintain possession of an object (e.g., a ball).

In an example, a shoe of the athlete 102 may include a transducer 10802 to collect sounds while the athlete participates in an athletic activity. Transducer 10802 may be embedded within a sole of the shoe, a tongue of the shoe, etc. Transducer 10802 may also be attached using laces of the shoe. Transducer 10802 may also be placed at any other desired location on athlete 102. Examples sounds collected by transducer 10802 may include kicking of a football, trapping of a soccer ball with a chest or foot, kicking a soccer ball, heading a soccer ball, etc. Transducer 10802 may include a processor to process collected transducer data, or may be coupled to a transceiver to communicate raw transducer data to another device (e.g., receiver 108, remote computer system 120, etc.) for processing. Athlete 102 may also wear multiple transducers positioned at any desired locations on the athlete's clothing or body. The transducers may be in wired or wireless communication with each other, as well as with other devices (e.g., receiver 108, remote computer system 120, etc.).

Camera 10804 may be attached to athlete 102. In an example, camera 10804 may be incorporated into receiver 108, discussed above, that is worn by athlete 102. Receiver 108 may include an accelerometer to measure acceleration data associated with athlete 102 as well as a battery to power the camera 10804. In other examples, camera 10804 may be a standalone device, or may be incorporated to other devices worn by athlete 102. Camera 10804 may be placed at other locations on the user than that shown in Fig. 108. For example, camera 10804 may be included in a shoe or soccer shin guard and may include a wide-angle lens. In another example, camera 10804 may communicate wirelessly to a phone or watch worn by athlete 102. Also, athlete 102 may wear multiple cameras positioned at desired locations on the athlete's clothing or body. The cameras may be in wired or wireless communication with each other, as well as with other devices (e.g., transducers 10802, remote computer system 120, etc.).

In an example, transducer 10802 and/or camera 10804 may be communicatively coupled to a transceiver 10806. For example, transceiver 10806 may be integrated within a single apparatus worn on body by athlete 102 that also houses one or both of transducer 10802 and camera 10804. Transceiver 10806 may also be a standalone unit worn by athlete 102. Transceiver 10806, transducer 10802 and camera 10804 may be controlled based on instructions received from at least one processor executing instructions stored by at least one memory to perform the functions described herein. Transceiver 10806, transducer 10802 and camera 10804 each may incorporate at least one processor, or may the at least one processor may be included in a different device (e.g., receiver 108, remote computer system 120, etc.).

Data generated by the transducer(s) 10802 and the camera(s) 10804 may be used to determine when the athlete 102 is in possession of object 130. The following describes remote computer system 120 processing data to determine whether athlete 102 is in possession of an object (e.g., soccer ball); however, other devices worn by athlete 102 or remote from athlete 102 instead of or in addition to system 120 may process the data. In an example, transducer(s) 10802 may communicate transducer data to remote computer system 120, and camera(s) 10804 may communicate image data to remote computer system 120.

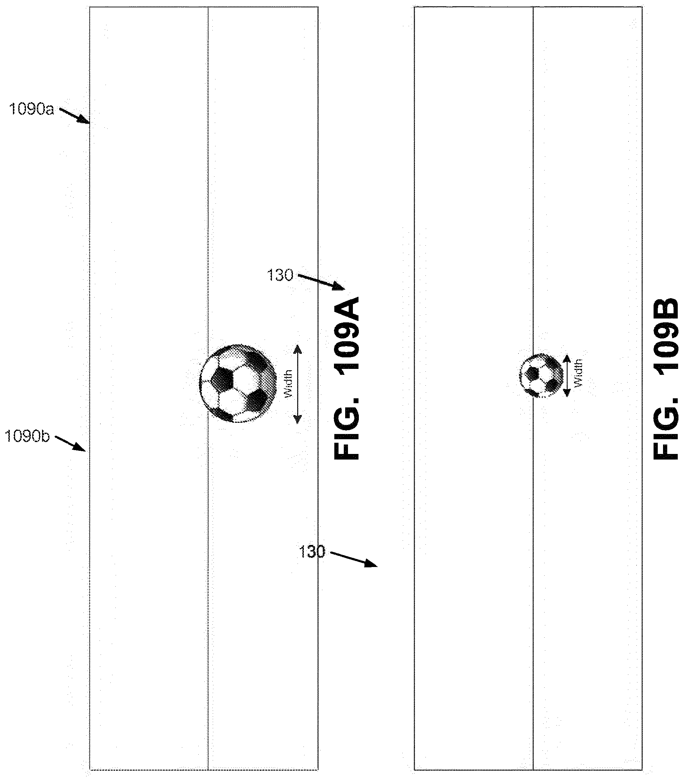

In an example, remote computer system 120 may process image data received from one or more cameras 10804 worn by athlete 102 during an athletic activity. Figs. 109A-B illustrate example images 1090a-b generated by camera 10804 worn by a user in accordance with example embodiments. Each of images 1090a-b may depict an object 130, such as, for example, a soccer ball. An actual size of the soccer ball may be relatively constant during the athletic activity, but the size of the soccer ball in each image 1090a-b may vary based on distance of the ball from camera 10804. For example, in image 1090a, soccer ball may appear to be larger than in image 1090b, due to the soccer ball in image 1090a being closer to the camera 10804 when image 1090a was taken as compared to when image 1090b was taken. Remote computer system 120 may process image data generated by the camera 10804 to determine a size of the soccer ball in each image. In an example, remote computer system 120 may determine the size of the soccer ball as one or more of a height, width, and area of the soccer ball within each image 1090a-b. As seen in Fig. 109a, the width of the soccer ball in image 1090a is larger than the width of soccer ball in image 1090b. Object 130 (e.g., soccer ball) may include a special reflective paint or may be made of a reflective material to aid remote computer system 120 in identifying object 130 within each image 1090a-b.

Remote computer system 120 may compare the determined size of object 130 to a size threshold to determine whether athlete 102 is in possession. In an example, remote computer system 120 may compare any or all of determined height, width, and area of object 130 to a corresponding threshold (e.g., height threshold, width threshold, area threshold, etc.). If the determined size is greater than or equal to the corresponding threshold, remote computer system 120 may determine that athlete 102 is in possession of object 130. If the determined size is less than the corresponding threshold, remote computer system 120 may determine that athlete 102 is not in possession of object 130.

In a further example, remote computer system 120 may receive image data from multiple cameras worn by multiple athletes to determine which athlete is in possession of object 130. Remote computer system 120 may receive and process image data from each camera to determine which camera provided image data in which object 130 has the largest size, and may determine that an athlete associated with that camera is in possession of object 130. In an optional example, if the largest size is less than the size threshold, remote computer system 120 may determine that none of the athletes are in possession of object 130.

In a further example, remote computer system 120 may process image data received from one or more cameras 10804 to determine speed of the object 130. In an example, remote computer system 120 may process image data over a sequence of images to detect a change in size of the object 130. Remote computer system 120 may process image data to determine a rate at which object 130 increases or decreases over time, and may translate this into speed of object 130. In another example, athlete 102 may wear an accelerometer, and remote computer system 120 may adjust a speed determined for object 130 based on a speed at which athlete 102 is moving.

Possession of object 130 may also be determined based on processing data provided by transducer 10802. In many instances, for example, a ball is a fixed size and contains a certain volume of air. When a ball is struck (e.g., when bounced on a hard surface, a dirt surface, a grass covered surface, when kicked or punched, etc.), sound is produced that resonates at a particular frequency based on the size and shape of the ball. Striking the ball may also cause a change in pressure within the ball where the pressure propagates from the surface that was struck to an opposite side of the ball. Once the opposite side is reached, the pressure signal may reflect resulting in an audible echo. The sound generated by initially striking the ball along with one or more echoes may be considered a sound signature for the ball. The sound signature may be unique and may be based on the way in which the ball was struck. For example, the ball may have a first signature when kicked that differs from a second signature when bounced on a hard surface (e.g., cement, hardwood flooring, etc.), and that differs from a third signature when bounced on a grass covered surface.

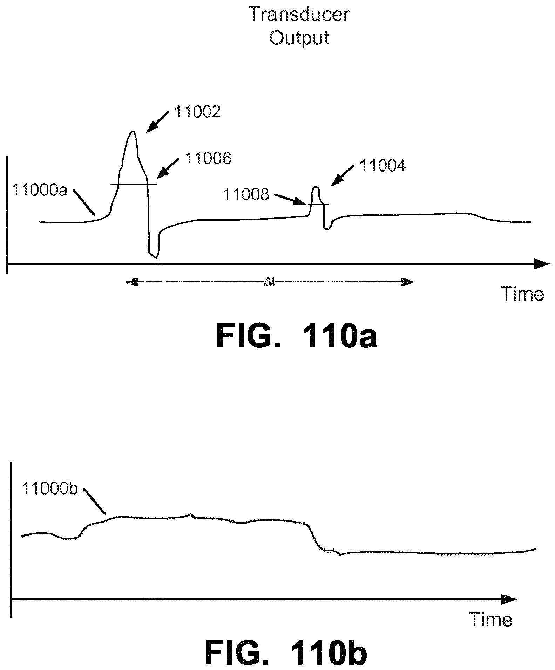

Transducer 10802 may capture transducer data for determining when athlete 102 is in possession of object 130 based on comparing the transducer data to one or more sound signatures. In an example, transducer 10802 may capture and convert sound into an electrical signal when object 130 is used to participate in an athletic activity. Figs. 110a-b illustrate example signals output by transducer 10802 in accordance with an example embodiment. Fig. 110a may correspond to a signal 11000a when a ball is kicked or impacts a hard surface. Signal 11000a may include a large spike 11002 corresponding to when the ball is initially struck. At a later time, signal 11000a may include an echo spike 11004. For comparison, Fig. 110b may depict a signal 11000b corresponding to when a ball is thrown.

Based on these different responses to being struck, signature templates may be created to permit remote computer system 120 to determine instances when athlete 102 is possession of object 130. A signature template may specify one or more parameters for determining possession based on transducer data. A signature template may be used to distinguish sounds associated with an athlete striking or manipulating object 130 during an athletic activity from other types of user movement that do not generate a sound signature.

In an example, a ball strike signature template may define parameters for a minimum peak voltage level 11006 for large spike 11002, a resonance frequency associated with spike 11002, a minimum peak voltage level 11008 for echo spike 11004, a resonance frequency associated with spike 11004, and a maximum amount of time (e.g., $\Delta t$) between peaks of spikes 11002 and 11004. A signature template may be defined using some of these or other parameters. Additional templates may be defined, and remote computer system 120 may compare the transducer data to the templates for determining when athlete 102 is in possession of object 130.

For example, a dribbling signature template may be created for when a soccer player is dribbling a soccer ball. A soccer player dribbling a soccer ball may frequently strike a ball with their feet, and this striking may have a particular sound signature. When a soccer player runs without the ball, transducer 10802 may pick up sounds, but these sounds will likely not include the sound signature unless nearby another player dribbling the soccer ball. The dribbling signature template may be used for identifying when transducer 10802 detects sounds associated with athlete 102 dribbling a soccer ball, and hence is in possession of the ball. Signature templates may thus permit remote computer system 120 to determine instances when athlete 102 is possession of object 130 based on transducer data.

In additional aspects, transducer 10802 may collect transducer data during an athletic activity and may forward the transducer data to remote computer system 120 for processing. If remote computer system 120 determines that the transducer data satisfies some or all parameters of a signature template, remote computer system 120 may determine that athlete 102 is in possession of object 130. Remote computer system 120 may determine whether athlete 102 is in possession of object 130 by comparing data provided by transducer 10802 with one or more signature templates. Remote computer system 120 may determine a time at which transducer data first satisfied some or all parameters of a signature template. Remote computer system 120 may then monitor whether comparisons with subsequent data provided by transducer 10802 continues to satisfy some or all parameters of a signature template. If they do, remote computer system 120 may increment the amount of time athlete 102 is determined to be in possession of object 130. If, however, a predetermined amount of time elapses and subsequent data provided by transducer 10802 does not satisfy some or all parameters of a signature template, remote computer system 120 may determine that athlete 102 is no longer in possession of object 130.

In another example, signature templates may be used to identify certain types of activities involving one or more athletes. In an example, a passing signature template may be created for when multiple soccer players are passing a soccer ball back and forth. Each soccer player may frequently strike a ball with their feet, and this striking may have a particular sound signature. The passing signature template may be used for identifying when transducer 10802 detects sounds associated with athletes 102 passing a ball back and forth. Remote computer 120 may also process transducer data to determine whether a certain type of activity has been detected.

In a further example, remote computer system 120 may receive transducer data from multiple transducers 10802 worn by multiple athletes to determine which athlete is in possession of object 130. Remote computer system 120 may receive and process transducer data to determine which of the transducers provided data that matches a signature template. For example, remote computer system 120 may determine whether data provided by one or both of the transducers includes a particular resonance frequency.

If only a single transducer provided data that satisfies the template, remote computer system 120 may identify an athlete associated with that transducer as being in possession of object 130. If multiple transducers provided data matching a template, remote computer system 120 may determine that there is disputed possession of object 130. In a further example, if multiple transducers provided data matching a template, remote computer system 120 may determine which transducer provided data of a signal having a largest amplitude, and may identify an athlete associated with that transducer as being in possession of object 130. Remote computer system 120 may also determine which athlete had possession immediately prior to a disputed possession as well as which athlete has possession immediately after the disputed possession when determining which athlete involved in a disputed possession is currently in possession of object 130.

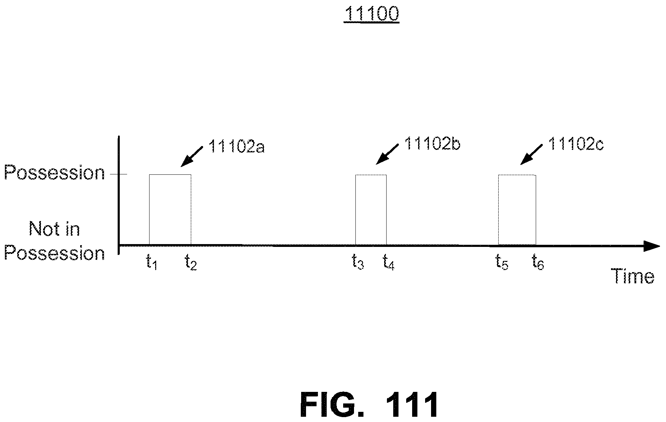

At the conclusion of an athletic activity, athlete 102 may access remote computer system 120 using, for example, a computer, a smart phone, or other device to determine during which periods of time of an athletic activity athlete 102 was in possession of object 130. Fig. 111 illustrates an example possession display 11100 in accordance with example embodiments. The possession display 11100 may indicate time periods during which athlete 102 was determined to be in possession of the object. For example, blocks 11102a-c may represent time intervals during which the athlete was in possession. The times between blocks 11102a-c may represent time intervals during which the athlete was not in possession of object 103.

Remote computer system 120 may also synchronize video of athlete 102 participating in an athletic activity with possession display 11000. In a further example, possession display 11000 may permit athlete 102 to move throughout the synchronized video to view video of when athlete 102 was determined to be in possession of object 130. For example, remote computer system 120 may associate time markers based on transitions of possession. Remote computer system 120 may use the time markers to concatenate the video for showing only segments of the video corresponding to when athlete 102 was determined to be in possession of object 130. Thus, the concatenate video may not include any of the video of when athlete 102 was not in possession. In a further example, to show transitions when athlete 102 gained possession of object 130, the concatenated video may include a predetermined amount of time before athlete 102 was determined to obtain possession of object 130.

Conversely, remote computer system 120 may concatenate the video using the time markers for showing only segments of the video corresponding to when athlete 102 was determined not to be in possession of object 130. In a further example, to show transitions when athlete lost possession of object 130, the concatenated video may include a predetermined amount of time before athlete 102 was determined to no longer have possession of object 130.

In another example, remote computer system 120 may concatenate the synchronized video to only include portions of the synchronized video in which transitions in possessions occurred involving athlete 102, as well as predetermined amounts of time before and after each transition.

In a further example, remote computer system 120 may process video for a team associated with athlete 102. For example, transducer data may include identifier information uniquely identifying each transducer 10802 worn by each team member. Remote computer system 120 may concatenate video by team to show when members of a first team took possession of object 130 away from a second team and to show when members of a first team lost possession of object 130 to a second team. Remote computers system 120 may include a predetermined amount of time in the video before and after each transition.

Remote computer system 120 may provide statistical possession information for each member of a team. In an example, system 120 may calculate a total amount of time during an athletic activity any member of a team was determined to be in possession of object 130, an amount of time each member was individually in possession, a length of time of each possession by each member, average length of possession (e.g., by team, by player, etc.), as well as other statistical metrics for possession based on time. Remote computer system 120 may also rank members based on length of time object 130 was possessed. The ranking may be a weighted ranking where possession preceding a particular desirable event (e.g., goal) is weighted more heavily than possession at other times.

In an example embodiment, remote computer system 120 may process the image data and the transducer data in combination to determine whether the athlete is in possession of object 130. For example, remote computer system 120 may make a first determination about whether athlete 102 is in possession of the object based on the image data, and make a second determination about whether athlete 102 is in possession of the object based on the transducer data. If either of the first and second determinations indicates that athlete 102 is in possession, then remote computer system 120 may conclude that athlete 102 is in possession even if the other of the first and second determinations indicates otherwise. In a further example, remote computer system 120 may only determine athlete 102 is in possession if both the first determination and the second determination indicate that athlete 102 has possession.

With reference again to Figure 108, to conserve life of a battery or other power source, transceiver 10806 may control when power is supplied to each of transducer 10802 and camera 10804. In an example, transceiver 10806 may communicate a proximity signal and listen for a return signal from object 130. For example, the proximity signal and the return signal may be radio frequency signals. Either or both may be encrypted. In an example, object 130 may include an antenna 10810 to receive the proximity signal and a response circuit 10808 for responding with the return signal. In an example, response circuit 10808 may incorporate a frequency doubler tag 9500, frequency doubler circuit 9501, or other device that may send a return signal in response to receiving a signal from transceiver 10806.

Transceiver 10806 may periodically or randomly communicate the proximity signal. Optionally, transceiver 10806 may encode or otherwise provide information for uniquely identifying transceiver 10806 in the proximity signal. The return signal may also be encoded or otherwise provide information for uniquely identifying object 130.

Transceiver 10806 or other processor may determine that object 130 is nearby when the return signal is received within a predetermined amount of time after communicating a proximity signal. For example, transceiver 10806 may have a first transmission range (e.g., 20 feet), and response circuit 10808 may have a second transmission range (e.g., 10 feet). The transmission ranges may be the same or different. Each proximity signal may also include timing information (e.g., time stamp) and the return signal may include the received timing information. Transceiver 10806 or other processor may process the timing information from the return signal to determine whether the return signal is received within a predetermined amount of time after a proximity signal was communicated.

In response to receiving a return signal, transceiver 10806 may cause power (e.g., battery power) to be supplied to one or both of transducer 10802 and camera 10804. For example, transceiver 10806 may communicate a power up signal to one or both of transducer 10802 and camera 10804. Transducer 10802 and camera 10804 may then collect data for determining whether athlete 102 is in possession of object 130. Transceiver 10806 may periodically or randomly communicate the proximity signal and listen for a return signal. If a return signal is not received within a predetermined amount of time, transceiver 10806 may remove power from being supplied to one or both of transducer 10802 and camera 10804 to conserve battery life. For example, transceiver 10806 may communicate a power down signal to one or both of transducer 10802 and camera 10804. Transceiver 10806 may continue communicating the proximity signal and listening for a return signal, thus limiting supplying power to transducer 10802 and/or camera 10804 to only situations when object 130 is nearby.

Transceiver 10806 may simultaneously or sequentially turn on camera 10804 and transducer 10802 based on whether a return signal is received from object 130. In an example, when a return signal is received, transceiver 10806 may first turn on transducer 10802, but leave camera 10804 turned off. Transducer 10802 may generate and process transducer data to determine when athlete 102 has contacted object 130 (e.g., kicked a ball, dribbled a ball, etc.). Subsequent to determining that athlete 102 has contacted object 130, transducer 10802 may communicate a power-on instruction instructing camera 10804 to turn on. Camera 10804 may then attempt to determine possession in conjunction with transducer 10802, as discussed above.

In another example, transceiver 10806 may first turn on camera 10804 subsequent to detecting a return signal, but leave transducer 10802 turned off. Camera 10804 may determine when athlete 102 is in possession or nearby to object 130, as described above. Subsequent thereto, camera 10804 may communicate a power-on instruction instructing transducer 10802 to turn on. Transducer 10802 may then attempt to determine possession in conjunction with camera 10804, as discussed above. Thus, coordinated operation of camera 10804, transducer 10802, and transceiver 10806 may save power by turning one or more off when object 130 is not within range of transceiver 10806.

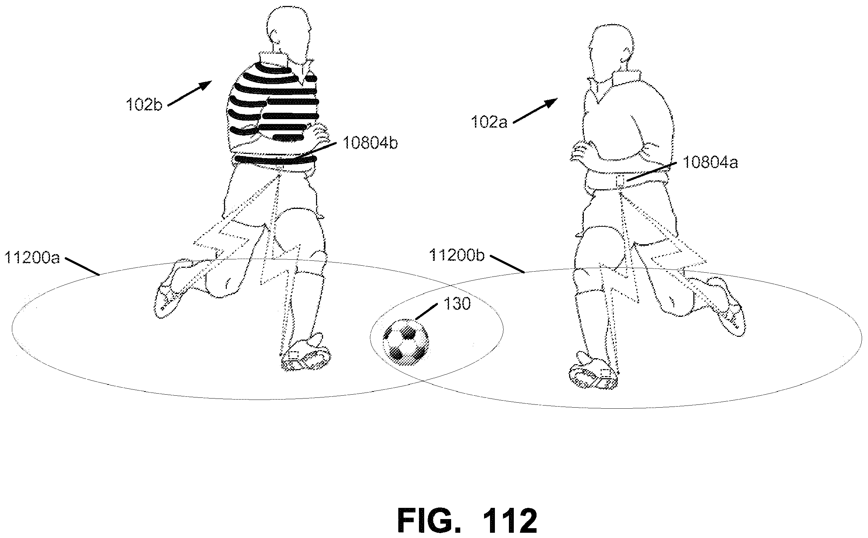

In additional aspects, remote computer system 120 may process image data from multiple players for determining which has possession of an object. Fig. 112 illustrates an example of determining contested possession of object 130 in accordance with example embodiments. As depicted, athletes 102a-b are attempting to possess object 130. Rings 11200a-b may correspond to a distance within which an athlete is determined to have possession. In Fig. 112, rings 11200a-b suggest that both athletes 102a-b possess object 130. When multiple athletes are within a predetermined distance of object 130, remote computer system 120 may determine that there is contested possession of object 130. Remote computer system 120 may identify contested possession when image data from multiple devices includes the object, and the size of the object from each device satisfies a size threshold.

When contested possession is identified, remote computer system 130 may determine which athlete had possession immediately prior to identifying contested possession. If the determined athlete is an athlete other than athletes 102a-b, remote computer system 130 may determine that neither of athletes 102a-b has established possession of the object. 130. If the determined athlete is one of athletes 102a-b, remote computer system 130 may increment the amount of time the determined athlete has possession.

During contested possession, remote computer system 120 may determine which of the athletes 102a-b has possession based on size of the object determined from the image data from the respective cameras 10804a-b. For example, remote computer system 120 may process image data from camera 10804a and determine that the size of object 130 is width W1. Remote computer system 120 may process image data from camera 10804b and determine that the size of object 130 is width W2. Remote computer system 120 may compare width W1 and W2, and determine that the athlete associated with image data that more largely depicts the object 130 has possession. For example, in Fig. 112, object 130 is closer to athlete 102b than athlete 102a. Object 130 would thus appear to be larger in image data generated by camera 10804b than in image data generated by camera 10804a. Remote computer system 120 may thus determine that athlete 102b has possession of the object.

In a further example, remote computer system 120 may determine that neither athlete has possession during periods of contested possession, and may only conclude that an athlete is in possession when a size of object 130 within image data provided by a single camera, and no other cameras, exceeds the size threshold.

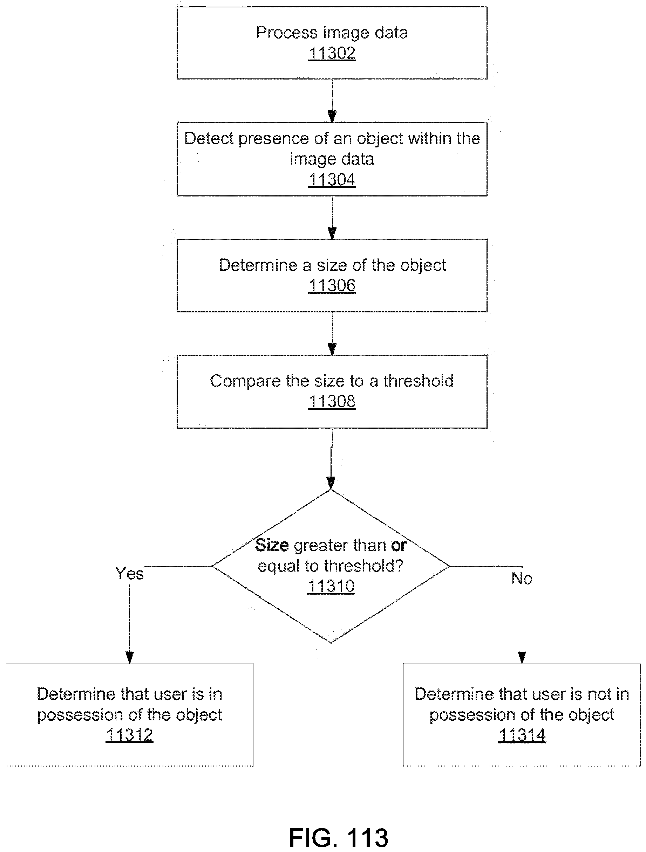

Figure 113 illustrates an example flow diagram of a method for determining whether a user is in possession of an object, in accordance with example embodiments. The method may be implemented by a single apparatus such as, for example, a computer, server, or other computational device, and/or may be stored on a non-transitory computer readable medium as a set of one or more computer executable instructions. The single apparatus may be worn by an athlete while participating in an athletic activity, or may be remote from the athlete. In other aspects, the method may be performed by multiple devices (e.g., multiple computers, multiple processors, and the like). The order of the blocks shown in Figure 113 is an example. The blocks may be arranged in other orders, each function described in each block may be performed one or more times, some blocks may be omitted, and/or additional blocks may be added. The method may begin at block 11302.

In block 11302, the method may include processing image data. In an example, remote computer system 120 may receive and process image data from camera 10804. In block 11304, the method may include detecting presence of an object within the image data. In an example, remote computer system 120 may detect presence of a ball within the image data. In block 11306, the method may include determining a size of the object. In an example, remote computer system 120 may determine a width of the soccer ball within the image data. In block 11308, the method may include comparing the size to a threshold. In an example, remote computer system 120 may compare the width to a threshold size for width. In block 11310, the method may include determining whether the size meets or exceeds the threshold. In block 11312, the method may include determining that a user is in possession of the object if the size meets or exceeds the threshold. In block 11314, the method may include determining that a user is not in possession of the object if the size does not meet or exceed the threshold. For example, remote computer system 120 may determine that the user is in possession of a soccer ball if the determined width is greater than or equal to a threshold width, and may determine that the user is not in possession of the soccer ball if the determined width is less than the threshold width. The method may then end, may repeat one or more times, and/or may return to any of the preceding blocks.

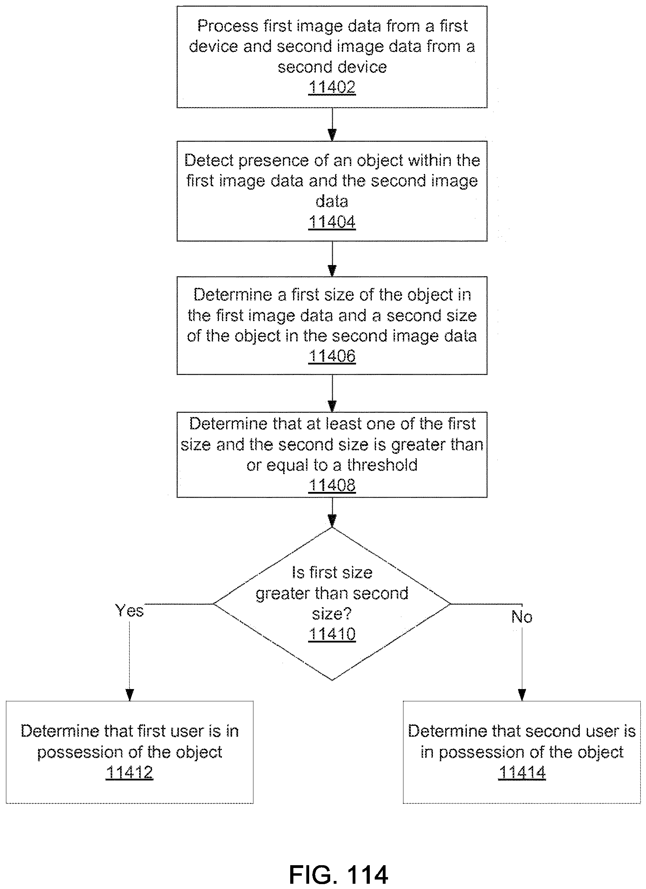

Figure 114 illustrates an example flow diagram of a method for determining which of multiple users is in possession of an object, in accordance with example embodiments. The method may be implemented by a single apparatus such as, for example, a computer, server, or other computational device, and/or may be stored on a non-transitory computer readable medium as a set of one or more computer executable instructions. The single apparatus may be worn by an athlete while participating in an athletic activity, or may be remote from the athlete. In other aspects, the method may be performed by multiple devices (e.g., multiple computers, multiple processors, and the like). The order of the blocks shown in Figure 114 is an example. The blocks may be arranged in other orders, each function described in each block may be performed one or more times, some blocks may be omitted, and/or additional blocks may be added. The method may begin at block 11402.

In block 11402, the method may include processing first image data from a first device and second image data from a second device. In an example, remote computer system 120 may receive first image data from a first camera 10804a and may receive second image data from a second camera 10804b. In block 11404, the method may include detecting presence of an object within the first image data and the second image data. In an example, remote computer system 120 may detect presence of a soccer ball in image data from each of the first and second cameras 10804a-b. In block 11406, the method may include determining a first size of the object in the first image data and a second size of the object in the second image data. In an example, remote computer system 120 may determine a size of the soccer ball in image data from the first camera 10804a and a size of the soccer ball in image data from the second camera 10804b. In block 11408, the method may include determining that at least one of the first size and the second size meets or exceeds a threshold. In block 11410, the method may include determining whether the first size is greater than the second size. In block 1412, the method may include determining that a first user associated with the first device is in possession of the object if the first size exceeds the second size. In block 11414, the method may include determining that a second user associated with the second device is in possession of the object if the second size exceeds the first size. In an example, remote computer system 120 may compare a size of the soccer ball from the image data of the first camera 10804a with a size of the soccer ball from the image data of the second camera 10804b, and determine that the user associated with the larger soccer ball size is in possession. The method may then end, may repeat one or more times, and/or may return to any of the preceding blocks.

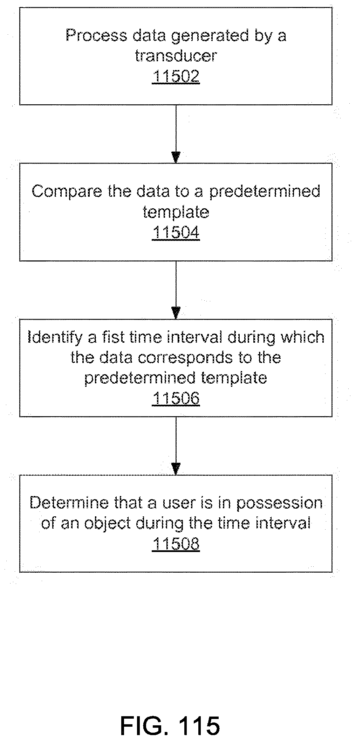

Figure 115 illustrates an example flow diagram of a method for processing transducer data for determining whether a user is in possession of an object, in accordance with example embodiments. The method may be implemented by a single apparatus such as, for example, a computer, server, or other computational device, and/or may be stored on a non-transitory computer readable medium as a set of one or more computer executable instructions. The single apparatus may be worn by an athlete while participating in an athletic activity, or may be remote from the athlete. In other aspects, the method may be performed by multiple devices (e.g., multiple computers, multiple processors, and the like). The order of the blocks shown in Figure 115 is an example. The blocks may be arranged in other orders, each function described in each block may be performed one or more times, some blocks may be omitted, and/or additional blocks may be added. The method may begin at block 11502.

In block 11502, the method may include processing data generated by a transducer. In an example, remote computer system 120 may receive and process data provided by transducer 10802. In block 11504, the method may include comparing the data to a template. In an example, remote computer system 120 may compare the transducer data to determine if one or more parameters of a signature template are satisfied. In an example, parameters may correspond to a particular resonance frequency, amplitude of one or more peaks, an amount of time between peaks, etc. Remote computer system 120 may, for example, determine whether the data includes a particular resonance frequency. In block 11506, the method may include identifying a first time interval during which the data corresponds to the template. In block 11508, the method may include determining that a user is in possession of an object during the first time interval. In an example, remote computer system 120 may determine a time period during which the transducer data satisfies the one or more parameters of the template, and determine that the user is in possession of the object at a beginning of the time period. Remote computer system 120 may determine that the user maintains possession of object 130 so long as subsequent data provided by transducer 10802 matches the template within a predetermined amount of time. The method may then end, may repeat one or more times, and/or may return to any of the preceding blocks.

## IV. Conclusion

The present invention is described above and in the accompanying drawings with reference to a variety of example structures, features, elements, and combinations of structures, features, and elements. The purpose served by the disclosure, however, is to provide examples of the various features and concepts related to the invention, not to limit the scope of the invention. One skilled in the relevant art will recognize that numerous variations and modifications may be made to the embodiments described above without departing from the scope of the present invention, as defined by the appended claims. For example, the various features and concepts described above in conjunction with Figs. 1-115 may be used individually and/or in any combination or sub-combination without departing from this invention.

For the avoidance of doubt the present application extends to the subject-matter described in the following numbered paragraphs (referred to as "Para" or "Paras"):

Para 1. An apparatus comprising:

at least one processor; and
at least one memory storing executable instructions that, when executed by the at least one processor, cause the apparatus at least to:

process image data;
detect presence of an object within the image data;
determine a first size of the object;
compare the first size to a threshold;
determine that a first user is in possession of the object if the first size meets or exceeds the threshold, and determine that the first user is not in possession of the object if the first size is less than the threshold.

Para 2. The apparatus of claim 1, wherein the executable instructions, when executed by the at least one processor, further cause the apparatus to communicate a proximity signal for determining whether the object is within range for controlling whether power is supplied to a camera providing the image data.

Para 3. The apparatus of claim 2, wherein the executable instructions, when executed by the at least one processor, further cause the apparatus to detect a return signal within a first predetermined amount of time of communicating the proximity signal and to provide power to the camera in response to the detecting.

Para 4. The apparatus of claim 2, wherein the executable instructions, when executed by the at least one processor, further cause the apparatus to determine that a return signal has not been received within a predetermined amount of time of communicating the proximity signal. Para 5. The apparatus of claim 4, wherein the executable instructions, when executed by the at least one processor, further cause the apparatus to cause the camera to power down.

Para 6. The apparatus of claim 1, wherein the executable instructions, when executed by the at least one processor, further cause the apparatus to:

process second image data received from a second device;
detect presence of the object within the first image data and the second image data;
determine a second size of the object in the second image data; and
determine that at least one of the first size and the second size meets or exceeds the threshold.

Para 7. The apparatus of claim 1, wherein the executable instructions, when executed by the at least one processor, further cause the apparatus to:

receive first data from a first transducer; and
compare the first data to a template, wherein the template identifies a particular resonance frequency.

Para 8. The apparatus of claim 7, wherein the first transducer is included in an article of footwear

Para 9. The apparatus of claim 7, wherein the at least one processor and the at least one memory are remote from the first transducer.

Para 10. The apparatus of claim 7, wherein the first transducer, the at least one processor, and the at least one memory are configured to be worn by the first user.

Para 11. The apparatus of claim 7, wherein the executable instructions, when executed by the at least one processor, further cause the apparatus to:

receive second data from a second transducer associated with a second user;
compare the first data and the second data to a template, wherein the template identifies a particular resonance frequency; and
determining which of the first user and the second user is in possession of the object based on the comparison.

Para 12. The apparatus of claim 11, wherein the executable instructions, when executed by the at least one processor, further cause the apparatus to:

identify a second time interval during which the second data corresponds to the template; and
determine that the second user is in possession of an object during the second time interval.

Para 13. A method comprising:

processing, by at least one processor, image data;
detecting, by the at least one processor, presence of an object within the image data;
determining a first size of the object;
comparing, by the at least one processor, the first size to a threshold;
determining that a first user is in possession of the object if the first size meets or exceeds the threshold, and determining that the first user is not in possession of the object if the first size is less than the threshold.

Para 14. The method of claim 13, further comprising:

communicating a proximity signal for determining whether the object is within range for controlling whether power is supplied to a camera providing the image data; and

detecting a return signal within a predetermined amount of time of communicating the proximity signal and providing power to the camera in response to the detecting.

Para 15. The method of claim 13, further comprising:

processing second image data received from a second device associated with a second user;
detecting presence of the object within the first image data and the second image data;
determining a second size of the object in the second image data; and
determining that at least one of the first size and the second size meets or exceeds the threshold.

Para 16. The method of claim 13, further comprising:

receiving first data from a first transducer; and
comparing the first data to a template, wherein the template identifies a particular resonance frequency, and wherein the determining that the first user is in possession of the object is further based on the comparison identifying the particular resonance frequency in the first data.

Para 17. A non-transitory computer readable medium storing executable instructions that, when executed, cause an apparatus at least to perform:

processing image data;
detecting presence of an object within the image data;
determining a first size of the object;
comparing the first size to a threshold;
determining that a first user is in possession of the object if the first size meets or exceeds the threshold, and determining that the first user is not in possession of the object if the first size is less than the threshold.

Para 18. The computer readable medium of claim 17, wherein the executable instructions, when executed, cause the apparatus to perform:

communicating a proximity signal for determining whether the object is within range for controlling whether power is supplied to a camera providing the image data;
detecting a return signal within a predetermined amount of time of communicating the proximity signal and providing power to the camera in response to the detecting.

Para 19. The computer readable medium of claim 17, wherein the executable instructions, when executed, cause the apparatus to perform:

processing second image data received from a second device;
detecting presence of the object within the first image data and the second image data;
determining a second size of the object in the second image data; and
determining that at least one of the first size and the second size meets or exceeds the threshold.

Para 20. The computer readable medium of claim 17, wherein the executable instructions, when executed, cause the apparatus to perform:

receiving first data from a first transducer; and
comparing the first data to a template, wherein the template identifies a particular resonance frequency, and wherein the determining that the first user is in possession of the object is further based on the comparison identifying the particular resonance frequency in the first data.

## Claims

**1.** A method comprising:

receiving and processing first data from a first transducer;
comparing the first data to a template, by determining whether the first data corresponds to one or more parameters of the template, the one or more parameters including a particular resonant frequency; and
determining that a first user is in possession of an object when the first data corresponds to the one or more parameters.

**2.** The method of claim 1, further comprising:
identifying a first time interval during which the first data corresponds to the one or more parameters.

**3.** The method of claim 1 or 2, wherein the first transducer is configured to be worn by the first user.

**4.** The method of any of claims 1-3, further comprising:

receiving second data from a second transducer associated with a second user;
compare the first data and the second data to a template; and
determine which of the first user and the second user is in possession of the object based on the comparison of the first data and the second data with the template.

**5.** The method of claim 4, further comprising:

identify a second time interval during which the second data corresponds to the template; and
determine that the second user is in possession of the object during the second time interval.

**6.** The method of any of claims 1-5, wherein a parameter in the one or more parameters of the template identifies a minimum peak voltage level corresponding to an impact force on the object.

**7.** The method of any of claims 1-6, wherein a parameter in the one or more parameters of the template identifies a minimum peak voltage level corresponding to an audible echo associated with an impact force on the object.

**8.** The method of any of claims 1-7, wherein a parameter in the one or more parameters of the template identifies a threshold amount of time between peak voltage levels.

**9.** The method of any of claims 1-8, comprising determining that the first user is not in possession of the object if the first data does not correspond to one or more parameters of the template for a predetermined period of time.

**10.** The method of any of claims 1-9, comprising:

communicating, with a transceiver, a proximity signal;
listening for a return signal from the object; and
if a return signal is received, communicate a powerup signal to the transducer and an image capturing device.

**11.** A non-transitory computer readable medium storing executable instructions that, when executed, cause a processor at least to perform the method according to any preceding claim.

**12.** An apparatus comprising:
at least one processor and a non-transitory computer readable medium according to claim 11.

**13.** The apparatus of claim 12, wherein the at least one processor and the at least one memory are remote from the first transducer.

100
122
102
120
116
108, 118
114
136
104
106
110
112
104
134
130
132
110
106

FIG. 1

114
108
104
112
106b
106
110
144
106a
140
108
114
140
112
110
106b
106
144
106a

FIG. 2A

FIG. 2B

100
102
108, 118
114
170
116
106
110
112
104
122
120
134
132
130
136
108
152
150
114
118
154
148
164
10 km/h
160
162

FIG. 2C

FIG. 2D

FIG. 2E

120
308
308a
User Interface
302
Processor
304
Memory
306
Power Supply
122
TX/RX
Other Input Devices
310
116
136
114, 154
112
118
Sensor
TX/RX
Processing System
152
Memory
150
Power Supply
108
106
First Shoe Sensor
110
TX/RX
110
TX/RX
Second Shoe Sensor
106
134
132
130
FIG. 3

400
102
108, 118
170
106
110
112
104
110
106
132
130

FIG. 4

108
118
Sensor
154
Input/
Output
Processing
System
152
Memory
150
Power Supply
400
130
132
120
308
308a
User Interface
122
TX/RX
302
Processor
304
Memory
306
Power Supply
Other
Input
Devices
310

FIG. 5

Digital "Possession"
102
130
130
600
Yes
No

FIG. 6

Analog "Possession"
700
702
704
102
130
Yes
Near
Far
Out

FIG. 7

Power for Tag and Radio
Power for Tag
Reader
Tag
Backscattered Signal

FIG. 8A

Power for Radio
Battery
Reader
Tag
Backscattered Signal
Power for Tag

FIG. 8B

Battery
Reader
Tag and Radio
Transmitted Signal from Tag
Power for Tag and Radio

FIG. 8C

Antenna
Power Amp
Low Noise Amp
RF + Baseband
Microcontroller
Re-Chargeable Battery
104
Embedded Primary Cell Battery
Active Circuitry
Modulator
Passive Circuitry
Auxiliary Sensor Interface
Antenna
130

FIG. 9

Antenna
VLSI Digital Radio
Microcontroller
Re-Chargeable Battery
104
Antenna
VLSI Digital Radio
Embedded Microcontroller
Embedded Primary Cell Battery
130

FIG. 10

H
L
~λ/3
~λ/6
1100

FIG. 11

Antenna
Duplexer
Power Amp
Modulator
LNA
A/D
Microcontroller
Power Supply
Re-Chargeable Battery
104
Diode
1/2 Antenna
2/2 Antenna
1202
1200

FIG. 12

102a
102b
132, 1202
130, 1200

FIG. 13

132, 1202
130, 1200
120


FIG. 14

1500
Player A
Total Games: 24
Total Mins: 2032
Top Speed: 17.3 kmh
Top Speed on Ball: 18.2 kmh
Game No.: 24
Select New Game
Speed
17.2
Km/h
Speed On Ball
15.2
Km/h
No. of Sprints
86
Sprints
Kick Power
85.6
Km/h
Gameline
Distance: 5,4 km
Time Played: 92 min
No. of Possessions: 54
20 km/h
1502
1502a
1502b
GOAL!
60:00
70:00
80:00
90:00

FIG. 15

1600

Team A

Total Games: 24

Game No.: 24

Select New Game

Total Sprint Speed

Select New Metric

| | | |
|---|---|---|
| Player A | | 17.2 Km/h |
| Player B | | 15.2 Km/h |
| Player C | | 16.6 Km/h |
| Player D | | 14.4 Km/h |
| Player E | | 13.1 Km/h |

AD A LINK

AD B LINK

AD C LINK

AD D LINK

FIG. 16

1700
Player A
Total Games: 24
Total Mins: 2032
Top Speed: 17.3 km/h
Top Speed on Ball: 16.2 km/h
Game No.: 24
Select New Game
Speed
17.2
Km/h
Speed On Ball
15.2
Km/h
Goal: 18.4 km/h
No. of Sprints
86
Sprints
Kick Power
85.6
Km/h
Gameline
Distance: 5.4 km
Time Played: 92 min
No. of Possessions: 54
20 km/h
1502
1502a
1502b
60:00
70:00
80:00
90:00

FIG. 17A

1750
Player A
Total Games: 25
Total Mins: 2121
Top Speed: 18.5 km/h
Top Speed on Ball: 16.2 km/h
Game No.: 25
Select New Game
Speed
18.5 Km/h
New Goal: 18.9 km/h
Speed On Ball
15.9 Km/h
No. of Sprints
85 Sprints
Kick Power
87.4 Km/h
Gameline
Distance: 5.2 km
Time Played: 89 min
No. of Possessions: 58
CONGRATULATIONS ON REACHING YOUR TOP SPEED GOAL!

FIG. 17B

Player A
Total Games: 24
Total Mins: 2032
Top Speed: 17.3 km/h
Top Speed on Ball: 16.2 km/h
Game No.: 24
Select New Game
Goal: 22.0 km/h
20.2
km/h
Speed
Goal: 92.0 km/h
86.3
km/h
Power
Goal: 10 mins
6.46
mins
Possession
Distance: 5.4 km
Time Played: 92 min
No. of Possessions: 54
Gameline
20 km/h
60:00
70:00
80:00
90:00
1502
1502a
1502b
1800

FIG. 18

Time
Pressure
Sensor Output
Accelerometer
Output

FIG. 19A

Time
Pressure Sensor Output
Accelerometer Output

FIG. 19B

FIG. 20

FIG. 21

FIG. 22
1: Player A possession and then kicks the ball
2: Player B proximity and then contact with foot to show first contact of possession
SENSING ARCHITECTURE
SS
IM
BS
CS
CP
FP
POTENTIAL EMBODIMENTS
A
P
G
C
T
R
RF
M
FIG. 23
SENSING ARCHITECTURE
SS
IM
BS
CS
CP
FP
POTENTIAL EMBODIMENTS
A
P
G
C
T
R
RF
M

SENSING
ARCHITECTURE
SS
IM
BS
CS
CP
FP
POTENTIAL
EMBODIMENTS
A
P
G
C
T
R
RF
M

FIG. 24

SENSING
ARCHITECTURE
SS
IM
BS
CS
CP
FP
POTENTIAL
EMBODIMENTS
A
P
G
C
T
R
RF
M

FIG. 25

FIG. 26

FIG. 27

SENSING
ARCHITECTURE
SS
IM
BS
CS
CP
FP
POTENTIAL
EMBODIMENTS
A
P
G
C
T
R
RF
M
SENSING
ARCHITECTURE
SS
IM
BS
CS
CP
FP
POTENTIAL
EMBODIMENTS
A
P
G
C
T
R
RF
M

SENSING
ARCHITECTURE
SS
IM
BS
CS
CP
FP
POTENTIAL
EMBODIMENTS
A
P
G
C
T
R
RF
M
1: Left or right
Ball/Boot impact
SENSING
ARCHITECTURE
SS
IM
BS
CS
CP
FP
POTENTIAL
EMBODIMENTS
A
P
G
C
T
R
RF
M

FIG. 28

FIG. 29

FIG. 30
SENSING
ARCHITECTURE
SS
IM
BS
CS
CP
FP
POTENTIAL
EMBODIMENTS
A
P
G
C
T
R
RF
M
1: Incoming pass
2: Outgoing pass after 1 touch
FIG. 31
SENSING
ARCHITECTURE
SS
IM
BS
CS
CP
FP
POTENTIAL
EMBODIMENTS
A
P
G
C
T
R
RF
M
1: Player A has possession,
Player B approaches
2: Player B attempts a tackle
3: Player A keeps possession,and
breaks away from Player B

FIG. 32

SENSING ARCHITECTURE

SS IM BS CS CP FP

POTENTIAL EMBODIMENTS

A P G C T

R RF M

1: Player A has possession and is dribbling with ball, Player B moves in to tackle

2: Player A looses possession to Player B during contested possession (each player in proximity to ball)

3: Player B leaves with possession or makes a successful pass to a team member

FIG. 33

SENSING
ARCHITECTURE
SS
IM
BS
CS
CP
FP
POTENTIAL
EMBODIMENTS
A
P
G
C
T
R
RF
M

1: Player A comes into Player B proximity, turns 360 over ball

2: Player A keeps possession,and breaks away from Player B

FIG. 34
1: Player Possession
2: Player Percentage near ball
3: Game Time
Time
SENSING ARCHITECTURE
SS
IM
BS
CS
CP
FP
POTENTIAL EMBODIMENTS
A
P
G
C
T
R
RF
M
FIG. 35
Speed
Passes
Speed on ball
Possessions
One touch passes
Man to Man marking
Closing in
Tracking back
Tackles
SENSING ARCHITECTURE
SS
IM
BS
CS
CP
FP
POTENTIAL EMBODIMENTS
A
P
G
C
T
R
RF
M

FIG. 36

SENSING
ARCHITECTURE
SS
IM
BS
POTENTIAL
EMBODIMENTS
A
P
G
C
T
SENSING
ARCHITECTURE
SS
IM
BS
POTENTIAL
EMBODIMENTS
A
P
G
C
T

FIG. 37

FIG. 38
SENSING
ARCHITECTURE
SS
IM
BS
POTENTIAL
EMBODIMENTS
A
P
G
C
T
FIG. 39
SENSING
ARCHITECTURE
SS
IM
BS
POTENTIAL
EMBODIMENTS
A
P
G
C
T
Elevation Angle

FIG. 40

5: Ball Spin and Speed

2: Core direction

4: Ball impact location

3: Foot path

1: Foot plant

SENSING ARCHITECTURE

SS IM BS CS

POTENTIAL EMBODIMENTS

A P G C T

FIG. 41

3: Ball Speed

1: Foot distance traveled

2: Ball impact

SENSING ARCHITECTURE

SS IM BS CS

POTENTIAL EMBODIMENTS

A P G C T

FIG. 42
5: Ball Spin and Speed
2: Core direction
4: Ball impact location
3: Foot path
1: Foot plant
Elevation Angle
5: Ball Spin and Speed
4: Ball impact location
3: Foot path
1: Foot plant
Elevation Angle
SENSING ARCHITECTURE
SS
IM
BS
CS
POTENTIAL EMBODIMENTS
A
P
G
C
T
FIG. 43
1: Player A has possession, and is running at speed.
2: Player A kicks the moving ball. The ball reports accurate speed.
SENSING ARCHITECTURE
SS
IM
BS
CS
CP
FP
POTENTIAL EMBODIMENTS
A
P
G
C
T
R
RF
M

FIG. 44

SENSING
ARCHITECTURE
SS
IM
BS
CS
CP
FP
POTENTIAL
EMBODIMENTS
A
P
G
C
T
R
RF
M
1: Player A has possession,
and is running at speed.
2: Player A kicks the moving ball.
The ball reports accurate speed and that
a successful pass has been made.
SENSING
ARCHITECTURE
SS
IM
BS
CS
CP
FP
POTENTIAL
EMBODIMENTS
A
P
G
C
T
R
RF
M

FIG. 45

FIG. 46
1: Player A has possession, Player B approaches
2: Player B attempts a tackle and gives away a free kick
3: Player A gets to place the ball and take a set place
SENSING ARCHITECTURE
SS
IM
BS
CS
CP
FP
POTENTIAL EMBODIMENTS
A
P
G
C
T
R
RF
M
FIG. 47
1: Penalty set distance and only keeper can be near the ball
2: Set place, varied distance and ball passes other players proximity
SENSING ARCHITECTURE
SS
IM
BS
CS
CP
FP
POTENTIAL EMBODIMENTS
A
P
G
C
T
R
RF
M

FIG. 48

SENSING
ARCHITECTURE
SS
IM
BS
CS
POTENTIAL
EMBODIMENTS
A
P
G
C
T

1: Opposition sets up free kick/penalty

2: Player takes a run up and kicks the ball

FIG. 49

SENSING
ARCHITECTURE
SS
IM
BS
CS
OP
FP
POTENTIAL
EMBODIMENTS
A
P
G
C
T
R
RF
M

1: Opposition sets up free kick or penalty

2: Keeper saves the ball, with a catch or parry

FIG. 50
1: Keeper saves ball
2: Keeper kicks/throws the ball
SENSING ARCHITECTURE
SS
IM
BS
CS
CP
FP
POTENTIAL EMBODIMENTS
A
P
G
C
T
R
RF
M
FIG. 51
1: Back-step
2: Side-step
SENSING ARCHITECTURE
CS
POTENTIAL EMBODIMENTS
A
G

FIG. 52

1: Player is running fast and changes direction rapidly

2: Player is now running on different course from before.

SENSING ARCHITECTURE

SS
CS

POTENTIAL EMBODIMENTS

A
G
C

FIG. 53

1: Player is running fast on ball and changes direction rapidly

2: Player is now running on different course from before.

SENSING ARCHITECTURE

SS
IM
BS
CS
CP
FP

POTENTIAL EMBODIMENTS

A
P
G
C
T
R
RF
M

FIG. 54
Speed
Passes
Speed on ball
Possessions
One touch passes
Man to Man marking
Closing in
Tracking back
Tackles
SENSING ARCHITECTURE
SS
CS
POTENTIAL EMBODIMENTS
A
G
C
FIG. 55
SENSING ARCHITECTURE
SS
CS
POTENTIAL EMBODIMENTS
A
G
C

FIG. 56
SENSING
ARCHITECTURE
SS
BS
CS
CP
FF
POTENTIAL
EMBODIMENTS
A
G
C
T
M
R
RF
1: Player A defensive posturing, getting ready to move quickly or block advancing player B movement
FIG. 57
SENSING
ARCHITECTURE
CS
CP
POTENTIAL
EMBODIMENTS
A
G
C
T
R
RF
1: Players in proximity
2: Player keeps other player in proximity

FIG. 58
SENSING
ARCHITECTURE
CS
CP
POTENTIAL
EMBODIMENTS
A
G
C
T
R
RF
FIG. 59
1: Players in proximity
2: Player A turns and breaks away
SENSING
ARCHITECTURE
CS
CP
SS
POTENTIAL
EMBODIMENTS
A
G
C
T
R
RF

FIG. 60

FIG. 61

SENSING
ARCHITECTURE
SS
IM
BS
CS
CP
FP
POTENTIAL
EMBODIMENTS
A
P
G
C
T
R
RF
M
SENSING
ARCHITECTURE
SS
IM
BS
CS
CP
FP
POTENTIAL
EMBODIMENTS
A
P
G
C
T
R
RF
M

3: Successful pass received
2: Pass through proximity radius
1: Ball/Boot impact
SENSING ARCHITECTURE
SS
IM
BS
CS
CP
FP
POTENTIAL EMBODIMENTS
A
P
G
C
T
R
RF
M
SENSING ARCHITECTURE
SS
IM
BS
CS
CP
FP
POTENTIAL EMBODIMENTS
A
P
G
C
T
R
RF
M

FIG. 62

FIG. 63

FIG. 64
4: Player collects the ball
5: Ball recognizes a throw
SENSING ARCHITECTURE
CS
BS
CP
POTENTIAL EMBODIMENTS
A
P
G
C
T
R
RF
FIG. 65
2/3: Player A kicks the ball off Player B and out of bounds
4/5: Player A or someone on his team picks up the ball and throws it back into play
SENSING ARCHITECTURE
SS
IM
BS
CS
CP
FP
POTENTIAL EMBODIMENTS
A
P
G
C
T
R
RF
M

FIG. 66
SENSING
ARCHITECTURE
SS
CS
POTENTIAL
EMBODIMENTS
A
C
FIG. 67
SENSING
ARCHITECTURE
SS
IM
BS
CS
CP
POTENTIAL
EMBODIMENTS
A
P
G
C
T
R
RF
3: Or throw
1: Keeper catch the ball
2: Keeper hold ball and kick out

FIG. 68
SENSING
ARCHITECTURE
SS
IM
BS
CS
CP
POTENTIAL
EMBODIMENTS
A
P
G
C
T
R
RF
1: Keeper parry's ball out of bounds
2: Opposition player places corner
FIG. 69
SENSING
ARCHITECTURE
SS
IM
BS
CS
CP
POTENTIAL
EMBODIMENTS
A
P
G
C
T
R
RF
1: Keeper catches the ball cleanly
2: Keeper unable to catch ball so parry's ball out of bounds

FIG. 70

1: Keeper dives down

2: Keeper takes the ball in their hands

SENSING ARCHITECTURE

SS IM BS CS CP

POTENTIAL EMBODIMENTS

A P G C T

R RF

FIG. 71

SENSING ARCHITECTURE

CS

POTENTIAL EMBODIMENTS

A

FIG. 72

FIG. 73

1: Keeper drops the ball
2: Ball bounces followed by kick event
SENSING ARCHITECTURE
SS
IM
BS
CP
POTENTIAL EMBODIMENTS
A
P
G
C
T
R
RF
1: Keeper collects the ball
2: Keeper sets up a by kick and kicks it
SENSING ARCHITECTURE
SS
IM
BS
CS
CP
POTENTIAL EMBODIMENTS
A
P
G
C
T
R
RF

FIG. 74

1: Keeper collects the ball

2: Keeper distributes the ball back in play

SENSING ARCHITECTURE

SS IM BS CS CP

POTENTIAL EMBODIMENTS

A P G C T

R RF

FIG. 75

SENSING ARCHITECTURE

SS IM BS CS CP

POTENTIAL EMBODIMENTS

A P G C T

R RF

FIG. 76

FIG. 77

SENSING
ARCHITECTURE
CS
POTENTIAL
EMBODIMENTS
RF
SENSING
ARCHITECTURE
CS
SS
POTENTIAL
EMBODIMENTS
RF
A
G
C
T

FIG. 78

SENSING
ARCHITECTURE
CS
SS
POTENTIAL
EMBODIMENTS
RF
A
G
C
T
SENSING
ARCHITECTURE
SS
IM
BS
CS
CP
FP
POTENTIAL
EMBODIMENTS
A
P
G
C
T
R
RF
M

FIG. 79

FIG. 80
SENSING ARCHITECTURE
SS
IM
BS
CS
CP
FP
POTENTIAL EMBODIMENTS
A
P
G
C
T
R
RF
M
FIG. 81
SENSING ARCHITECTURE
CS
POTENTIAL EMBODIMENTS
A
M
G
C

FIG. 82

FIG. 83

SENSING
ARCHITECTURE
SS
IM
BS
CS
CP
FP
POTENTIAL
EMBODIMENTS
A
P
G
C
T
R
RF
M
SENSING
ARCHITECTURE
SS
IM
BS
CS
CP
FP
POTENTIAL
EMBODIMENTS
A
P
G
C
T
R
RF
M

FIG. 84

FIG. 85

SENSING
ARCHITECTURE
SS
IM
BS
CS
CP
FP
POTENTIAL
EMBODIMENTS
A
P
G
C
T
R
RF
M
Herdest kick during game
SENSING
ARCHITECTURE
SS
BS
CS
CP
FP
POTENTIAL
EMBODIMENTS
R
RF
M
C
MC

SENSING
ARCHITECTURE
SS
BS
CS
CP
FP
IM
POTENTIAL
EMBODIMENTS
R
RF
M
C
MC
A
T
SENSING
ARCHITECTURE
BS
POTENTIAL
EMBODIMENTS
MC
P
Pressure
Magnetic Field
Intensity

FIG. 86

FIG. 87

FIG. 88

FIG. 89

SENSING ARCHITECTURE
BS
CS
CP
IM
POTENTIAL EMBODIMENTS
R
RF
M
C
MC
A
T
P
SENSING ARCHITECTURE
SS
BS
FP
IM
POTENTIAL EMBODIMENTS
R
RF
M
MC
A
T
G

SENSING
ARCHITECTURE
SS
BS
FP
IM
POTENTIAL
EMBODIMENTS
R
RF
M
MC
A
T
G
SENSING
ARCHITECTURE
SS
POTENTIAL
EMBODIMENTS
RF
M
MC

FIG. 90

FIG. 91

FIG. 92
SENSING ARCHITECTURE
SS
POTENTIAL EMBODIMENTS
RF
M
MC
FIG. 93
SENSING ARCHITECTURE
SS
POTENTIAL EMBODIMENTS
RF
M
1: Magnet switch is in rest position.
2: Ball comes near and switch is pulled up to strike contact showing proximity to ball

FIG. 94

FREQUENCY DOUBLER TAG
ANTENNA
9502
9500
FREQUENCY DOUBLER CIRCUIT
9501

FIG. 95

9600
9601
9602
Y
X
N

FIG. 96

9700
Z
Theta
Y
Phi
X

FIG. 97

dB (Gain Total)
1.5870e+000
1.3399e-001
-1.3190e+000
-2.7719e+000
-4.2249e+000
-5.6779e+000
-7.1308e+000
-8.5838e+000
-1.0037e+001
-1.1490e+001
-1.2943e+001
-1.4396e+001
-1.5849e+001
-1.7302e+001
-1.8755e+001
-2.0208e+001
-2.1661e+001

9800
Z
Y
X
Phi

FIG. 98

dB (Gain Total)
4.5696e+000
2.4204e+000
2.7110e-001
-1.8782e+000
-4.0275e+000
-6.1767e+000
-8.3260e+000
-1.0475e+001
-1.2625e+001
-1.4774e+001
-1.6923e+001
-1.9072e+001
-2.1222e+001
-2.3371e+001
-2.5520e+001
-2.7669e+001
-2.9819e+001

9900
X
Y
Z
9901
9902
9903
9904

FIG. 99

10000
Z
Theta
Phi
Y
X
dB (Gain Total)
3.3851e+000
1.8855e+000
3.8600e-001
-1.1135e+000
-2.6131e+000
-4.1126e+000
-5.6121e+000
-7.1116e+000
-8.6112e+000
-1.0111e+001
-1.1610e+001
-1.3110e+001
-1.4609e+001
-1.6109e+001
-1.7608e+001
-1.9108e+001
-2.0607e+001

FIG. 100

10100
Z
Theta
Y
Phi
X

FIG. 101

dB (Gain Total)
5.5625e+000
4.2880e+000
3.0135e+000
1.7390e+000
4.6447e-001
-8.1003e-001
-2.0845e+000
-3.3590e+000
-4.6335e+000
-5.9081e+000
-7.1826e+000
-8.4571e+000
-9.7316e+000
-1.1006e+001
-1.2281e+001
-1.3555e+001
-1.4830e+001

10200

Z

Y

X

FIG. 102

10300
Z
Theta
Y
Phi
X
dB (Gain Total)
1.9432e+000
5.1316e-001
-9.1691e-001
-2.3470e+000
-3.7771e+000
-5.2071e+000
-6.6372e+000
-8.0673e+000
-9.4973e+000
-1.0927e+001
-1.2357e+001
-1.3788e+001
-1.5218e+001
-1.6648e+001
-1.8078e+001
-1.9508e+001
-2.0938e+001

FIG. 103

10400
Z
Theta
Phi
Y
X
dB (Gain Total)
2.4433e+000
1.6835e+000
9.2362e-001
1.6377e-001
-5.9608e-001
-1.3559e+000
-2.1158e+000
-2.8756e+000
-3.6355e+000
-4.3953e+000
-5.1552e+000
-5.9150e+000
-6.6749e+000
-7.4347e+000
-8.1946e+000
-8.9544e+000
-9.7143e+000

FIG. 104

10500
10501
NFF transceiver
COM P
PA8
TIM1_CH1
Vcc
Vdd
Off board
Connectors
uC STM32F103x
ADC
10514
UART
UTAG
DUART
10502
uC MHz Crystal
SPI
Vcc&Vdd
Accelerometer
Vcc
EN
LDO
Vdd
LDO
Vcharge
Battery
charger
RfEN
PAO
Vcc
10513
BB amplifier low
l/f noise
RfEN
10512
RfEN
BB filters low
l/f noise
10511
Vcc
10506
Sub
harmonic
mixer
Filter
RfEN
Vcc
LNA
10515
Filter
10504b
RfEN
TxEN
GPIOs
Vdd
Vcc
Nordic Radio
SPI
32 kHz Crystal
SP12 (P813.P814.PB15)
Vcc&Vdd
TxEN
Vcc
PLL fast
tuning
fo
10507
RfEN
Vosc
Ref oscillator
Vcc
TxEN
10510
Variable gain
PA 10m W max
2xfo reject
2xfo reject
10504a
10551
10552
10505

FIG. 105

10600

| Distance/ m | SNR /dB | Calculated and simulated Signal strength/ dBm |
|---|---|---|
| .74 | 10 | -132.2 |
| .7 | 11.5 | -130.7 |
| .6 | 15.7 | -126.5 |
| .5 | 20.6 | -121.6 |
| .4 | 26.6 | -115.6 |
| .3 | 34.4 | -107.8 |
| .2 | 45.1 | -97.1 |
| .1 | 62.7 | -79.5 |

FIG. 106

10700
10701
START SWEEP SIGNAL (CHIRP)
10702
TRANSMIT SIGNAL TO BALL AT CURRENT SWEEP FREQUENCY
10703
RECEIVE RETURNED SIGNAL FROM BALL
10704
COMPARE FREQUENCY OF RETURNED SIGNAL WITH CURRENT DOUBLED TRANSMIT (SWEEP) FREQUENCY TO OBTAIN FREQUENCY DIFFERENCE
YES
10705
CONTINUE SWEEP?
NO
10706
DETERMINE PROXIMITY OF BALL FROM FREQUENCY DIFFERENCE INFORMATION

FIG. 107

102
10806
10804
10802
130
10810
10808

FIG. 108

1090a
1090b
130
Width
130
Width

FIG. 109A

FIG. 109B

Transducer
Output
11002
11006
11004
11000a
11008
Δt
Time

FIG. 110a

11000b
Time

FIG. 110b

11100
Possession
Not in Possession
11102a
11102b
11102c
$t_1$
$t_2$
$t_3$
$t_4$
$t_5$
$t_6$
Time

FIG. 111

10804a
102a
11200b
130
10804b
102b
11200a

FIG. 112

Process image data
11302
Detect presence of an object within the image data
11304
Determine a size of the object
11306
Compare the size to a threshold
11308
Size greater than or equal to threshold?
11310
Yes
No
Determine that user is in possession of the object
11312
Determine that user is not in possession of the object
11314

FIG. 113

Process first image data from a first device and second image data from a second device
11402
Detect presence of an object within the first image data and the second image data
11404
Determine a first size of the object in the first image data and a second size of the object in the second image data
11406
Determine that at least one of the first size and the second size is greater than or equal to a threshold
11408
Is first size greater than second size?
11410
Yes
No
Determine that first user is in possession of the object
11412
Determine that second user is in possession of the object
11414

FIG. 114

Process data generated by a transducer
11502
Compare the data to a predetermined template
11504
Identify a fist time interval during which the data corresponds to the predetermined template
11506
Determine that a user is in possession of an object during the time interval
11508

FIG. 115

Europäisches
Patentamt
European
Patent Office
Office européen
des brevets

# EUROPEAN SEARCH REPORT

Application Number
EP 23 16 7562

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/304497 A1 (MOLYNEUX JAMES [US] ET AL) 15 December 2011 (2011-12-15)<br>* paragraph [0072] - paragraph [0073] *<br>* paragraph [0088] - paragraph [0094] *<br>* paragraph [0119] - paragraph [0154] *<br>----- | 1-13 | INV.<br>A43B1/00<br>A63B24/00<br>G01S13/34<br>G10L25/51<br>A61B5/11<br>A61B5/00<br>G06V20/10<br>G01S13/75<br>G01S13/82<br>G06V20/40<br>A43B3/34<br>A43B5/02 |
| X | GB 2 440 510 A (SANGHA PARM [GB]) 6 February 2008 (2008-02-06)<br>* page 5, line 5 - line 12 *<br>* page 10, line 7 - line 16 *<br>----- | 1,2,11,12 | |
| | | | TECHNICAL FIELDS SEARCHED (IPC) |
| | | | G06K<br>A43B<br>A63B<br>G01S<br>G10L |

The present search report has been drawn up for all claims

1

| Place of search | Date of completion of the search | Examiner |
|---|---|---|
| The Hague | 5 June 2023 | Boltz, Sylvain |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 16 7562

This annex lists the patent family members relating to the patent documents cited in the above-mentioned European search report. The members are as contained in the European Patent Office EDP file on
The European Patent Office is in no way liable for these particulars which are merely given for the purpose of information.

05-06-2023

| Patent document cited in search report | Publication date | Patent family member(s) | Publication date |
|---|---|---|---|
| US 2011304497 A1 | 15-12-2011 | BR PI1107035 A2 | 10-11-2015 |
| | | CA 2762500 A1 | 29-06-2012 |
| | | CA 2883098 A1 | 29-06-2012 |
| | | CN 202538303 U | 21-11-2012 |
| | | CN 202983134 U | 12-06-2013 |
| | | EP 2472288 A1 | 04-07-2012 |
| | | JP 5657512 B2 | 21-01-2015 |
| | | JP 5974070 B2 | 23-08-2016 |
| | | JP 5976771 B2 | 24-08-2016 |
| | | JP 6049677 B2 | 21-12-2016 |
| | | JP 2012139493 A | 26-07-2012 |
| | | JP 2015091323 A | 14-05-2015 |
| | | JP 2015111126 A | 18-06-2015 |
| | | JP 2015111127 A | 18-06-2015 |
| | | JP 2017003595 A | 05-01-2017 |
| | | KR 20120076418 A | 09-07-2012 |
| | | US 2011304497 A1 | 15-12-2011 |
| | | US 2012262329 A1 | 18-10-2012 |
| | | US 2014046463 A1 | 13-02-2014 |
| | | US 2014358261 A1 | 04-12-2014 |
| | | US 2015258373 A1 | 17-09-2015 |
| | | US 2015258413 A1 | 17-09-2015 |
| | | US 2015294141 A1 | 15-10-2015 |
| | | US 2015297951 A1 | 22-10-2015 |
| | | US 2016059074 A1 | 03-03-2016 |
| | | US 2016059075 A1 | 03-03-2016 |
| GB 2440510 A | 06-02-2008 | GB 2440510 A | 06-02-2008 |
| | | WO 2008015465 A1 | 07-02-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 66074312
- US 4890111 A
- US 6073086 A
- US 6157898 A
- US 6148271 A
- US 6151563 A
- US 20070191083 A
- US 20070059675 A
- US 20070060425 A
- US 20070299625 A
- US 20080085790 A
- US 20080084351 A
- US 20080088303 A
- US 20080090683 A
- WO 2008080626 A
- WO 2008104247 A
- WO 2008119479 A